# EUROPEAN PATENT APPLICATION

(11) **EP 4 566 638 A2**
(43) Date of publication of application: **11.06.2025**
(21) Application number: 25155195.8
(22) Date of filing: 27.05.2016
(51) Int. Cl.: A61L 27/54

(54) **IMPLANT FOR TREATMENT OF AN OCULAR CONDITION**

(30) Priority: 29.05.2015 US 201562168292 P; 18.08.2015 US 201562206535 P; 30.11.2015 US 201562260964 P; 29.04.2016 US 201662329769 P
(62) Divisional of application: 16804168.9
(71) Applicant: Allergan, Inc., Irvine, CA 92612 (US)
(72) Inventor: Garcia, Andres, Durham, North Carolina, 27707 (US); Verhoeven, Rozemarijn Suzanne, Pittsboro, North Carolina, 27312 (US); Navratil, Tomas, Carrboro, North Carolina, 27510 (US); Schiffman, Rhett, Morrisville, North Carolina, 27560 (US)
(74) Representative: Hoffmann Eitle

(57) **Abstract**

The present disclosure relates to the field of pharmaceutical compositions, ocular implants, and systems and methods for treating an ocular condition. In certain aspects, the disclosure provides ocular implant systems for treating post-operative inflammation. In certain aspects, the ocular implants taught herein are rapid release extended treatment implants.

## Description

### CROSS REFERENCE TO RELATED APPLICATIONS

The present Application claims priority to U.S. Provisional Application No. 62/168,292, filed on May 29, 2015, and U.S. Provisional Application No. 62/206,535, filed on August 18, 2015, and U.S. Provisional Application No. 62/260,964, filed on November 30, 2015, and U.S. Provisional Application No. 62/329,769, filed on April 29, 2016.

### FIELD

The present disclosure relates to the field of pharmaceutical compositions, implants formed from pharmaceutical compositions, systems of treating an ocular condition, methods of forming implants, and methods of treating ocular conditions.

### BACKGROUND

Cataracts affect more than 20 million Americans over the age of 40 and cataract surgery is the most common ophthalmic surgery performed in developed countries, with more than 3 million surgeries performed in the United States every year. The most common post-operative complication is anterior segment inflammation. Corticosteroids are commonly used to suppress ocular inflammation and their use following cataract surgery has become a standard practice.

The topical corticosteroids currently available for post-operative inflammation and pain suffer from several drawbacks. First, the corticosteroids are dependent on ocular tissue distribution for efficacy. This is problematic, because following topical application of eye drops, generally 80% of the product is eliminated via the nasolacrimal drainage, thus limiting delivery to the target ocular tissues. Second, because up to 80% of the steroid is eliminated via the nasolacrimal drainage, patients undergoing regular treatment with these steroids suffer from prolonged systemic exposure to the drug. And third, the present corticosteroid treatments on the market suffer from significant compliance problems. This may be due in part to the majority of patients who are candidates for cataract surgery being of advanced age, which is often associated with compliance problems, particularly for topical steroids that require multiple times per day dosing (Burns 1992, Chennamaneni 2013, Shell 1984, Winfield 1990).

Consequently, there is a need in the art for the development of effective pharmaceutical compositions, delivery systems, and methods for treating post-operative ocular pain and inflammation, which do not suffer from these drawbacks.

### BRIEF SUMMARY

The present disclosure addresses a crucial need in the art, by providing an extended treatment and rapid delivery vehicle pharmaceutical formulation that may be directly administered to the subconjunctival region of an eye, or intracamerally placed, and that does not suffer from the drawbacks of the current topical application paradigm.

In certain embodiments, the disclosure relates to precisely engineered biocompatible drug delivery systems and methods of making and utilizing such systems. In aspects, the drug delivery systems comprise a rapid release delivery vehicle that undergoes complete or nearly complete dissolution upon exposure to the physiological conditions of an eye, said dissolution of the delivery vehicle leading to the complete release of therapeutic agent cargo to the site of treatment.

The biocompatible drug delivery systems taught herein are, in some embodiments, engineered using the PRINT^{®} particle technology (Envisia Therapeutics Inc., North Carolina). The PRINT^{®} technology allows for uniform size, shape, and dose concentration in the disclosed drug delivery systems.

Further, the disclosure provides methods of utilizing the taught precisely engineered biocompatible drug delivery systems to treat, *inter alia*, conditions of the eye and tissues surrounding or associated with the eye.

Conditions preventable or treatable according to the present disclosure include post-operative inflammation and/or pain that may be due to a number of surgical procedures or injury.

In certain embodiments, the present disclosure relates to pharmaceutical compositions for treating an ocular condition, comprising: a biocompatible polymer matrix and at least one therapeutic agent.

In certain embodiments, the present disclosure provides for pharmaceutical compositions for treating an ocular condition, comprising: an ocular implant. In aspects, the ocular implant comprises a biocompatible polymer matrix that contains a homogenously dispersed therapeutic agent therein. In some embodiments, the ocular implant is a "non-extruded" ocular implant.

In a particular embodiment, the disclosure provides a pharmaceutical composition for treating an ocular condition, or condition of the surrounding or associated tissues, comprising: A) a biocompatible polymer matrix; and B) at least one therapeutic agent homogenously dispersed within the polymer matrix.

In certain embodiments, the disclosure provides for an extended treatment rapid delivery system for treating post-operative ocular inflammation in an eye of a patient in need thereof, comprising: A) a biocompatible polymer matrix based ocular implant rapid delivery vehicle; and B) at least one therapeutic agent homogeneously dispersed within the polymer matrix, wherein said ocular implant rapid delivery vehicle is formulated to substantially disintegrate within 24 hours of being placed into the eye, and wherein said disintegration of the ocular implant rapid delivery vehicle results in substantially all of the at least one therapeutic agent being released into the eye within 24 hours, and wherein the at least one therapeutic agent provides a therapeutic effect to the eye of the patient for at least 2 days, and wherein said ocular implant rapid delivery vehicle is formulated to reduce post-operative corneal thickening in a patient in need thereof. In some aspects, the therapeutic agent has low solubility in water, or is hardly soluble in water, or is not soluble in water. In some aspects, the therapeutic agent is a corticosteroid. In some aspects, the therapeutic agent is difluprednate. In some aspects the biocompatible polymer matrix comprises poly(ethylene glycol) (*i.e.* "PEG") polymers.

In certain embodiments, the biocompatible polymer matrix comprises as a % w/w of the overall implant pharmaceutical composition: about 5% to about 95% w/w, or about bout 5% to about 90% w/w, or about 5% to about 80%, or about 5% to about 70%, or about 5% to about 60%, or about 10% to about 90% w/w, or about 10% to about 80%, or about 10% to about 70%, or about 10% to about 60%, or about 20% to about 90%, or about 20% to about 80%, or about 20% to about 70%, or about 20% to about 60%, or about 30% to about 90%, or about 30% to about 80%, or about 30% to about 70%, or about 30% to about 60%, or about 40% to about 90%, or about 40% to about 80%, or about 40% to about 70%, or about 40% to about 60% , or about 50% to about 90%, or about 50% to about 80%, or about 50% to about 70%, or about 50% to about 60%, or about 60% to about 90%, or about 60% to about 85%, or about 65% to about 85%, or about 60% to about 80%, or about 60% to about 70%; or about 45% to about 80%, or about 45% to about 75%, or about 45% to about 70%, or about 45% to about 65%, or about 45% to about 60%, or about 45% to about 55%, or about 45% to about 50%, or about 70% to about 80%, or about 65% to about 85%, or about 85% to about 95%, or about 92.5% to about 95%, or about 55% to about 70% w/w of the pharmaceutical composition.

In certain embodiments, the biocompatible polymer matrix is comprised of a first polymer. In aspects, the first polymer comprises as a % w/w of the biocompatible polymer matrix: about 1% to about 100%, or about 1% to about 90% w/w, or about 1% to about 80%, or about 1% to about 70%, or about 1% to about 60%, or about 1% to about 50%, or about 1% to about 40%, or about 1% to about 30%, or about 1% to about 20%, or about 1% to about 15%, or about 1% to about 10%, or about 1% to about 5%. In aspects, the first polymer is a PEG polymer. In aspects, the PEG polymer is PEG 3,350. In aspects, the PEG polymer (*e.g.*, PEG 3,350) can be present as the sole polymer in the biocompatible polymer matrix. In aspects, the PEG polymer (*e.g.*, PEG 3,350) can be present in a mixture of polymers in the biocompatible polymer matrix.

In certain embodiments, the biocompatible polymer matrix is comprised of a first polymer. In aspects, the first polymer comprises as weight of the biocompatible polymer matrix: about 1 µg to about 1,000 µg, about 1 µg to about 500 µg, or about 1 µg to about 400 µg, or about 1 µg to about 300 µg, or about 1 µg to about 200 µg, or about 1 µg to about 100 µg, or about 1 µg to about 50 µg, or about 1 µg to about 40 µg, or about 1 µg to about 30 µg, or about 1 µg to about 20 µg, or about 1 µg to about 10 µg, or about 1 to about 5 µg. In aspects, the first polymer comprises as weight of the biocompatible polymer matrix: about 3 µg to about 5 µg, about 11 µg to about 15 µg, or about 15 µg to about 16 µg, or about 16 to about 17 µg, or about 35 µg to about 53 µg. In aspects, the first polymer is a PEG polymer. In aspects, the PEG polymer is PEG 3,350. In aspects, the PEG polymer (*e.g.*, PEG 3,350) can be present as the sole polymer in the biocompatible polymer matrix. In aspects, the PEG polymer (*e.g.*, PEG 3,350) can be present in a mixture of polymers in the biocompatible polymer matrix.

In certain embodiments, the biocompatible polymer matrix is comprised of a second polymer. In aspects, the second polymer comprises as a % w/w of the biocompatible polymer matrix: about 1% to about 100%, or about 1% to about 90% w/w, or about 1% to about 80%, or about 1% to about 70%, or about 1% to about 60%, or about 1% to about 50%, or about 1% to about 40%, or about 1% to about 30%, or about 1% to about 20%, or about 1% to about 15%, or about 1% to about 10%, or about 1% to about 5%. In aspects, the second polymer is a PEG polymer. In aspects, the PEG polymer is PEG 100,000. In aspects, the PEG polymer (*e.g.*, PEG 100,000) can be present as the sole polymer in the biocompatible polymer matrix. In aspects, the PEG polymer (*e.g.*, PEG 100,000) can be present in a mixture of polymers in the biocompatible polymer matrix.

In certain embodiments, the biocompatible polymer matrix is comprised of a second polymer. In aspects, the second polymer comprises as weight of the biocompatible polymer matrix: about 1 µg to about 1,000 µg, about 1 µg to about 500 µg, or about 1 µg to about 400 µg, or about 1 µg to about 300 µg, or about 1 µg to about 200 µg, or about 1 µg to about 100 µg, or about 1 µg to about 50 µg, or about 1 µg to about 40 µg, or about 1 µg to about 30 µg, or about 1 µg to about 20 µg, or about 1 µg to about 10 µg, or about 1 to about 5 µg. In aspects, the second polymer comprises as weight of the biocompatible polymer matrix: about 26 µg to about 34 µg, or about 92 µg to about 121 µg, about 121 µg to about 128 µg, or about 131 µg to about 136 µg, or about 284 to about 430 µg. In aspects, the second polymer is a PEG polymer. In aspects, the PEG polymer is PEG 100,000. In aspects, the PEG polymer (*e.g.*, PEG 100,000) can be present as the sole polymer in the biocompatible polymer matrix. In aspects, the PEG polymer (*e.g.*, PEG 100,000) can be present in a mixture of polymers in the biocompatible polymer matrix.

In certain embodiments, the biocompatible polymer matrix is comprised of a first polymer and a second polymer. In aspects, the first polymer and the second polymer comprise as a % w/w ratio of the biocompatible polymer matrix: about 1%/99% to about 99%/1%, or about 5%/95% to about 95%/5%, or about 10%/90% to about 90%/10%, or about 15%/85% to about 85%/15%, or about 20%/80% to about 80%/20%, or about 25%/75% to about 75%/25%, or about 30%/70% to about 70%/30%, or about 35%/65% to about 65%/35%, or about 40%/60% to about 60%/40%, or about 45%/55% to about 55%/45%, or about 50%/50%. In aspects, the first polymer and the second polymer comprises as a % w/w ratio of the biocompatible polymer matrix: about 11%/89%. In aspects, the first polymer and the second polymer of PEG polymers. In aspects, the first polymer is a PEG 3,350 polymer, and the second polymer is a PEG 100,000 polymer.

In certain embodiments, the biocompatible polymer matrix is comprised of a first polymer and a second polymer. In aspects, the first polymer and the second polymer respectively comprises as a weight of the biocompatible polymer matrix: about 1 µg to about 1000 µg and about 1 µg to about 1000 µg, or about 1 µg to about 100 µg and about 500 µg to 1000 µg; about 3 µg to about 50 µg and about 25 µg to 500 µg; or 3 µg to about 60 µg and about 25 µg to 500 µg; or about 11 µg to 15 µg and about 92 µg to about 121 µg; or about 15 µg to about 16 µg and about 121 µg to about 128 µg; about 16 µg to about 17 µg and about 132 µg to about 135 µg; or about 35 µg to about 53 µg and about 284 µg to about 427 µg; or about 3 µg to about 4 µg and about 27 µg to about 34 µg.

In certain embodiments, the pharmaceutical implant comprises as a biocompatible polymer matrix content: about 1 µg to about 1000 µg, or about 1 µg to about 900 µg, or about 1 µg to about 800 µg, or about 1 µg to about 700 µg, or about 1 µg to about 600 µg, or about 1 µg to about 500 µg, or about 1 µg to about 450 µg, or about 1 µg to about 400 µg, or about 1 µg to about 350 µg, or about 1 µg to about 300 µg, or about 1 µg to about 250 µg, or about 1 µg to about 200 µg, or about 1 µg to about 150 µg, or about 1 µg to about 100 µg, or about 1 µg to about 50 µg. In certain embodiments, ocular implant comprises as a biocompatible polymer matrix content: about 30 µg to about 40 µg, or about 100 µg to about 140 µg, or about 130 µg to about 150 µg, or about 145 µg to about 155 µg, or about 320 µg to about 480 µg.

In certain embodiments, the therapeutic agent comprises as a % w/w of the overall implant pharmaceutical composition: about 1% to about 90%, or about 1% to about 80%, or about 1% to about 70%, or about 1% to about 60%, or about 1% to about 55%, or about 1% to about 50%, or about 1% to about 45%, or about 1% to about 40%, or about 1% to about 35%, or about 1% to about 30%, or about 1% to about 25%, or about 1% to about 20%, or about 1% to about 15%, or about 1% to about 10%, or about 1% to about 5%, or about 5% to about 90%, or about 5% to about 80%, or about 5% to about 70%, or about 5% to about 60%, or about 5% to about 55%, or about 5% to about 50%, or about 5% to about 45%, or about 5% to about 40%, or about 5% to about 35%, or about 5% to about 30%, or about 5% to about 25%, or about 5% to about 20%, or about 5% to about 15%, or about 5% to about 10%, or about 10% to about 90%, or about 10% to about 80%, or about 10% to about 70%, or about 10% to about 60%, or about 10% to about 55%, or about 10% to about 50%, or about 10% to about 45%, or about 10% to about 40%, or about 10% to about 35%, or about 10% to about 30%, or about 10% to about 25%, or about 10% to about 20%, or about 10% to about 15%, or about 15 % to about 90%, or about 15% to about 80%, or about 15% to about 70%, or about 15% to about 60%, or about 15% to about 55%, or about 15% to about 50%, or about 15% to about 45%, or about 15% to about 40%, or about 15% to about 35%, or about 15% to about 30%, or about 15% to about 25%, or about 15% to about 20%, or about 20% to about 90%, or about 20% to about 80%, or about 20% to about 70%, or about 20% to about 60%, or about 20% to about 55%, or about 20% to about 50%, or about 20% to about 45%, or about 20% to about 40%, or about 20% to about 35%, or about 20% to about 30%, or about 20% to about 25%, or about 30% to about 90%, or about 30% to about 80%, or about 30% to about 70%, or about 30% to about 60%, or about 30% to about 55%, or about 30% to about 50%, or about 30% to about 45%, or about 30% to about 40%, or about 30% to about 35%, or about 40% to about 90%, or about 40% to about 80%, or about 40% to about 70%, or about 40% to about 60%, or about 40% to about 55%, or about 40% to about 50%, or about 40% to about 45%, or about 45% to about 90%, or about 45% to about 80%, or about 45% to about 75%, or about 45% to about 70%, or about 45% to about 65%, or about 45% to about 60%, or about 45% to about 55%, or about 45% to about 50%,or about 50% to about 90%, or about 50% to about 80%, or about 50% to about 70%, or about 50% to about 60%, or about 50% to about 55%, or about 5% to about 8%, or about 10% to about 15%, or about 15% to about 35%, or about 30% to about 45% or about 40% to about 60%; w/w of the pharmaceutical composition.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises PEG polymers, PEG 3,350 and PEG 100,000. In aspects, the PEG 3,350 is present in an amount of from about 11 µg to about 15 µg per implant (*e.g.*, about 12 µg to about 14 µg per implant), and the PEG 100,000 is present in an amount of from about 92 µg to about 121 µg per implant (*e.g.*, about 99 µg to about 114 µg). In aspects, the implant comprises difluprednate in an amount of 24 to 56 µg per implant (e.g., 32 to 48 µg per implant). In some embodiments, the implant is a rod-shaped implant having dimensions of about 225 µm × about 225 µm × about 2,925 µm. In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises PEG polymers, PEG 3,350 and PEG 100,000. In aspects, the PEG 3,350 is present in an amount of from about 15 µg to about 16 µg per implant, and the PEG 100,000 is present in an amount of from about 121 µg to about 128 µg per implant. In aspects, the implant comprises difluprednate in an amount of 16 to 24 µg per implant. In some embodiments, the implant is a rod-shaped implant having dimensions of about 225 µm × about 225 µm × about 2,925 µm. In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises PEG polymers, PEG 3,350 and PEG 100,000. In aspects, the PEG 3,350 is present in an amount of from about 16 µg to about 17 µg per implant, and the PEG 100,000 is present in an amount of from about 132 µg to about 135 µg per implant. In aspects, the implant comprises difluprednate in an amount of 8 to 12 µg per implant. In some embodiments, the implant is a rod-shaped implant having dimensions of about 225 µm × about 225 µm × about 2,925 µm. In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises PEG polymers, PEG 3,350 and PEG 100,000. In aspects, the PEG 3,350 is present in an amount of from about 35 µg to about 53 µg per implant, and the PEG 100,000 is present in an amount of from about 285 µg to about 427 µg per implant. In aspects, the implant comprises difluprednate in an amount of 320 to 480 µg per implant. In some embodiments, the implant is a rod-shaped implant having dimensions of about 300 µm × about 300 µm × about 6,000 µm. In aspects, the implant has a volume of 540,000,000 cubic microns ± 10%. In aspects, the implant is sized and structured to allow for administration in a 21 or 22 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises PEG polymers, PEG 3,350 and PEG 100,000. In aspects, the PEG 3,350 is present in an amount of from about 3 µg to about 4 µg per implant, and the PEG 100,000 is present in an amount of from about 27 µg to about 34 µg per implant. In aspects, the implant comprises difluprednate in an amount of 16 to 24 µg per implant. In some embodiments, the implant is a rod-shaped implant having dimensions of about 300 µm × about 300 µm × about 6,000 µm. In aspects, the implant has a volume of 540,000,000 cubic microns ± 10%. In aspects, the implant is sized and structured to allow for administration in a 21 or 22 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 40% to about 60% w/w of the overall implant.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 65% to about 85% w/w of the overall implant.

In some embodiments, the at least one therapeutic agent is selected from the group consisting of: a corticosteroid, steroid, NSAID, allergy treatment medications, pain treatment medication, or pharmaceutically acceptable salts thereof, and mixtures thereof, as well as biologics and other medications for treating or preventing conditions of the eye.

In particular embodiments, the at least one therapeutic agent is selected from the group consisting of: dexamethasone, fluticasone, loteprednol, difluprednate, fluorometholone, and prednisolone. In other particular embodiments, the at least one therapeutic agent is selected from the group consisting of: beclomethasone, betamethasone, dexamethasone, difluprednate, fluocinolone, fluocinolone acetonide, fluorometholone, fluticasone propionate, loteprednol, loteprednol etabonate, fluorometholone, prednisolone, prednisolone acetate, prednisolone sodium phosphate, triamcinolone, triamcinolone acetonide, triamcinolone benetonide, triamcinolone hexacetonide.

In one embodiment, the at least one therapeutic agent comprises difluprednate or a pharmaceutically acceptable salt thereof or the active metabolite thereof.

In some aspects, the implant degrades in less than 30 days and provides sustained release of the therapeutic agent for the less than the 30 days over which the implant is degrading. In some embodiments, the implant releases a substantially steady state of the therapeutic agent for more than 25 days, 20 days, 15 days, or 10 days of the 30 days over which the implant is degrading to regulate a condition of the eye over such time period.

In some embodiments, however, the implant is designed to dissolve in less than 10 days, less than 5 days, less than 1 day, less than 12 hours, less than 8 hours, less than 4 hours, less than 1 hour, or less than 30 minutes.

In some aspects, the biocompatible implant substantially dissolves immediately upon administration to the eye of a patient (either via intracameral or subconjunctival administration) and the therapeutic agent is released into the eye upon said dissolution of the implant and is available to treat the eye for an extended period of at least about 2-10 days, or about 2-14 days, or about 2-28 days, or about 25-35 days, or about 26-34 days, or about 27-33 days, or about 28 days.

In some embodiments, the implant is formulated to reduce post-operative intraocular pressure (IOP) in a patient in need thereof for at least 1 day, or at least 5 days, or at least 10 days, or at least 15 days, or at least 20 days, or at least 25 days, or at least 30 days, or at least 35 days, or at least 40 days, or at least 45 days, or at least 50 days. In embodiments, IOP is reduced to within about 20%, or about 15%, or about 12.5%, or about 10%, or about 7.5%, or about 5%, or about 2.5%, or about 1% of a baseline.

In some embodiments, after administration of an implant to a patient in need thereof, intraocular pressure (IOP) is not elevated by more than 10 mmHg, or more than 9 mmHg, or more than 8 mmHg, or more than 7 mmHg, or more than 6 mmHg, or more than 5 mmHg, or more than 4 mmHg, or more than 3 mmHg, or more than 2 mmHg, or more than 1 mmHg. In some embodiments, after administration of an implant to a patient in need thereof, intraocular pressure (IOP) is not elevated by more than 90%, or more than 80%, or more than 70%, or more than 60%, or more than 50%, or more than 40%, or more than 30%, or more than 20%, or more than 10%, or more than 5% of a baseline.

In aspects, pharmacokinetics of the ocular implants described herein are measured in a rabbit model. In embodiments, the average Cₘₐₓ for the therapeutic agent or active metabolite thereof in the plasma is about 80% to about 120% of 0.99-2.75 ng/mL. In embodiments, the average AUCₗₐₛₜ for the therapeutic agent or active metabolite thereof in the plasma is about 80% to about 120% of 0.495-2.44 d*ng/mL. In embodiments, the average Cₘₐₓ for the therapeutic agent or active metabolite thereof in the aqueous humor is about 80% to about 120% of 3,420-11,158 ng/mL. In embodiments, the average AUCₗₐₛₜ for the therapeutic agent or active metabolite thereof in the aqueous humor is about 80% to about 120% of 3,323-10,427 d*ng/mL. In embodiments, the average Cₘₐₓ for the therapeutic agent or active metabolite thereof in the bulbar conjunctiva is about 80% to about 120% of 106-233 ng/mL. In embodiments, the average AUCₗₐₛₜ for the therapeutic agent or active metabolite thereof in the bulbar conjunctiva is about 80% to about 120% of 135-716 d*ng/mL. In embodiments, the average Cₘₐₓ for the therapeutic agent or active metabolite thereof in the cornea is about 80% to about 120% of 11,283-39,875 ng/mL. In embodiments, the average AUCₗₐₛₜ for the therapeutic agent or active metabolite thereof in the cornea is about 80% to about 120% of 9,329-33,262 d*ng/mL. In embodiments, the average Cₘₐₓ for the therapeutic agent or active metabolite thereof in the trabecular meshwork is about 80% to about 120% of 9,43 5-24,825 ng/mL. In embodiments, the average AUCₗₐₛₜ for the therapeutic agent or active metabolite thereof in the trabecular meshwork is about 80% to about 120% of 8,571-29,174 d*ng/mL. In embodiments, the average Cₘₐₓ for the therapeutic agent or active metabolite thereof in the iris/ciliary body is about 80% to about 120% of 13,115-41,700 ng/mL. In embodiments, the average AUCₗₐₛₜ for the therapeutic agent or active metabolite thereof in the iris/ciliary body is about 80% to about 120% of 13,174-49,403 d*ng/mL. In embodiments, the average Cₘₐₓ for the therapeutic agent or active metabolite thereof in the retina is about 80% to about 120% of 89.9-92.1 ng/mL. In embodiments, the average AUCₗₐₛₜ for the therapeutic agent or active metabolite thereof in the retina is about 80% to about 120% of d*222-764 ng/mL.

In certain embodiments, the implants are able to reduce post-operative corneal thickening by about 1%, or about 5%, or about 10%, or about 20%, or about 30%, or about 40%, or about 50%, or about 60%, or about 70%, or about 80%, or about 90%, or about 100%. In embodiments, the implants described herein are able to reduce post-operation corneal thickening in the range of from about 1% to about 100%, or about, or about 5% to about 100%, or about 10% to about 100%, or about 20% to about 100%, or about 30% to about 100%, or about 40% to about 100%, or about 50% to about 100%, or about 60% to about 100%, or about 70% to about 100%, or about 80% to about 100%, or about 90% to about 100%.

In other embodiments, the implants described herein are able to reduce post-surgery corneal thickening below a baseline established prior to surgery by about 1% to about 100%, or about, or about 1% to about 90%, or about 1% to about 80%, or about 1% to about 70%, or about 1% to about 60%, or about 1% to about 50%, or about 1% to about 40%, or about 1% to about 30%, or about 1% to about 20%, or about 1% to about 10%, or about 1% to about 5%, or 1% or more, or about 5% or more, or about 10% or more, or about 20% or more, or about 30% or more, or about 40% or more, or about 50% or more.

In some embodiments, the implants described herein are able to reduce corneal thickness to or below a baseline within about 30 days or less, e.g., less than or equal to about 25 days, or less than or equal to 20 days, or less than or equal to 15 days, or less than or equal to 10 days, or less than or equal to 7 days, or less than or equal to 6 days, or less than or equal to 5 days, or less than or equal to 4 days, or less than or equal to 3 days, or less than or equal to 2 days, or less than or equal to 1 days. Therefore, in some embodiments, the implants described herein can reduce surgery-induced corneal thickening to or below a baseline within about 1 day after administration of said implant.

In some embodiments, the reduction in corneal thickening comprises as a percent change from a baseline: about 1%, or about 2%, or about 3%, or about 4%, or about 5%, or about 6%, or about 7%, or about 8%, or about 9%, or about 10%. In embodiments, the reduction in corneal thickening comprises as a percent change from baseline of from about 1% to about 20%, or from about 1% to about 15%, or from about 1% to about 10%, or from about 1% to about 9%, or from about 1% to about 8%, or from about 1% to about 7%, or from about 1% to about 6%, or form about 1% to about 6%, or from about 1% to about 5%, or from about 1% to about 4%, or from about 1% to about 3%, or from about 1% to about 2%.

In embodiments disclosed herein is an extended treatment rapid delivery system for treating post-operative ocular inflammation in an eye of a patient in need thereof, comprising: A) a biocompatible polymer matrix based ocular implant rapid delivery vehicle; and B) at least one therapeutic agent homogeneously dispersed within the polymer matrix, wherein said ocular implant rapid delivery vehicle is formulated to reduce corneal thickening which is associated with administration of an implant.

In embodiments which entail formulating an implant to reduce corneal thickening which is associated with administration of an implant, the amount of polymer matrix per implant is less than about 500 µg, or less than about 400 µg, or less than about 300 µg, or less than about 200 µg, or less than about 150 µg, or less than about 140 µg, or less than about 130 µg, or less than about 120 µg, or less than about 110 µg, or less than about 100 µg, or less than about 90 µg, or less than about 80 µg, or less than about 70 µg, or less than about 60 µg, or less than about 50 µg, or less than about 40 µg, or less than about 30 µg, or less than about 20 µg, or less than about 10 µg. In embodiments, the implant comprises a polymer matrix content of from about 10 µg to about 100 µg, or about 10 µg to about 90 µg, or about 10 µg to about 80 µg, or about 10 µg to about 70 µg, or about 10 µg to about 60 µg, or about 10 µg to about 50 µg, or about 10 µg to about 40 µg, or about 10 µg to about 30 µg, or about 10 µg to about 20 µg.

In some embodiments which entail formulating an implant to reduce corneal thickening which is associated with administration of a hydrophilic implant, the reduction in corneal thickening can be achieved by decreasing the percent weight ratio (% w/w) of the polymer matrix relative to the therapeutic agent. In embodiments, the percent weight ratio (% w/w) of the polymer matrix relative to the therapeutic agent is from about 20% to about 95%, or from about 20% to about 90%, or from about 20% to about 85%, or from about 20% to about 80%, or from about 20% to about 75%, or from about 20% to about 70%, or form about 20% to about 65%, or from about 20% to about 60%, or from about 20% to about 55%, or from about 20% to about 50%, or from about 20% to about 45%, or from about 20% to about 40%, or from about 20% to about 35%, or 30% to about 85%, or from about 30% to about 80%, or from about 30% to about 75%, or from about 30% to about 70%, or form about 30% to about 65%, or from about 30% to about 60%, or from about 30% to about 55%, or from about 30% to about 50%, or from about 30% to about 45%, or from about 30% to about 40%, or from about 30% to about 35%.

In aspects, pharmacokinetics of the ocular implants described herein are measured in a rabbit model. In embodiments, the average Cₘₐₓ for the therapeutic agent or active metabolite thereof in the plasma is about 80% to about 120% of 0.99-2.75 ng/mL. In embodiments, the average AUCₗₐₛₜ for the therapeutic agent or active metabolite thereof in the plasma is about 80% to about 120% of 0.495-2.44 ng/mL. In embodiments, the average Cₘₐₓ for the therapeutic agent or active metabolite thereof in the aqueous humor is about 80% to about 120% of 3,420-11,158 ng/mL. In embodiments, the average AUCₗₐₛₜ for the therapeutic agent or active metabolite thereof in the aqueous humor is about 80% to about 120% of 3,323-10,427 ng/mL. In embodiments, the average Cₘₐₓ for the therapeutic agent or active metabolite thereof in the bulbar conjunctiva is about 80% to about 120% of 106-233 ng/mL. In embodiments, the average AUCₗₐₛₜ for the therapeutic agent or active metabolite thereof in the bulbar conjunctiva is about 80% to about 120% of 135-716 ng/mL. In embodiments, the average Cₘₐₓ for the therapeutic agent or active metabolite thereof in the cornea is about 80% to about 120% of 11,283-39,875 ng/mL. In embodiments, the average AUCₗₐₛₜ for the therapeutic agent or active metabolite thereof in the cornea is about 80% to about 120% of 9,329-33,262 ng/mL. In embodiments, the average Cₘₐₓ for the therapeutic agent or active metabolite thereof in the trabecular meshwork is about 80% to about 120% of 9,435-24,825 ng/mL. In embodiments, the average AUCₗₐₛₜ for the therapeutic agent or active metabolite thereof in the trabecular meshwork is about 80% to about 120% of 8,571-29,174 ng/mL. In embodiments, the average Cₘₐₓ for the therapeutic agent or active metabolite thereof in the iris/ciliary body is about 80% to about 120% of 13,115-41,700 ng/mL. In embodiments, the average AUCₗₐₛₜ for the therapeutic agent or active metabolite thereof in the iris/ciliary body is about 80% to about 120% of 13,174-49,403 ng/mL. In embodiments, the average Cₘₐₓ for the therapeutic agent or active metabolite thereof in the retina is about 80% to about 120% of 89.9-92.1 ng/mL. In embodiments, the average AUCₗₐₛₜ for the therapeutic agent or active metabolite thereof in the retina is about 80% to about 120% of 222-764 ng/mL.

In some embodiments, the ocular implant is a rod-shaped implant comprising a shortest dimension of between about 100 to about 500 µm and a longest dimension of between about 2,000 to about 8,000 µm in length.

In other embodiments, the ocular implant has an aspect ratio of width to length of between 1:2 and 1:40.

In some embodiments, the implant is a rod-shaped implant selected from the group consisting of: a rod-shaped implant having dimensions of about 225 µm × about 225 µm × about 3,000 µm, a rod-shaped implant having dimensions of about 225 µm × about 225 µm × about 4,000 µm, a rod-shaped implant having dimensions of about 300 µm × about 300 µm × about 6,000 µm, a rod-shaped implant having dimensions of about 400 µm × about 400 µm × about 6,000 µm, and a rod-shaped implant having dimensions of about 130 µm × about 180 µm × about 1,500 µm.

In some embodiments, the implant is a rod-shaped implant having dimensions of about 130µm × about 180 µm × about 1,500 µm. In some embodiments, the implant has dimensions within 10%, 7.5%, 5%, 2.5%, 2%, 1.5%, 1%, 0.5%, 0.25%, or 0.1% of the aforementioned 130 µm × about 180 µm × about 1,500 µm.

In some embodiments, the implant is a rod-shaped implant having dimensions of about 225 µm × about 225 µm × about 2,925 µm. In some embodiments, the implant has dimensions within 10%, 7.5%, 5%, 2.5%, 2%, 1.5%, 1%, 0.5%, 0.25%, or 0.1% of the aforementioned 225 µm × about 225 µm × about 2,925 µm.

In some embodiments, the implant is a rod-shaped implant having dimensions of about 330 µm × about 330 µm × about 6,000 µm. In some embodiments, the implant has dimensions within 10%, 7.5%, 5%, 2.5%, 2%, 1.5%, 1%, 0.5%, 0.25%, or 0.1% of the aforementioned 330 µm × about 330 µm × about 6,000 µm.

In some embodiments, the implant is a rod-shaped implant having dimensions of about 300 µm × about 300 µm × about 6,000 µm. In some embodiments, the implant has dimensions within 10%, 7.5%, 5%, 2.5%, 2%, 1.5%, 1%, 0.5%, 0.25%, or 0.1% of the aforementioned 300 µm × about 300 µm × about 6,000 µm.

Further still, are disclosed pharmaceutical compositions for treating an ocular condition, comprising: A) a biocompatible polymer matrix; and B) at least one therapeutic agent homogenously dispersed within the polymer matrix. Another embodiment disclosed herein is a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 2,925 microns; a width within 10%, 7.5%, 5%, 2.5%, 2%, 1.5%, 1%, 0.5%, 0.25%, 0.1% of 225 microns; and a height within 10%, 7.5%, 5%, 2.5%, 2%, 1.5%, 1%, 0.5%, 0.25%, 0.1% of 225 microns.

Another embodiment disclosed herein is a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 6,000 microns; a width within 10%, 7.5%, 5%, 2.5%, 2%, 1.5%, 1%, 0.5%, 0.25%, 0.1% of 330 microns; and a height within 10%, 7.5%, 5%, 2.5%, 2%, 1.5%, 1%, 0.5%, 0.25%, 0.1% of 330 microns.

Another embodiment disclosed herein is a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 6,000 microns; a width within 10%, 7.5%, 5%, 2.5%, 2%, 1.5%, 1%, 0.5%, 0.25%, 0.1% of 300 microns; and a height within 10%, 7.5%, 5%, 2.5%, 2%, 1.5%, 1%, 0.5%, 0.25%, 0.1% of 300 microns.

Another embodiment disclosed herein is a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 1,500 microns; a width within 10%, 7.5%, 5%, 2.5%, 2%, 1.5%, 1%, 0.5%, 0.25%, 0.1% of 130 microns; and a height within 10%, 7.5%, 5%, 2.5%, 2%, 1.5%, 1%, 0.5%, 0.25%, 0.1% of 180 microns.

In certain embodiments, the disclosure presents a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 4,000 microns; a width within 10%, 7.5%, 5%, 2.5%, 2%, 1.5%, 1%, 0.5%, 0.25%, 0.1% of 225 microns; and a height within 10%, 7.5%, 5%, 2.5%, 2%, 1.5%, 1%, 0.5%, 0.25%, 0.1% of 225 microns.

In another aspect, taught herein is a pharmaceutical composition for treating an ocular condition, comprising: a biocompatible implant comprising at least one polymer; and a therapeutic agent homogenously dispersed within the at least one polymer; wherein the implant comprises: a length within 10%, 7.5%, 5%, 2.5%, 2%, 1.5%, 1%, 0.5%, 0.25%, 0.1% of 6,000 microns; a width within 10%, 7.5%, 5%, 2.5%, 2%, 1.5%, 1%, 0.5%, 0.25%, 0.1% of 400 microns; and a height within 10%, 7.5%, 5%, 2.5%, 2%, 1.5%, 1%, 0.5%, 0.25%, 0.1% of 400 microns.

In other embodiments, the disclosure provides a pharmaceutical composition for treating an ocular condition, comprising: a biocompatible implant comprising at least one polymer; and a therapeutic agent homogenously dispersed within the at least one polymer; wherein the implant comprises: a therapeutic agent weight percent within 10%, 7.5%, 5%, 2.5%, 2%, 1.5%, 1%, 0.5%, 0.25%, 0.1% of 25% of the implant overall weight; a weight percent of the at least one polymer within 10%, 7.5%, 5%, 2.5%, 2%, 1.5%, 1%, 0.5%, 0.25%, 0.1% of 75% of the implant overall weight.

In other embodiments, the disclosure provides a pharmaceutical composition for treating an ocular condition, comprising: a biocompatible implant comprising at least one polymer; and a therapeutic agent homogenously dispersed within the at least one polymer; wherein the implant comprises: a therapeutic agent weight percent within 10%, 7.5%, 5%, 2.5%, 2%, 1.5%, 1%, 0.5%, 0.25%, 0.1% of 50% of the implant overall weight; a weight percent of the at least one polymer within 10%, 7.5%, 5%, 2.5%, 2%, 1.5%, 1%, 0.5%, 0.25%, 0.1% of 50% of the implant overall weight.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 65% to about 85% w/w of the overall implant (e.g., about 70% to about 80% w/w) and the therapeutic agent comprises about 15% to about 35% w/w of the overall implant (e.g., about 20% to about 30% w/w). In aspects, the therapeutic agent is difluprednate and the polymer matrix comprises PEG polymers. In aspects, the PEG polymers are PEG 3,350 and PEG 100,000. In aspects, the ratio of PEG 3,350 to PEG 100,000 is 11%/89%. In some embodiments, the implant is a rod-shaped implant having dimensions of about 225 µm × about 225 µm × about 2,925 µm. In aspects, the implant comprises difluprednate in an amount of 24 to 56 µg per implant (e.g., 32 to 48 µg per implant). In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 40% to about 60% w/w of the overall implant and the therapeutic agent comprises about 40% to about 60% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG3,350 to PEG 100,000 is 11%/89%. In some embodiments, the implant is a rod-shaped implant having dimensions of about 300 µm × about 300 µm × about 6,000 µm. In aspects, the implant comprises difluprednate in an amount of 640 to 960 µg per implant. In aspects, the implant has a volume of 540,000,000 cubic microns ± 10%. In aspects, the implant is sized and structured to allow for administration in a 21 gauge needle or a 22 gauge needle. In aspects, the implant is designed for subconjunctival administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 40% to about 60% w/w of the overall implant and the therapeutic agent comprises about 40% to about 60% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG3,350 to PEG 100,000 is 11%/89%. In some embodiments, the implant is a rod-shaped implant having dimensions of about 330 µm × about 330 µm × about 6,000 µm. In aspects, the implant comprises difluprednate in an amount of 320 to 480 µg per implant. In aspects, the implant has a volume of 653,400,000 cubic microns ± 10%.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 40% to about 60% w/w of the overall implant and the therapeutic agent comprises about 40% to about 60% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG3,350 to PEG 100,000 is 11%/89%. In some embodiments, the implant is a rod-shaped implant having dimensions of about 300 µm × about 300 µm × about 6,000 µm. In aspects, the implant comprises difluprednate in an amount of 320 to 480 µg per implant. In aspects, the implant has a volume of 540,000,000 cubic microns ± 10%.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 85% to about 90% w/w of the overall implant and the therapeutic agent comprises about 10% to about 15% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG3,350 to PEG 100,000 is 11%/89%. In some embodiments, the implant is a rod-shaped implant having dimensions of about 225 µm × about 225 µm × about 2,925 µm. In aspects, the implant comprises difluprednate in an amount of 16 to 24 µg per implant. In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 92.5% to about 95% w/w of the overall implant and the therapeutic agent comprises about 5% to about 7.5% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG 3,350 to PEG 100,000 is 11%/89%. In some embodiments, the implant is a rod-shaped implant having dimensions of about 225 µm × about 225 µm × about 2,925 µm. In aspects, the implant comprises difluprednate in an amount of 8 to 12 µg per implant. In aspects, the implant has a volume of 148,078,125 cubic microns ± 10%. In aspects, the implant is sized and structured to allow for administration in a 27 gauge needle. In aspects, the implant is designed for intracameral administration.

In certain embodiments, the disclosure provides for an ocular implant comprising: A) a biocompatible polymer matrix; and B) at least one therapeutic agent homogenously dispersed within the polymer matrix, wherein the biocompatible polymer matrix comprises about 55% to about 70% w/w of the overall implant and the therapeutic agent comprises about 30% to about 45% w/w of the overall implant. In aspects, the therapeutic agent is difluprednate and the polymer matrix comprises PEG polymers. In aspects, the PEG polymers are PEG 3,350 and PEG 100,000. In aspects, the ratio of PEG3,350 to PEG 100,000 is 11%/89%. In some embodiments, the implant is a rod-shaped implant having dimensions of about 130 µm × about 180 µm × about 1,500 µm. In aspects, the implant comprises difluprednate in an amount of 16 to 24 µg per implant. In aspects, the implant has a volume of 35,100,000 cubic microns ± 10%. In aspects, the implant is sized and structured to allow for administration in a 25, 26, or 27 gauge needle. In aspects, the implant is designed for intracameral administration.

In an aspect, one, two, three, four, five, six, seven, eight, nine, or more implants are provided in the method and implanted. The plurality of implants may be implanted simultaneously into the eye of a patient, sequentially during the same procedure, or sequentially over a period of time during different procedures. In some aspects, a patient receives periodic implants such as weekly, monthly, bimonthly, quarterly, semi-annual, or yearly implants.

Another embodiment provided herein is a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 202,500,000 cubic microns.

Also provided is a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 960,000,000 cubic microns.

Also provided is a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 148,078,125 cubic microns.

Also provided is a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 653,400,000 cubic microns.

Another embodiment provides a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 540,000,000 cubic microns.

Another embodiment provides a method for treating or preventing an ocular condition, comprising: implanting at least one implant into an eye of a patient in need of treatment wherein each implant has a volume within 10%, 7.5%, 5%, 2.5%, 2%, 1.5%, 1%, 0.5%, 0.25%, 0.1% of 35,100,000 cubic microns.

In an aspect, the disclosure provides a method for preventing or treating post-operative inflammation and/or pain in an eye of a patient in need of treatment comprising: inserting at least one implant having a volume within 10%, 7.5%, 5%, 2.5%, 2%, 1.5%, 1%, 0.5%, 0.25%, 0.1% of 202,500,000 cubic microns and therapeutic agent content between about 20wt% and about 55wt%; or inserting at least one implant, having a volume within 10%, 7.5%, 5%, 2.5%, 2%, 1.5%, 1%, 0.5%, 0.25%, 0.1% of 540,000,000 cubic microns and therapeutic agent content between about 25wt% and about 55wt%; or inserting at least one implant, having a volume within 10%, 7.5%, 5%, 2.5%, 2%, 1.5%, 1%, 0.5%, 0.25%, 0.1% of 960,000,000 cubic microns and therapeutic agent content between about 25wt% and about 45wt%; or inserting at least one implant, having a volume within 10%, 7.5%, 5%, 2.5%, 2%, 1.5%, 1%, 0.5%, 0.25%, 0.1% of 148,078,125 cubic microns and therapeutic agent content between about 15wt% to about 35wt%, about 10wt% to about 15wt%, or about 5wt% to about 7.5wt%; or inserting at least one implant, having a volume within 10%, 7.5%, 5%, 2.5%, 2%, 1.5%, 1%, 0.5%, 0.25%, 0.1% of 653,400,000 cubic microns, or 540,000,000 cubic microns, and therapeutic agent content between about 40wt% to about 60wt%; or inserting at least one implant, having a volume within 10%, 7.5%, 5%, 2.5%, 2%, 1.5%, 1%, 0.5%, 0.25%, 0.1% of 35,100,000 cubic microns and therapeutic agent content between about 29.6wt% and about 44.4wt%;. In aspects, the post-operative inflammation and/or pain is managed for at least 2 days following insertion into the subconjunctival, or intracameral, region of the eye.

In an aspect, the disclosure provides a method for preventing or treating post-operative inflammation and/or pain in an eye of a patient undergoing an ocular surgical procedure, or who has undergone an ocular surgical procedure, comprising: inserting at least one implant having a volume within 10%, 7.5%, 5%, 2.5%, 2%, 1.5%, 1%, 0.5%, 0.25%, 0.1% of 202,500,000 cubic microns and therapeutic agent content between about 20wt% and about 55wt%; or inserting at least one implant, having a volume within 10%, 7.5%, 5%, 2.5%, 2%, 1.5%, 1%, 0.5%, 0.25%, 0.1% of 540,000,000 cubic microns and therapeutic agent content between about 25wt% and about 55wt%; or inserting at least one implant, having a volume within 10%, 7.5%, 5%, 2.5%, 2%, 1.5%, 1%, 0.5%, 0.25%, 0.1% of 960,000,000 cubic microns and therapeutic agent content between about 25wt% and about 45wt%; or inserting at least one implant, having a volume within 10%, 7.5%, 5%, 2.5%, 2%, 1.5%, 1%, 0.5%, 0.25%, 0.1% of 148,078,125 cubic microns and therapeutic agent content between about 15wt% to about 35wt%, about 10wt% to about 15wt%, or about 5wt% to about 7.5wt%; or inserting at least one implant, having a volume within 10%, 7.5%, 5%, 2.5%, 2%, 1.5%, 1%, 0.5%, 0.25%, 0.1% of 653,400,000 cubic microns, or 540,000,000 cubic microns, and therapeutic agent content between about 40wt% to about 60wt%; or inserting at least one implant, having a volume within 10%, 7.5%, 5%, 2.5%, 2%, 1.5%, 1%, 0.5%, 0.25%, 0.1% of 35,100,000 cubic microns and therapeutic agent content between about 29.6wt% and about 44.4wt%. In aspects, the post-operative inflammation and/or pain is managed for at least 2 days following insertion into the subconjunctival, or intracameral, region of the eye.

In embodiments, implants may have a volume of 35,100,000 cubic microns, 202,500,000 cubic microns, or 540,000,000 cubic microns, or 960,000,000 cubic microns, or 148,078,125 cubic microns, or 653,400,000 cubic microns. In some embodiments, the volume from implant to implant may vary by about 0.1% to about 10%. The disclosure provides for compositions comprising the implants, kits comprising the implants, methods of utilizing the aforementioned implants, and systems comprising the implants with the stated cubic micron volumes.

In certain embodiments, the aforementioned polymer matrix excludes other polymers from being present in the composition. For instance, in some aspects PLGA is not present. In other aspects, the aforementioned implants only comprise PEG polymers and exclude other polymers from being present. In some embodiments, hot melt extrusion is not used to fabricate the implants. In some embodiments, in-situ gelation is not utilized to fabricate the implants.

In certain embodiments, the pharmaceutical formulations exclude implants that are not of the following volumes: 202,500,000 ± 10% cubic microns, or 540,000,000 ± 10% cubic microns, or 960,000,000 ± 10% cubic microns, or 148,078,125 ± 10% cubic microns, or 653,400,000 ± 10% cubic microns, or 35,100,00 ± 10% cubic microns. Some embodiments exclude implants that are not of the following dimensions: about 130 µm × about 180 µm × about 1,500 µm; about 225 µm × about 225 µm × about 4,000 µm, or about 300 µm × about 300 µm × about 6,000 µm, or about 400 µm × about 400 µm × about 6,000 µm, or about 225 µm × about 225 µm × about 2,925 µm, or about 330 µm × about 330 µm × about 6,000 µm. Some embodiments taught herein exclude implants that are not fabricated in a mold based method, such as for example by PRINT^{®} technology fabrication.

In some aspects, the disclosure provides a method of prevention and/or treatment of post-operative inflammation and/or pain, in a human subject by administering-via subconjunctival injection, or intracameral injection-a solid, biocompatible, rod-shaped subconjunctival, or intracameral, implant to the subject.

In a particular aspect, the implants of the disclosure do not migrate substantially from their initial position. In other aspects, the implants may move substantially from their initial position.

In embodiments of the disclosed methods, post-operative inflammation in a human is controlled for at least 2 days, or 7 days, or 10 days, or 14 days, or 20 days, or 24 days, or in aspects at least 28 days, following implantation of at least one implant, via subconjunctival injection, or intracameral injection, to the eye of a patient. The implants may comprise a therapeutic agent content ranging from about: 1 to 1200 µg per implant, 1 to 1000 µg per implant, 1 to 800 µg per implant, 1 to 500 µg per implant, 1 to 400 µg per implant, 1 to 300 µg per implant, 1 to 200 µg per implant, 1 to 150 µg per implant, 1 to 140 µg per implant, 1 to 130 µg per implant, 1 to 120 µg per implant, 1 to 110 µg per implant, 1 to 100 µg per implant, 1 to 90 µg per implant, 1 to 80 µg per implant, 1 to 70 µg per implant, 1 to 60 µg per implant, 1 to 50 µg per implant, 1 to 40 µg per implant, 1 to 30 µg per implant, 1 to 20 µg per implant, 1 to 10 µg per implant, or 1 to 8 µg per implant. The implants may comprise therapeutic agent content ranging from about: about 8 to about 480 µg per implant, about 8 to about 12 µg per implant, about 16 to about 24 µg per implant, about 32 to 48 µg per implant, about 24 to about 56 µg per implant, or about 320 to about 480 µg per implant. In some embodiments, the drug is difluprednate. It should be appreciated that the dose of the therapeutic agent in a given implant composition may be selected to be between the ranges given herein; however, once the dose is selected, the dose between implants for that application is substantially consistent, wherein the dose between implants varies less than 10%, less than 5%, less than 2%, less than 1%, less than 0.5% or less than 0.1% on a weight percentage basis.

In some aspects, an implant formulated for intracameral placement may comprise a therapeutic agent concentration of about 20 µg to about 60 µg ± 10 µg per implant, or may comprise a therapeutic agent concentration of about 24 µg to about 56 µg ±10 µg per implant, or may comprise a therapeutic agent concentration of about 40 µg ±10 µg per implant, or may comprise a therapeutic agent concentration of about 8 µg to about 56 µg ±10 µg per implant, or may comprise a therapeutic agent concentration of about 8 µg to about 12 µg ±10 µg per implant, or may comprise a therapeutic agent concentration of about 16 µg to about 24 µg ±10 µg per implant, or may comprise a therapeutic agent concentration of about 24 µg to about 56 µg ±10 µg per implant..

In some aspects, an implant formulated for subconjunctival placement may comprise a therapeutic agent concentration of about 300 µg to about 500 µg ±10 µg per implant, or may comprise a therapeutic agent concentration of about 320 µg to about 480 µg ±10 µg per implant, or may comprise a therapeutic agent concentration of about 400 µg ±10 µg per implant.

Also disclosed is a pharmaceutical composition, comprising: at least one ocular implant, wherein said at least one ocular implant has a volume of 33,750,000 ± 10% cubic microns; and comprises: A) a biocompatible polymer matrix; and B) at least one therapeutic agent homogenously dispersed within the polymer matrix.

Further provided by the present disclosure is a pharmaceutical composition, comprising: at least one ocular implant, wherein said at least one ocular implant has a volume of 148,078,125 ± 10% cubic microns; and comprises: A) a biocompatible polymer matrix; and B) at least one therapeutic agent homogenously dispersed within the polymer matrix.

Further provided by the present disclosure is a pharmaceutical composition, comprising: at least one ocular implant, wherein said at least one ocular implant has a volume of 653,400,000 ± 10% cubic microns; and comprises: A) a biocompatible polymer matrix; and B) at least one therapeutic agent homogenously dispersed within the polymer matrix.

Further provided by the present disclosure is a pharmaceutical composition, comprising: at least one ocular implant, wherein said at least one ocular implant has a volume of 540,000,000 ± 10% cubic microns; and comprises: A) a biocompatible polymer matrix; and B) at least one therapeutic agent homogenously dispersed within the polymer matrix.

Some embodiments entail administering one ocular implant having a volume of 202,500,000 ± 10% cubic microns to an eye. Other embodiments entail administering two ocular implants each having a volume of 202,500,000 ± 10% cubic microns to an eye. Yet other embodiments entail administering three ocular implants each having a volume of 202,500,000 ± 10% cubic microns to an eye. Yet other embodiments entail administering three or more ocular implants each having a volume of 202,500,000 ± 10% cubic microns to an eye. In some embodiments, each of the aforementioned ocular implants having a volume of 202,500,000 ± 10% cubic microns contains a difluprednate content of from 35 µg to 125 µg.

Some embodiments entail administering one ocular implant having a volume of 148,078,125 ± 10% cubic microns to an eye. Other embodiments entail administering two ocular implants each having a volume of 148,078,125 ± 10% cubic microns to an eye. Yet other embodiments entail administering three ocular implants each having a volume of 148,078,125 ± 10% cubic microns to an eye. Yet other embodiments entail administering three or more ocular implants each having a volume of 148,078,125 ± 10% cubic microns to an eye. In some embodiments, each of the aforementioned ocular implants having a volume of 148,078,125 ± 10% cubic microns contains a difluprednate content of from about 8 µg to about 56 µg. In some aspects, the implants comprise about 40ug difluprednate. In aspects which entail administering two ocular implants to an eye, said implants comprise from about 8 µg to about 56 µg (*e.g.,* about 40µg) difluprednate per implant, and the amount of difluprednate administered to the eye is from 16 µg to about 112 µg (*e.g.,* about 80 µg). In aspects which entail administering three ocular implants to an eye, said implants comprise from about 8 µg to about 56 µg (*e.g.,* about 40µg) difluprednate per implant, and the amount of difluprednate administered to the eye is about from 32 µg to about 168 µg (*e.g.,* about 121 µg). In aspects, the implant is sized and structured to allow for administration in a 27 gauge needle.

Some embodiments entail administering one ocular implant having a volume of 653,400,000 ± 10% cubic microns to an eye. Other embodiments entail administering two ocular implants each having a volume of 653,400,000 ± 10% cubic microns to an eye. Yet other embodiments entail administering three ocular implants each having a volume of 653,400,000 ± 10% cubic microns to an eye. Yet other embodiments entail administering three or more ocular implants each having a volume of 653,400,000 ± 10% cubic microns to an eye. In some embodiments, each of the aforementioned ocular implants having a volume of 653,400,000 ± 10% cubic microns contains a difluprednate content of from about 200 µg to about 500 µg. In some aspects, the implants comprise about 390 µg difluprednate.

Some embodiments entail administering one ocular implant having a volume of 540,000,000 ± 10% cubic microns to an eye. Other embodiments entail administering two ocular implants each having a volume of 540,000,000 ± 10% cubic microns to an eye. Yet other embodiments entail administering three ocular implants each having a volume of 540,000,000 ± 10% cubic microns to an eye. Yet other embodiments entail administering three or more ocular implants each having a volume of 540,000,000 ± 10% cubic microns to an eye. In some embodiments, each of the aforementioned ocular implants having a volume of 540,000,000 ± 10% cubic microns contains a difluprednate content of from about 200 µg to about 500 µg. In some aspects, the implants comprise about of from 390 µg to about 480 µg difluprednate. In aspects which entail administering two ocular implants to an eye, said implants comprise about of from 390 µg to about 480 µg (*e.g.,* 402.5 µg) difluprednate per implant, and the amount of difluprednate administered to the eye is from about 780 µg to about 960 µg (*e.g.,* 805 µg). In aspects which entail administering three ocular implants to an eye, said implants comprise from about 90 µg to about 480 µg (*e.g.,* about 402.5 µg) difluprednate per implant, and the amount of difluprednate administered to the eye is 1170 µg to about 2880 µg (*e.g.,* about 1207.5 µg). In aspects, the implant is sized and structured to allow for administration in a 21 gauge needle or a 22 gauge needle.

Some embodiments entail administering one ocular implant having a volume of 35,100,000 ± 10% cubic microns to an eye. Other embodiments entail administering two ocular implants each having a volume of 35,100,000 ± 10% cubic microns to an eye. Yet other embodiments entail administering three ocular implants each having a volume of 35,100,000 ± 10% cubic microns to an eye. Yet other embodiments entail administering three or more ocular implants each having a volume of 35,100,000 ± 10% cubic microns to an eye. In some embodiments, each of the aforementioned ocular implants having a volume of 35,100,000 ± 10% cubic microns contains a difluprednate content of from about 16 µg to about 24 µg. In some aspects, the implants comprise about 20 µg difluprednate per implant. In aspects, the implant is sized and structured to allow for administration in a 24 or 25 gauge needle.

Some embodiments entail administering one ocular implant having a volume of 960,000,000 ± 10% cubic microns to an eye. Other embodiments entail administering two ocular implants each having a volume of 960,000,000 ± 10% cubic microns to an eye. Yet other embodiments entail administering three ocular implants each having a volume of 960,000,000 ± 10% cubic microns to an eye. Yet other embodiments entail administering three or more ocular implants each having a volume of 960,000,000 ± 10% cubic microns to an eye. In some embodiments, each of the aforementioned ocular implants having a volume of 960,000,000 ± 10% cubic microns contains a difluprednate content of from 200 µg to 650 µg,

Some embodiments taught herein provide a biocompatible ocular implant, comprising: at least one therapeutic agent that is homogeneously dispersed within a biocompatible polymer matrix; wherein said biocompatible ocular implant is formulated to release a therapeutically effective amount of the at least one therapeutic agent for a period of time of at least about 2 days upon administration to a patient; and wherein said at least one biocompatible ocular implant demonstrates an in vitro release profile as set forth in the present disclosure. Other embodiments taught herein provide a biocompatible ocular implant, comprising: at least one therapeutic agent that is homogeneously dispersed within a biocompatible polymer matrix; wherein said biocompatible ocular implant is formulated to release a therapeutically effective amount of the at least one therapeutic agent for a period of time up to about 2 days upon administration to a patient, at which point approximately 100% of the at least one therapeutic agent will have been released; and wherein said at least one biocompatible ocular implant demonstrates an in vitro release profile as taught herein.

In particular embodiments, the disclosure provides for biocompatible ocular implants that demonstrate an in vivo release profile corresponding to the in vitro release profiles depicted by the implants of the **FIGs.**

In particular embodiments, the disclosure provides for biocompatible ocular implants that demonstrate PK characteristics corresponding to the PK characteristics depicted by the implants of the **FIGs.**

In particular embodiments, the disclosure provides for biocompatible rapid release extended treatment ocular implants.

Further, the disclosure provides for polymer matrix compositions that demonstrate the release profiles illustrated in any of the disclosed figures or tables, or release profiles that are mathematically derivable from the data in said figures and tables.

In some aspects, the ocular implant is formulated for prevention and/or treatment of post-operative inflammation. In some aspects, the ocular implant is formulated for prevention of post-operative inflammation. In some aspects, the ocular implant is formulated for treating post-operative inflammation, allergy treatment or prevention, pain treatment or prevention, combinations thereof or the like.

In one embodiment, the ocular implant is sized and structured to allow for implantation of the implant into the subconjunctival region of a human eye.

In one embodiment, the ocular implant is sized and structured to allow for implantation of the implant into the intracameral region of a human eye.

In some aspects, the ocular implant is formulated to not increase in size by more than 5% during the entire period between initial administration to a patient and degradation or dissolution of the implant.

In some aspects, the ocular implant is sized and structured to allow for administration with a 19 to 30 gauge needle. In an aspect, the implant is sized and structured to allow for administration with a 21 gauge needle. In an aspect, the implant is sized and structured to allow for administration with a 22 gauge needle. In an aspect, the implant is sized and structured to allow for administration with a 24 gauge needle. In an aspect, the implant is sized and structured to allow for administration with a 25 gauge needle. In an aspect, the implant is sized and structured to allow for administration with a 26 gauge needle. In an aspect, the implant is sized and structured to allow for administration with a 27 gauge needle. The needle may have walls that are normally dimensioned, thin walls, or ultrathin walls.

In an aspect, the implant is sized and structured to allow for intracameral placement into a human eye via administration with a 27 gauge needle.

In an aspect, the implant is sized and structured to allow for subconjunctival placement into a human eye via administration with 21 or 22 gauge needles.

A particular aspect of the present disclosure is that the present implants can be designed with a size and shape to maximize needle fit and minimize needle gauge. In particular embodiments, the inner diameter of the needle is not more than between about 5-50 microns larger than the maximum cross-sectional dimension of the implant.

In an embodiment, the inner diameter of the needle is not more than about 15 micrometers larger than the maximum cross-sectional dimension of the implant.

In some embodiments, the implant and needle form a kit, where the implants are designed to have a maximum cross-sectional dimension that is not more than about 25, 20, 15, 10, or 5 micrometers smaller than the inner diameter of the needle.

In some embodiments, the implants are cylindrical in shape and the cross-sectional diameter of the cylindrical implant is not more than about 25, 20, 15, 10, or 5 microns smaller than the inner diameter of the needle.

In a particular embodiment, the ocular implant is manufactured by a process comprising: 1) providing a mold, wherein the mold comprises a plurality of recessed areas formed therein; 2) disposing a volume of liquid material in the plurality of recessed areas; 3) forming a plurality of substantially uniform implants; and 4) harvesting the implants from the patterned template, wherein each of said implants substantially mimics the recessed areas.

Some embodiments comprise a kit for administering a biocompatible ocular implant, comprising: (a) at least one biocompatible ocular implant; wherein said at least one biocompatible ocular implant comprises at least one therapeutic agent that is homogeneously dispersed within a biocompatible polymer matrix; and (b) a single use ocular implant applicator that comprises a needle or needlelike device. In some embodiments, the needle or needlelike device is 27 gauge or larger. In some aspects, the needle or needlelike device is 21 or 22 gauge. In some aspects, the needle or needlelike device is 25 or 26 gauge.

In some aspects, the implants produced according to the present disclosure exhibit a therapeutic agent release profile that has very low inter-implant variability. The therapeutic agent release profiles exhibited by some implants of the present disclosure are consistent across implants and demonstrate variation that is not statistically significant. Consequently, the drug release profiles demonstrated by embodiments of the implants exhibit coefficients of variation that are within a confidence interval and not biologically relevant.

In some aspects, the therapeutic agent content amongst implants of a given configuration is highly consistent. In particular embodiments, the implants of the present disclosure possess a therapeutic agent content that does not vary significantly amongst implants of a specific configuration. In an embodiment, the therapeutic agent content of implants having a given configuration does not vary in a statistically significant manner from one another.

### BRIEF DESCRIPTION OF THE DRAWINGS

**FIG. 1A** and **FIG. 1B** show release profiles for a series of implants tested under in vitro conditions. **FIG. 1A** shows the percent difluprednate released as a function of time (days). **FIG. 1B** shows the release rate (µg/day) as a function of time (days).
**FIG. 2A to 2F** show release profiles for a series of implants tested under in vitro conditions. **FIG. 2A** shows the percent difluprednate released as a function of time (days). **FIG. 2B** shows the percent difluprednate released as a function of time (days). **FIG. 2C** shows the release rate (µg/day) as a function of time (days). **FIG. 2D** shows the release rate (µg/day) as a function of time (days). **FIG. 2E** shows the cumulative difluprednate released (µg) as a function of time (days). **FIG. 2F** shows the cumulative difluprednate released (µg) as a function of time (days).
**FIG. 3** shows the Hackett-McDonald Ocular Exam Score as a function of time (days) for an in vivo animal study.
**FIG. 4** shows the difluprednate (µg) present in retrieved implants as a function of time (days).
**FIG. 5A to 5C** shows release profiles for a series of implants tested under in vitro conditions. **FIG. 5A** shows the percent difluprednate released as a function of time (days). **FIG. 5B** shows the release rate (µg/day) as a function of time (days). **FIG. 5C** shows the cumulative difluprednate released (µg) as a function of time (days).
**FIG. 6A to 6C** show release profiles for a series of implants tested under in vitro conditions. **FIG. 6A** shows the percent difluprednate released as a function of time (days). **FIG. 6B** shows the release rate (µg/day) as a function of time (days). **FIG. 6C** shows the cumulative difluprednate released (µg) as a function of time (days).
**FIG. 7** shows the Hackett-McDonald Ocular Exam Score as a function of time (days) for an in vivo animal study.
**FIG. 8** shows the Hackett-McDonald Ocular Exam Score as a function of time (days) for an in vivo animal study.
**FIG. 9** illustrates the effect of subconjunctival ENV905 in a corneal incision/paracentesis rabbit model of post-operative inflammation.
**FIG. 10** illustrates the effect of subconjunctival ENV905 in a corneal incision/paracentesis rabbit model of post-operative inflammation.
**FIG. 11** illustrates the effect of subconjunctival ENV905 in a phacoemulsification/paracentesis rabbit model of post-operative inflammation.
**FIG. 12** illustrates the effect of intracameral ENV905 in a corneal incision/paracentesis rabbit model of post-operative inflammation.
**FIG. 13** illustrates the effect of intracameral delivery in a phacoemulsification/paracentesis rabbit model of post-operative inflammation.
**FIG. 14** illustrates the Total Hackett-McDonald Ophthalmic Exam Score (Mean ± SD) for all intracameral implants tested in an in vivo study.
**FIG. 15** illustrates the Total Hackett-McDonald Ophthalmic Exam Score (Mean ± SD) for all intracameral and subconjunctival implants tested in an in vivo study.
**FIG. 16** illustrates the Total Hackett-McDonald Ophthalmic Exam Scores from a non-GLP tolerability study.
**FIG. 17** illustrates plasma Desacetyl Difluprednate (DFB) levels in rabbits after IC administration of ENV905-1 and SCJ administration of ENV905-2.
**FIG. 18** illustrates Difluprednate (DFBA) and Desacetyl Difluprednate (DFB) levels in various ocular matrixes collected in rabbits after IC administration of ENV905-1, including: aqueous humor, bulbar conjunctiva, cornea (enriched with trabecular meshwork), iris/ciliary body, and retina.
**FIG. 19** illustrates Difluprednate (DFBA) and Deascetyl Difluprednate (DFB) levels in various ocular matrixes collected in rabbits after SCJ administration of ENV905-2, including: aqueous humor, bulbar conjunctiva, subconjunctival dose site; cornea (enriched with trabecular meshwork), iris/ciliary body, and retina.
**FIG. 20** illustrates the Total Hackett-McDonald Ophthalmic Exam Score (Mean ± SEM) for the compositions tested in the ENV905-PRE-008 study over 30 days. DUREZOL^{®} (0.05%), ENV905-1 (placebo), ENV905-1 (difluprednate), ENV905-3 (difluprednate), and ENV905-4 (difluprednate) were evaluated in this study.
**FIG. 21** illustrates Deascetyl Difluprednate (DFB) levels in blood plasma (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 22A** illustrates Difluprednate levels in the cornea (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 22B** illustrates Desacetyl Difluprednate (DFB) levels in the cornea (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 23A****.** illustrates Difluprednate (DFB) levels in the trabecular meshwork (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 23B****.** illustrates Desacetyl Difluprednate (DFB) levels in the trabecular meshwork (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 24A****.** illustrates Desacetyl Difluprednate (DFB) levels in the iris/ciliary body (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 24B****.** illustrates Desacetyl Difluprednate (DFB) levels in the iris/ciliary body (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 25A** illustrates the central corneal thickness data (% change from baseline) in rabbits from a non-GLP tolerability study measured after injection, and after administration of ENV905-1 (placebo), ENV905-1 (difluprednate), ENV905-5 (placebo), and ENV905-5 (difluprednate).
**FIG. 25B** illustrates the inferior corneal thickness data (% change from baseline) in rabbits from a non-GLP tolerability study measured after injection, and after administration of ENV905-1 (placebo), ENV905-5 (placebo), and ENV905-5 (difluprednate).
**FIG. 26** illustrates systemic exposure to Deascetyl Difluprednate (DFB) for ENV905-1 (44.0 µg/eye) compared to DUREZOL^{®}.
**FIG. 27** illustrates exposure to Deascetyl Difluprednate (DFB) in the Iris/Ciliary Body for ENV905-1 (44.0 µg/eye) compared to DUREZOL^{®}.
**FIG. 28** illustrates exposure to Desacetyl Difluprednate (DFB) in the Trabecular Meshwork for ENV905-1 (44.0 µg/eye) compared to DUREZOL^{®}.
**FIG. 29** illustrates intraocular pressure measured overtime for ENV905-1 (without difluprednate), ENV905-1 (low; 1 implant per eye), ENV905-1 (mid; 2 implants per eye), and ENV905-1 (3 implants per eye).
**FIG. 30** Deascetyl Difluprednate (DFB) pressure measured overtime for ENV905-1 (without difluprednate), ENV905-1 (1 implant per eye), ENV905-1 (2 implants per eye), and ENV905-1 (3 implants per eye).
**FIG. 31A** illustrates Difluprednate (DFB) levels in bulbar conjunctiva (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 31B** illustrates Desacetyl Difluprednate (DFB) levels in bulbar conjunctiva (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 32A** illustrates Difluprednate levels in retina (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 32B** illustrates Desacetyl Difluprednate (DFB) levels in retina (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 33A** illustrates Difluprednate levels in aqueous humor (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 33B** illustrates Desacetyl Difluprednate (DFB) levels in aqueous humor (ng/mL) for the compositions tested in the ENV905-ADME-002 study over 28 days. DUREZOL^{®} (0.05%), ENV905-1 (1x implant), ENV905-1 (2x implant), and ENV905-1 (3x implant) were evaluated in this study.
**FIG. 34** illustrates the central corneal thickness data (µm) in rabbits from a toxicity study measured after administration of ENV905-1 (placebo) and ENV905-1 (active).

### DETAILED DESCRIPTION

Provided herein are pharmaceutical compositions for treating or preventing an ocular condition. In embodiments, the pharmaceutical composition comprises: a biocompatible polymer matrix and a therapeutic agent, which is included in the polymer matrix. The therapeutic agent may be dispersed homogeneously throughout the polymer matrix.

As described herein, multiple pharmaceutical compositions have been fabricated and/or contemplated in the form of an implant, resulting in highly effective pharmaceutically active products, including ocular therapeutic treatments, including: (1) sustained release ocular implants, as well as (2) immediate/rapid delivery and extended treatment ocular implants.

In various embodiments, these pharmaceutical compositions include a therapeutic agent dispersed throughout a polymer matrix formed into an ocular implant. In some aspects, the ocular implant degrades slowly in a human eye and gradually, or in a sustained way, releases the active therapeutic agent. In other aspects, the ocular implant dissolves immediately upon being exposed to the physiological conditions of an eye, and the active therapeutic agent is available to treat the loci for an extended amount of time.

In a particular embodiment, the pharmaceutical composition of the present disclosure comprises: i) a biocompatible polymer or polymers, and ii) a therapeutic agent such as, for example, a drug effective for use in the prevention and/or treatment of an ocular condition, such as post-operative inflammation and/or pain.

### Definitions

"About" means plus or minus a percent (e.g., ± 10% or ± 5%) of the number, parameter, or characteristic so qualified, which would be understood as appropriate by a skilled artisan to the scientific context in which the term is utilized. Furthermore, since all numbers, values, and expressions referring to quantities used herein, are subject to the various uncertainties of measurement encountered in the art, then unless otherwise indicated, all presented values may be understood as modified by the term "about."

As used herein, the articles "a," "an," and "the" may include plural referents unless otherwise expressly limited to one-referent, or if it would be obvious to a skilled artisan from the context of the sentence that the article referred to a singular referent.

Where a numerical range is disclosed herein, then such a range is continuous, inclusive of both the minimum and maximum values of the range, as well as every value between such minimum and maximum values. Still further, where a range refers to integers, every integer between the minimum and maximum values of such range is included. In addition, where multiple ranges are provided to describe a feature or characteristic, such ranges can be combined. That is to say that, unless otherwise indicated, all ranges disclosed herein are to be understood to encompass any and all subranges subsumed therein. For example, a stated range of from "1 to 10" should be considered to include any and all subranges between the minimum value of 1 and the maximum value of 10. Exemplary subranges of the range "1 to 10" include, but are not limited to, 1 to 6.1, 3.5 to 7.8, and 5.5 to 10.

As used herein, the term "polymer" is meant to encompass both homopolymers (polymers having only one type of repeating unit) and copolymers (a polymer having more than one type of repeating unit).

"Biodegradable polymer" means a polymer or polymers, which degrade in vivo, under physiological conditions. The release of the therapeutic agent occurs concurrent with, or subsequent to, the degradation of a biodegradable polymer over time.

The terms "biodegradable" and "bioerodible" are used interchangeably herein. A biodegradable polymer may be a homopolymer, a copolymer, or a polymer comprising more than two different polymeric units.

As used herein, the term "biocompatible" means a material, such as a polymer, that is compatible with living tissue. Biocompatible polymers may be biodegradable, bioerodible, or dissolvable.

As used herein, the term "polymer matrix" refers to a homogeneous mixture of polymers. In other words, the polymer matrix does not include a mixture of polymers wherein one portion of the polymer matrix is different from the other portion by ingredient, density, and etc. For example, the polymer matrix does not include a composition containing a core and one or more outer layers, nor a composition containing a drug reservoir and one or more portions surrounding the drug reservoir. The mixture of polymers may be of the same type, e.g. two different PEG polymers, or of different types, e.g. PEG polymers combined with PCL polymers.

"Ocular condition" means a disease, ailment, or condition, which affects or involves the ocular region.

The term "hot-melt extrusion" or "hot-melt extruded" is used herein to describe a process, whereby a blended composition is heated and/or compressed to a molten (or softened) state and subsequently forced through an orifice, where the extruded product (extrudate) is formed into its final shape, in which it solidifies upon cooling.

The term "non-extruded implant" or "non-hot melt extruded implant" refers to an implant that was not manufactured in a process that utilizes an extrusion step.

As used herein, "drug release" refers to the release of the at least one therapeutic agent, or drug, from an implant. The drug release may be sustained release, controlled release, or rapid release.

"Sustained release" or "controlled release" refers to the release of at least one therapeutic agent, or drug, from an implant at a predetermined rate. Sustained release implies that the therapeutic agent is not released from the implant sporadically, in an unpredictable fashion. The term "sustained release" may include a "burst phenomenon" associated with deployment. In some example embodiments, an initial burst of at least one therapeutic agent may be desirable, followed by a more gradual release thereafter. The release rate may be steady state (commonly referred to as "timed release" or zero order kinetics), that is the at least one therapeutic agent is released in even amounts over a predetermined time (with or without an initial burst phase), or may be a gradient release.

In some embodiments, the ocular implant polymer matrix fully disintegrates, dissolves, dissociates, dissolutes, and/or otherwise completely diminishes, upon placement into the eye under normal physiological conditions. In these embodiments, the ocular implant is often termed a "rapid delivery vehicle" or "rapid release vehicle." The ocular implant fully, or nearly completely, dissolves upon placement into the eye and consequently releases the entirety of the therapeutic agent payload dispersed within said ocular implant polymer matrix. Consequently, in these embodiments, it is the pharmacokinetics associated with the therapeutic agent contained within the ocular implant that allows for the achievement of an extended treatment concentration of drug, in the surrounding tissues of the eye that prevents and/or treats a condition of the eye, such as for example: post-operative inflammation, allergy, pain, combinations thereof, and the like. For example, factors such as the therapeutic agent content, the water solubility of the therapeutic agent, the route of administration, and the physicochemical properties of the therapeutic agent all factor to control the "extended treatment" availability of the therapeutic agent to the loci of the eye. In some aspects, the physiochemical attributes of difluprednate, in combination with the rapid delivery ocular implant (e.g. a PEG based implant that immediately dissolves upon placement into an aqueous environment) and its subsequent subconjunctival or intracameral placement, leads to an extended treatment bioavailability of difluprednate for at least 2 days, or 7 days, or 14 days, or 20 days, or at least about 28 days in an eye.

"Therapeutically effective amount" means a level or amount of a therapeutic agent needed to treat an ocular condition; or the level or amount of a therapeutic agent that produces a therapeutic response or desired effect in the subject to which the therapeutic agent was administered. Thus, a therapeutically effective amount of a therapeutic agent, such as a difluprednate, is an amount that is effective in reducing at least one symptom of an ocular condition. Achieving a therapeutically effective amount may be achieved through sustained release of a therapeutic agent from an implant or through extended treatment when a therapeutic agent is rapidly released from an implant and the therapeutic agent provides extended treatment over time.

As used herein, the term "baseline" refers to a proper reference measurement established prior to surgery. The baseline measurement can be obtained by any suitable method. In some embodiments, "baseline" refers to the thickness of the cornea prior to surgery. In other embodiments, "baseline" refers to the thickness of the cornea prior to administration of an ocular implant. In still other embodiments, "baseline" refers intraocular pressure measured prior to surgery. In yet other embodiments, "baseline" refers to the intraocular pressure of a healthy eye.

As used herein, PEG is used to represent poly(ethylene glycol) of all molecular weights including polyethylene oxides.

### Ocular Anatomy

In particular embodiments, the implants described herein are subconjunctival implants manufactured for placement into the subconjunctival region of the human eye.

In particular embodiments, the implants described herein are intracameral implants manufactured for placement into the intracameral region of the human eye.

In certain embodiments, the steady and sustained release of therapeutic agent from the implant achieves a concentration of drug in the surrounding tissues of the eye that prevents and/or treats a condition of the eye, such as for example post-operative inflammation, allergy, pain, combinations thereof, and the like.

Therapeutic agents for the treatment of ocular inflammation are typically administered to the eye in solution-based formulations. For example, DUREZOL^{®} is an emulsion which is applied topically to the eye as eye drops, and U.S. Patent Application Publication 2016/0120879 describes injectable suspensions which are administered into the anterior chamber of the eye. However, such solution-based formulations suffer from imprecise localization of the active at the site of inflammation, which reduces efficacy. Additionally, injectable suspensions can result in adverse events, such as blurred vision, due to localization of the suspension near the cornea. Further, the therapeutic agents contained within the suspension can polymerize after administration, which also reduces efficacy.

In contrast to solution-based formulations, the implants disclosed herein are formulated to remain localized at the site of administration, which improves efficacy and reduces the incidence of adverse events associated with translocation of injectable suspensions. The implants disclosed herein are formulated to prevent aggregation of the therapeutic agents. Further, the implants exhibit improved dose uniformity compared to solution-based formulations.

### Biocompatible Polymers

In certain embodiments, the implants described herein are engineered to provide maximal approximation of the implant to the human eye. In certain embodiments, the implants are made of polymeric materials.

In embodiments, the polymer materials used to form the implants described herein are biodegradable. In embodiments, the polymer materials may be any combination of polylactic acid, glycolic acid, polycaprolactone (PCL), and co-polymers thereof, which provides sustained-release of the therapeutic agent into the eye over time.

Suitable polymeric materials or compositions for use in the implants include those materials which are compatible, which is biocompatible, with the eye so as to cause no substantial interference with the functioning or physiology of the eye. Such polymeric materials may be biocompatible, biodegradable, or bioerodible In particular embodiments, examples of useful polymeric materials include, without limitation, such materials derived from and/or including organic esters and organic ethers, which when degraded result in physiologically acceptable degradation products. Also, polymeric materials derived from and/or including, anhydrides, amides, orthoesters and the like, by themselves or in combination with other monomers, may also find use in the present disclosure. The polymeric materials may be addition or condensation polymers. The polymeric materials may be cross-linked or non-cross-linked. For some embodiments, besides carbon and hydrogen, the polymers will include at least one of oxygen and nitrogen. The oxygen may be present as oxy, e.g. hydroxy or ether, carbonyl, e.g. non-oxo-carbonyl, such as carboxylic acid ester, and the like. The nitrogen may be present as amide, cyano and amino.

In embodiments, the implant polymer matrix is selected from: (1) PEG with no other polymers mixed in (disclosed in further detail herein) to form a rapid release delivery vehicle, meaning, less than about, 12 hours, 8 hours, 4 hours, 2 hours, or 1 hour for the implant to dissolve and release its active cargo; (2) PEG/PLGA mixed polymer matrix particles (disclosed in further detail herein) that give a longer release and degradation profile for the implants, which extend for up to 1 day, 2 days, 5 days, 10 days, and 14 days; and (3) PEG/PCL mixed polymer matrix particles (disclosed in further detail herein), which extend release for more than 14 days, 20 days, or 28 days.

In one embodiment, polymers of hydroxyaliphatic carboxylic acids, either homopolymers or copolymers, and polysaccharides are useful in the implants. Polyesters can include polymers of D-lactic acid, L-lactic acid, racemic lactic acid, glycolic acid, polycaprolactone, co-polymers thereof, and combinations thereof.

Some characteristics of the polymers or polymeric materials for use in embodiments of the present disclosure may include biocompatibility, compatibility with the selected therapeutic agent, ease of use of the polymer in making the therapeutic agent delivery systems described herein, a desired half-life in the physiological environment, and hydrophilicity.

In one embodiment, the biocompatible polymer matrix used to manufacture the implant is a polyether, for example a polyethylene glycol (PEG). PEGs are prepared by polymerization of ethylene oxide and are available in a wide range of molecular weights. PEG has found medical use as an excipient in pharmaceutical products, in lubricating eye drops, film coatings, and ointment bases. PEG may be synthesized at a variety of molecular weights. Used herein are PEGs with molecular weights that include 500-100,000,000 Da, 2,000 Da, 3,000 Da, 3,300 Da, 3,350 Da, 100,000 Da, 3,000 Da, 35,000 Da, 100,000 Da, and 1,000,000 Da. In some aspects, PEGs with molecular weights from 500 to 1,000,000 Daltons are used. PEG is represented by the general structure (1).

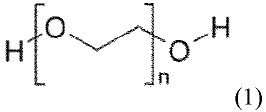

In one embodiment utilizing PEGs, the ocular implant formulations will be comprised of micronized difluprednate, the active ingredient, and a blend of two PEGs with different average molecular weights, the inactive ingredients. The PEGs will have an average molecular weight of 100,000 daltons (Da) (PEG 100K) and 3,350 Da (PEG 3,350). These water-soluble PEGs function as both a diluent and a binder for the micronized difluprednate to form an injectable, solid implant formulation.

PEG 100K is a relatively large and hard polymer. Because of these physical characteristics, implants comprising PEG 100K as the sole polymer in a polymer matrix typically do not contain a homogenous dispersion of a therapeutic agent, and the amount of therapeutic agent between batches is not uniform. PEG 3,350 is a relatively small and soft polymer. PEG 3,350 is difficult to handle during processing, which typically results in non-uniform dosages of therapeutic agent between batches. However, the inventors discovered that the combination of PEG 100K and PEG 3,350, at the ratios disclosed herein, can in some aspects be critical to fabricating implants with dose uniformity.

In embodiments, the PEGs are long-chain polymers of ethylene oxide with a formula of OH-(CH₂-CH₂-O)ₙ-H, where n is the average number of oxyethylene groups present in the molecule. These polymers have been used extensively in approved products across multiple routes of administration including oral, topical, ophthalmic, rectal, vaginal, intrasynovial, intra-articular, intramuscular, and intravenous (Inactive Ingredients Database). PEGs are generally considered to have low toxicity by all routes of administration, and have been shown to be very safe in a large battery of tests using a large range of routes and molecular weights (Herold 1982, Smyth 1947, Smyth 1950, Smyth 1970, Webster 2009).

In one embodiment, the biocompatible polymer matrix used to manufacture the implant is a synthetic aliphatic polyester, for example a polycaprolactone. PCL is formed by ring opening polymerization of ε-caprolactone. Polycaprolactone degrades by hydrolysis of its ester linkages. Polycaprolactone has found use in medical devices such as sutures, tissue scaffold, or as dental splints. Polycaprolactone may be synthesized at a variety of molecular weights. Preferred molecular weights, in some aspects, include 2,000 Da, 14,000 Da, and 45,000 Da. In some aspects, PCL with a molecular weight of from 2000 to 45,000 Daltons are used. Polycaprolactone is represented by the general structure (2).

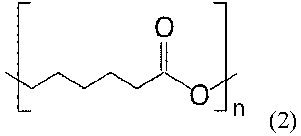

In one embodiment, the biocompatible polymer matrix used to manufacture the implant is a synthetic aliphatic polyester, for example, a polymer of lactic acid and/or glycolic acid, and includes poly-(D,L-lactide) (PLA), polyglycolic acid (PGA), and/or the copolymer poly-(D, L-lactide-co-glycolide) (PLGA).

PLGA and PLA polymers are known to degrade via backbone hydrolysis (bulk erosion) and the final degradation products are lactic and glycolic acids, which are non-toxic and considered natural metabolic compounds. Lactic and glycolic acids are eliminated safely via the Krebs cycle by conversion to carbon dioxide and water.

PLGA is synthesized through random ring-opening co-polymerization of the cyclic dimers of glycolic acid and lactic acid. Successive monomeric units of glycolic or lactic acid are linked together by ester linkages. The ratio of lactide to glycolide can be varied, altering the biodegradation characteristics of the product. By altering the ratio it is possible to tailor the polymer degradation time. Importantly, drug release characteristics are affected by the rate of biodegradation, molecular weight, and degree of crystallinity in drug delivery systems. By altering and customizing the biocompatible polymer matrix, the drug delivery profile can be changed.

PCL, PLA, PGA, and PLGA are cleaved predominantly by non-enzymatic hydrolysis of its ester linkages throughout the polymer matrix, in the presence of water in the surrounding tissues. PCL, PLA, PGA, and PLGA polymers are biocompatible, because they undergo hydrolysis in the body to produce the original monomers. Lactic and glycolic acids are nontoxic and eliminated safely via the Krebs cycle by conversion to carbon dioxide and water. The biocompatibility of PCL, PLA, PGA and PLGA polymers has been further examined in both non-ocular and ocular tissues of animals and humans. The findings indicate that the polymers are well tolerated.

Examples of PLA polymers, which may be utilized in an embodiment of the disclosure, include the RESOMER^{®} Product line available from Evonik Industries identified as, but are not limited to, R 207 S, R 202 S, R 202 H, R 203 S, R 203 H, R 205 S, R 208, R 206, and R 104 . Examples of suitable PLA polymers include both acid and ester terminated polymers with inherent viscosities ranging from approximately 0.15 to approximately 2.2 dL/g when measured at 0.1% w/v in CHCl₃ at 25°C with an Ubbelhode size 0c glass capillary viscometer.

The synthesis of various molecular weights of PLA is possible. In one embodiment, PLA, such as RESOMER^{®} R208S, with an inherent viscosity of approximately 1.8 to approximately 2.2 dl/g, can be used. In another embodiment, PLA, such as RESOMER^{®} R203S, with an inherent viscosity of approximately 0.25 to approximately 0.35 dl/g can be used

Resomer's R203S and R208S are poly(D,L-lactide) or PLA ester-terminated polymers with the general structure (3):

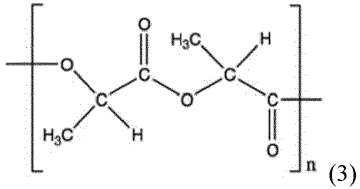

Examples of PLGA polymers, which may be utilized in an embodiment of the disclosure, include the RESOMER^{®} Product line from Evonik Industries, including RG 502, RG 502 H, RG 503, RG 503 H, RG 504, RG 504 H, RG 505, RG 506, RG 653 H, RG 752 H, RG 752 S, RG 753 H, RG 753 S, RG 755, RG 755 S, RG 756, RG 756 S, RG 757 S, RG 750 S, RG 858, and RG 858 S. Such PLGA polymers include both acid and ester terminated polymers with inherent viscosities ranging from approximately 0.14 to approximately 1.7 dl/g when measured at 0.1% w/v in CHCl₃ at 25°C with an Ubbelhode size 0c glass capillary viscometer. Example polymers used in various embodiments of the disclosure may include variation in the mole ratio of D,L-lactide to glycolide from approximately 50:50 to approximately 85:15, including, but not limited to, 50:50, 65:35, 75:25, and 85:15.

The synthesis of various molecular weights of PLGA with various D,L-lactide-glycolide ratios is possible. In one embodiment, PLGA, such as RESOMER^{®} RG 752 S, with an inherent viscosity of approximately 0.16 to approximately 0.24 dl/g can be used as the matrix material of the present implants.

Resomer RG752S is a poly(D,L-lactide-co-glycolide) or ester-terminated PLGA copolymer (lactide:glycolide ratio of 75:25) with the general structure (4):

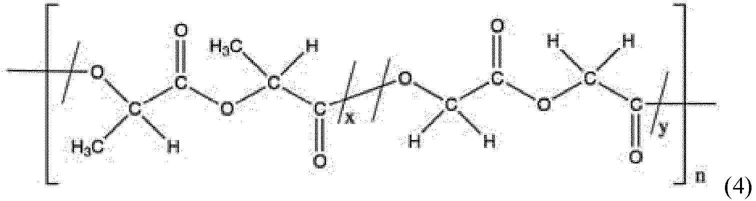

The polymers used to form the implants of the disclosure have independent properties associated with them that when combined provide the properties needed to provide release of a therapeutically effective amount.

A few of the primary polymer characteristics that control therapeutic agent release rates are: polymer solubility, the molecular weight distribution, polymer end group, for example , acid or ester, and the ratio of polymers and/or copolymers in the polymer matrix. The present disclosure provides examples of polymer matrices that possess desirable therapeutic agent release characteristics by manipulating one or more of the aforementioned properties to develop a suitable ocular implant.

### Drug Release Profile Manipulation

The rate of drug release from biocompatible implants depends on several factors. For example, the surface area of the implant, the therapeutic agent content, the water solubility of the therapeutic agent, and the speed of polymer degradation or dissolution.

The versatility of PCL, PEG, PGA, PLA, and PLGA allows for construction of delivery systems to tailor the drug release for treating a variety of front and back of the eye diseases.

When the versatility of PCL, PEG, PGA, PLA, and PLGA polymers are combined with the manufacturing techniques of the present disclosure, for example PRINT^{®} (Envisia Therapeutics Inc.) particle fabrication technology, then a host of custom tailored and highly consistent and predictable drug release profiles can be created, which were not possible based upon the technology of the prior art, such as for example extrusion.

That is, with the present mold based particle fabrication technology, implants can be manufactured that exhibit a drug release profile that has highly reproducible characteristics implant to implant. The drug release profiles exhibited by various implants of the present disclosure are consistent implant to implant and demonstrate variation that is not statistically significant. Consequently, the drug release profiles demonstrated by embodiments of the implants exhibit coefficients of variation that are within a confidence interval and not biologically relevant. The ability to produce implants that demonstrate such a high degree of consistent drug release is advancement over the state of the art.

### Drug Release Kinetics

Drug release is influenced by many factors including: polymer composition, therapeutic agent content, implant morphology, porosity, tortuosity, surface area, method of manufacture, and deviation from sink conditions, just to name a few. The present mold based manufacturing techniques-utilized in embodiments of the disclosure-are able to manipulate implant morphology, porosity, tortuosity, and surface area in ways that the prior art methods were incapable of doing. For instance, the highly consistent drug release profiles, highly consistent implant morphologies, and highly consistent homogeneous drug dispersions achievable by the present methods, were not available to prior art practitioners relegated to utilizing a heat extrusion based method of manufacture.

In general, therapeutic agent release occurs in 3 phases: (a) an initial burst release of therapeutic agent from the surface, (b) followed by a period of diffusional release, which is governed by the inherent dissolution of therapeutic agent (diffusion through internal pores into the surrounding media) and lastly, (c) therapeutic agent release associated with biodegradation of the polymer matrix. The rapid achievement of high therapeutic agent concentrations, followed by a longer period of continuous lower-dose release, makes such delivery systems ideally suited for acute-onset diseases that require a loading dose of therapeutic agent followed by tapering doses.

More recent advancements in drug delivery systems have allowed for biphasic release characteristics with an initial high (burst) rate of therapeutic agent release followed by sustained zero-order (linear) kinetic release (*i.e.,* therapeutic agent release rate from the polymer matrix is steady and independent of the therapeutic agent concentration in the surrounding milieu) over longer periods. In addition, when desired for treating chronic diseases such as elevated IOP, these therapeutic agent delivery systems can be designed to have steady state release following zero order kinetics from the onset.

In aspects, the mono-polymeric PEG implants of the present disclosure, however, do not follow the above bi and tri phase release profiles. The PEG implants of the present disclosure, in embodiments, are rapid release, where the PEG monomers disassociate with each other when exposed to biologic conditions and the therapeutic agent captured therein is released into the surrounding tissues.

### Therapeutic Agents

Suitable therapeutic agents for use in various embodiments of the disclosure may be found in the Orange Book published by the Food and Drug Administration, which lists therapeutic agents approved for treating post-operative inflammation.

In some embodiments, the therapeutic agents that can be used according to the disclosure include: steroids, corticosteroids, NSAIDs, allergy medications, antihistamines, antibiotics, pharmaceutically acceptable salts thereof, biologic molecules, and combinations thereof.

Suitable examples of the aforementioned steroids include, but are not limited to, 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, fluocinolone.

Suitable examples of the aforementioned corticosteroids include, but are not limited to, cortisone, prednisolone, fluorometholone, dexamethasone, difluprednate, medrysone, loteprednol, fluazacort, hydrocortisone, prednisone, betamethasone, prednisone, prednisolone, fluocinolone acetonide, methylprednisolone, triamcinolone hexacetonide, paramethasone acetate, diflorasone, fluocinonide, rimexolone, derivatives thereof, and mixtures thereof.

Suitable examples of the aforementioned NSAIDs include, but are not limited to, aspirin, diclofenac, flurbiprofen, ibuprofen, ketorolac, naproxen, bromfenac, and suprofen.

Suitable examples of the aforementioned allergy medications and antihistamines include, but are not limited to, loratadine, hydroxyzine, diphenhydramine, chlorpheniramine, brompheniramine, cyproheptadine, terfenadine, clemastine, triprolidine, carbinoxamine, diphenylpyraline, phenindamine, azatadine, tripelennamine, dexchlorpheniramine, dexbrompheniramine, methdilazine, and trimeprazine doxylamine, pheniramine, pyrilamine, chlorcyclizine, thonzylamine, and derivatives thereof.

Suitable examples of the aforementioned antibiotics include, but are not limited to, cefazolin, cephradine, cefaclor, cephapirin, ceftizoxime, cefoperazone, cefotetan, cefutoxime, cefotaxime, cefadroxil, ceftazidime, cephalexin, cephalothin, cefamandole, cefoxitin, cefonicid, ceforanide, ceftriaxone, cefadroxil, cephradine, cefuroxime, ampicillin, amoxicillin, cyclacillin, ampicillin, penicillin G, penicillin V potassium, piperacillin, oxacillin, bacampicillin, cloxacillin, ticarcillin, azlocillin, carbenicillin, methicillin, nafcillin, erythromycin, tetracycline, doxycycline, minocycline, aztreonam, chloramphenicol, ciprofloxacin hydrochloride, clindamycin, metronidazole, gentamicin, lincomycin, tobramycin, vancomycin, polymyxin B sulfate, colistimethate, colistin, azithromycin, augmentin, sulfamethoxazole, trimethoprim, and derivatives thereof.

In one embodiment, the implants of the present disclosure utilize dexamethasone, fluticasone, bromfenac sodium, loteprednol etabonate, or difluprednate.

In a particular embodiment, the implants of the present disclosure utilize difluprednate.

### Difluprednate

Difluprednate is a synthetic GR agonist, and a difluorinated derivative of prednisolone. Difluprednate may be provided in a micronized form in order to ensure consistent biopharmaceutical properties when incorporated into the final dosage form. The chemical structure of difluprednate is found below:

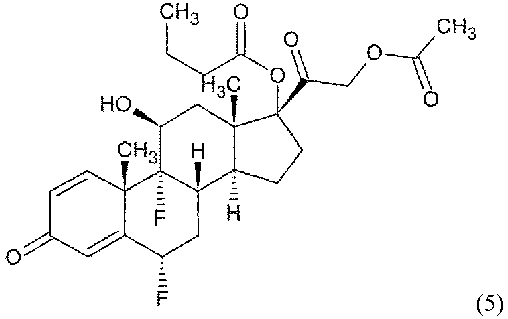

Difluprednate undergoes deacetylation in vivo to 6α, 9-difluoroprednisolone 17-butyrate (deacetyl difluprednate; DFB), an active metabolite of difluprednate.

### Pharmaceutical Compositions

In embodiments, the pharmaceutical composition is comprised of the biocompatible polymer matrix and at least one therapeutic agent.

The biocompatible polymer matrix is comprised of polymers meeting the desired characteristics. For example, desired characteristics may include a specific therapeutic agent release rate or a specific duration of action. The biocompatible polymer matrix may be comprised of one polymer, two polymers, or many polymers, such as three, four, five polymers, or more polymers.

In some embodiments, the compositions may comprise polymers utilizing the same monomer, such as compositions comprising various PEG homopolymers, or compositions comprising various PCL homopolymers. However, even if the polymers of the composition utilize the same monomer, the polymers may differ in other characteristics, such as, for example, inherent viscosity or molecular weight.

In other embodiments, the compositions may comprise polymers utilizing different monomers, such as compositions comprising a PEG homopolymer and a PCL homopolymer. However, even if the polymers of the compositions utilize different monomers, the polymers may be similar in other characteristics, such as for example, inherent viscosity or molecular weight.

In one embodiment, the pharmaceutical composition comprises a biocompatible polymer matrix and at least one therapeutic agent homogeneously dispersed throughout the polymer matrix. Further, the presently discussed pharmaceutical compositions comprising a biocompatible polymer matrix and at least one therapeutic agent, may, in certain embodiments, also exclude other polymers. That is, in some embodiments, the aforementioned polymer matrix only includes one polymer and active therapeutic agent.

In embodiments, the therapeutic agent is blended with the biocompatible polymer matrix to form the pharmaceutical composition. The amount of therapeutic agent used in the pharmaceutical composition depends on several factors such as: biocompatible polymer matrix selection, therapeutic agent selection, rate of release, duration of release desired, configuration of pharmaceutical composition, and ocular pharmacokinetics, to name a few.

For example, the therapeutic agent may comprise approximately 0.1 to approximately 60.0 weight percent of the pharmaceutical composition. In some embodiments, the therapeutic agent comprises approximately 5.0 to approximately 55.0 weight percent of the pharmaceutical composition. In other embodiments, the therapeutic agent comprises approximately 20.0 to approximately 55.0 weight percent of the pharmaceutical composition.

Implants having various compositions, sizes, and shapes were fabricated and tested as set forth in the disclosure; however, it will be appreciated that these are non-limiting examples of implant designs contemplated by the present disclosure.

### Fabrication of an Ocular Implant

Various methods may be used to produce the implants. Methods include, but are not limited to, solvent casting, phase separation, interfacial methods, molding, compression molding, injection molding, extrusion, co-extrusion, heat extrusion, die cutting, heat compression, and combinations thereof. In certain embodiments, the implants are molded.

In particular embodiments, the implants of the present disclosure are fabricated through the PRINT^{®} particle fabrication technology (Envisia Therapeutics Inc., North Carolina). In particular, the implants are made by molding the materials intended to make up the implants in polymeric mold cavities.

The molds can be polymer-based molds and the mold cavities can be formed into any desired shape and dimension. Uniquely, as the implants are formed in the cavities of the mold, the implants are highly uniform with respect to shape, size, and composition. Due to the consistency among the physical and compositional makeup of each implant of the present pharmaceutical compositions, the pharmaceutical compositions of the present disclosure provide highly uniform release rates and dosing ranges. The methods and materials for fabricating the implants of the present disclosure are further described and disclosed in issued patents and pending patent applications, each of which are incorporated herein by reference in their entirety: U.S. Pat. Nos. 8,518,316; 8,444,907; 8,420,124; 8,268,446; 8,263,129; 8,158,728; 8,128,393; 7,976,759; U.S. Pat. Application Publications Nos. 2013-0249138, 2013-0241107, 2013-0228950, 2013-0202729, 2013-0011618, 2013-0256354, 2012-0189728, 2010-0003291, 2009-0165320, 2008-0131692; and pending U.S. Application Nos. 13/852,683 filed March 28, 2013 and 13/950,447 filed July 25, 2013.

The mold cavities can be formed into various shapes and sizes. For example, the cavities may be shaped with curved edges, sharp or square edges, circular or arched perimeters, flat sides, substantially parallel sides, pointed ends, cylindrical, prism, rectangular prism, triangular prism, pyramid, square pyramid, triangular pyramid, cone, cylinder, torus, or rod. The cavities may have the same shape or may have different shapes. In certain aspects of the disclosure, the shapes of the implants are a cylinder, rectangular prism, and rod. In a particular embodiment, the implant is a rod having substantially a square, rectangular, circular, or oval cross-section taken normal to the long axis of the implant.

The mold cavities can be dimensioned from nanometer to micrometer to millimeter dimensions and larger. For certain embodiments of the disclosure, mold cavities are dimensioned in the micrometer and millimeter range. For example, cavities may have a smallest dimension of between approximately 50 nanometers and approximately 750 µm. In some aspects, the smallest mold cavity dimension may be between approximately 1 µm and approximately 500 µm. In other aspects, the smallest mold cavity dimension may be between approximately 225 µm and approximately 400 µm. For example, mold cavities may have a largest dimension of between approximately 1,000 µm and approximately 10,000 µm. In other aspects, the largest mold cavity dimension may be between approximately 2,000 µm and approximately 6,000 µm. In other aspects, the largest mold cavity dimension may be between approximately 4,000 µm and approximately 6,000 µm.

The implants can have an aspect ratio of width-to-length from 1:1 to greater than 1:30. In some embodiments, the width-to-length aspect ratio of the implant is between 1:2 to 1:25. In some embodiments, the width-to-length aspect ratio of the implant is between 1:5 to 1:20.In some embodiments, the width-to-length aspect ratio of the implant is between 1:10 to 1:20. In some embodiments, the width-to-length aspect ratio of the implant is between 1:15 to 1:20.

In one embodiment, a rod with dimensions of about 130 µm × about 180 µm × about 1,500 µm (H × W × L) is fabricated.

In one embodiment, a rod with dimensions of about 225 µm × about 225 µm × about 4,000 µm (H × W × L) is fabricated.

In another embodiment, a rod with dimensions of about 225 µm × about 225 µm × about 2,925 µm (H × W × L) is fabricated.

In another embodiment, a rod with dimensions of about 300 µm × about 300 µm × about 6,000 µm (H × W × L) is fabricated.

In another embodiment, a rod with dimensions of about 330 µm × about 330 µm × about 6,000 µm (H × W × L) is fabricated.

In a further embodiment, a rod with dimensions of about 400 µm × about 400 µm × about 6,000 µm (H × W × L) is fabricated.

Once fabricated, the implants may remain on an array for storage, or may be harvested immediately for storage and/or utilization. Implants may be fabricated using sterile processes, may be sterilized after fabrication, or may be fabricated using sterile processes and sterilized after fabrication. Thus, the present disclosure contemplates kits that include a storage array that has fabricated implants attached thereon. These storage array/implant kits provide a convenient method for mass shipping and distribution of the manufactured implants. In embodiments, fabricated implants are packaged individually.

### Delivery Devices

In embodiments, a delivery device may be used to insert the implant into the eye or eyes for treatment or prevention of ocular diseases.

Suitable devices can include a needle or needle-like applicator. In some embodiments, the smallest dimension of an implant may range from approximately 50 µm to approximately 750 µm, and therefore a needle or needle-like applicator with a gauge ranging from approximately 19 to 27 may be utilized depending on the cross-sectional dimension of the implant to inner diameter of the needle as detailed elsewhere herein. The delivery device may be a syringe with an appropriately sized needle or may be a syringe-like device with a needle-like applicator.

For example, in embodiments in which an implant is sized and structure to allow for administration in a 27 gauge needle (*e.g.,* for a rod-shaped implant having dimensions of 225 µm × about 225 µm × about 2,925 µm), the applicator needle has a gauge of 27 and a length of 13mm. In other embodiments in which an implant is sized and structured to allow for administration in a 21 gauge needle (*e.g.* for a rod-shaped implant having dimensions of 300 µm × about 300 µm × about 6,000 µm), the applicator needle has a gauge of 21 and a length of 25mm. In other embodiments in which an implant is sized and structured to allow for administration in a 25, 26, or 27 gauge needle (*e.g.,* for a rod-shaped implant having dimensions of 130 µm × about 180 µm × about 1,500 µm), the applicator needle has a gauge of 25, 26, or 27 and length of 13mm.

Delivery routes include punctual, intravitreal, subconjunctival, intracameral, lens, intrascleral, fornix, anterior sub-Tenon's, suprachoroidal, posterior sub-Tenon's, subretinal, anterior chamber, and posterior chamber, to name a few.

In embodiments, an implant or implants are delivered to the subconjunctival region of a patient's eye to prevent and/or treat post-operative inflammation.

In embodiments, an implant or implants are delivered to the intracameral region of a patient's eye to prevent and/or treat post-operative inflammation.

### Kits

In embodiments, the implant and delivery device may be combined and presented as a kit for use.

The implant may be packaged separately from the delivery device and loaded into the delivery device just prior to use.

Also, the implant may be loaded into the delivery device prior to packaging. In this case, once the kit is opened, the delivery device is ready for use.

Components may be sterilized individually and combined into a kit, or may be sterilized after being combined into a kit.

Further, as aforementioned, a kit may include an array with implants bound thereon.

### Use of Ocular Implant for Treatment

In one aspect of the disclosure, there is presented a method of preventing and/or treating post-operative inflammation. The method comprises placing a biocompatible implant in an eye, degrading or dissolving the implant, and releasing a therapeutic agent which is effective to prevent and/or treat post-operative inflammation.

In aspects of the disclosure, the eye is that of an animal. For example, a dog, cat, horse, cow (or any agricultural livestock or domesticated pet), or human.

In one aspect of the disclosure, there is presented a method of preventing and/or treating post-operative pain. The method comprises placing a biocompatible implant in an eye, degrading or dissolving the implant, and releasing a therapeutic agent which is effective to prevent and/or treat post-operative pain. The pain treated or prevented may or may not be associated with inflammation.

### Corneal Thickening

Not to be bound by a particular theory, corneal thickening (also known as corneal edema) refers to inflammation which is induced in response to an ocular operation, such as surgery or administration of an ocular implant. This effect is characterized by the buildup of excess aqueous fluid in corneal tissue. Corneal thickening is well known in the art to be a factor which hinders the improvement of vision days after surgery. *See* Donnenfeld, et al, Am. J. of Ophthalmology, Vol 152 (4); 2011: 609-617, which is herein incorporated by reference in its entirety. Implants comprising hydrophilic polymers, such as PEGs, can also induce corneal thickening by causing cells located near the site of administration to uptake excess aqueous fluid thereby becoming inflamed. Thus, corneal thickening which occurs after ocular administration of a hydrophilic polymer can exacerbate the corneal thickening effect observed after cataract surgery and further impair vision.

Corticosteroids are known to cause thinning of the cornea after ocular administration. Thus, corticosteroids are typically administered in an emulsified formulation after cataract surgery to reduce corneal thickening. However, emulsified corticosteroid formulations are not able to reduce corneal thickening to a baseline at least a month after surgery. *See* Donnenfeld, et al, Am. J. of Ophthalmology, Vol 152 (4); 2011: 609-617.

**In** some embodiments, the inventors discovered implant formulations which overcome the limitations in the prior. Thus, in some embodiments, the implants described herein are able to reduce post-operation corneal thickening in the range of from about 1% to about 100%. That is, in some embodiments, implants can be formulated to offset post-operative corneal thickening.

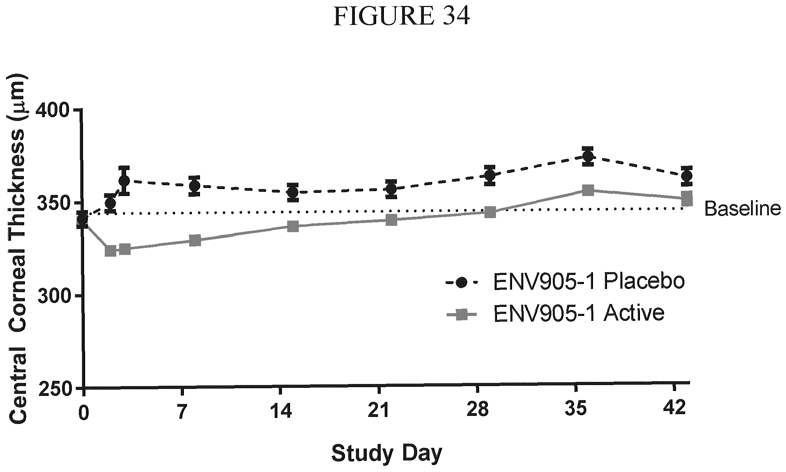

**In** some embodiments, the implants described herein are able to reduce corneal thickening to a baseline established prior to surgery within about 30 days or less. Accordingly, in some embodiments, the implants described herein can reduce post-operative corneal thickening to a baseline within about 1 day after administration of said implant. In other embodiments, the implants described herein are able to reduce post-surgery corneal thickening below a baseline established prior to surgery by about 1% to about 10%. In further embodiments, the implants can maintain reduced post-operative corneal thickening for at least about 42 days. *See* FIG. 34.

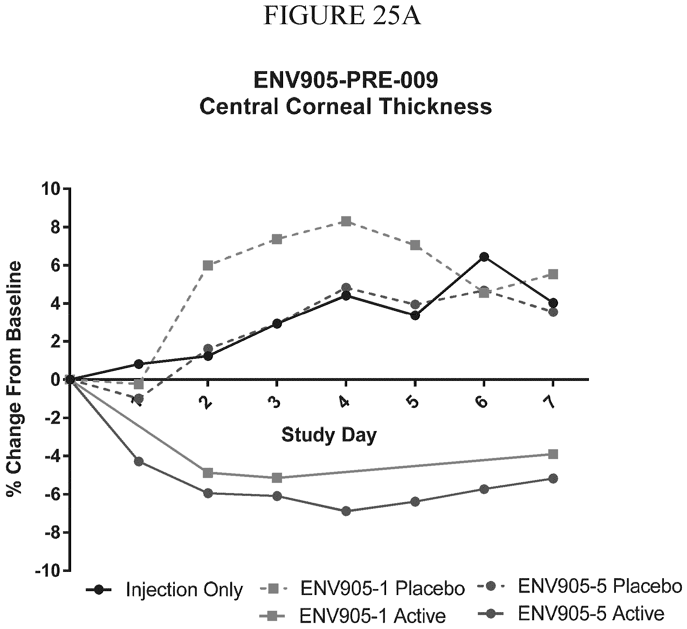

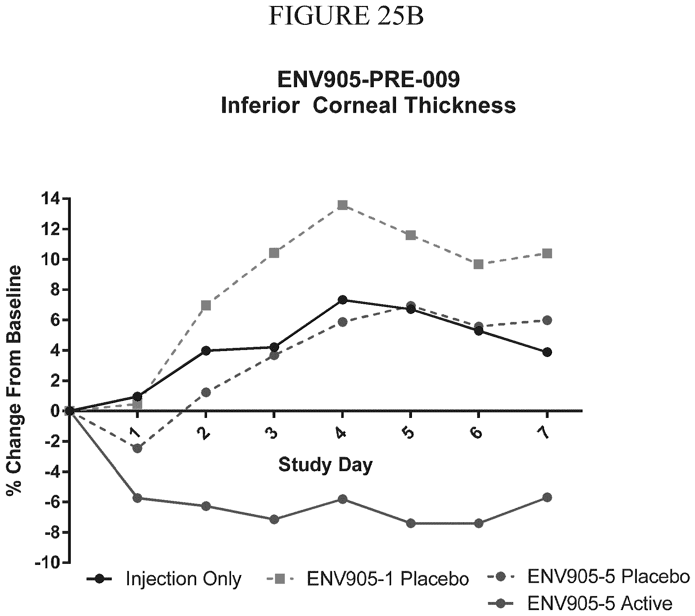

**In** some embodiments, implants described herein can be formulated to reduce corneal thickening observed after surgery and/or after administration of an implant. *See* **FIG. 25A** and **25B****.** That is, in some embodiments, the implants described herein can be formulated to prevent the corneal thickening effect associated with administration of a hydrophilic implant. In some embodiments, reducing the mass of the polymer matrix in the ocular implant can decrease corneal thickening. Accordingly, in embodiments, the amount of polymer matrix per implant is less than about 100 µg, e.g., less than about 40 µg. In further embodiments, the percent weight ratio (% w/w) of the polymer matrix relative to the therapeutic agent is less than about 65%, e.g., less than about 45%.

### Course of Treatment

Over the course of treatment, the biocompatible polymer matrix degrades or dissolves releasing the therapeutic agent. Once the therapeutic agent has been completely released, the polymer matrix is expected to be degraded or dissolved. Complete polymer matrix degradation may take longer than the complete release of the therapeutic agent. Polymer matrix degradation may occur at the same rate as the release of the therapeutic agent. Polymer matrix dissolution provides for rapid release of the therapeutic agent.

Current treatments for prevention and/or treatment of post-operative inflammation require the patient to place drops in their eyes each day. The pharmaceutical composition of the disclosure is designed for release of an effective amount of therapeutic agent, eliminating the need for daily drops. The effective amount of the therapeutic agent may be provided through sustained release or rapid release. For example, the pharmaceutical composition may be designed to release an effective amount of therapeutic agent for less than one day to approximately one day, two days, three days, four days, five days, six days, seven days, eight days, nine days, ten days, eleven days, twelve days, thirteen days, fourteen days, 28 days, or longer. In aspects, the pharmaceutical composition is designed to release an effective amount of therapeutic agent for one day, two days, three days, five days, seven days, ten days, twelve days, or longer. In other aspects, the pharmaceutical composition is designed to release an effective amount of therapeutic agent for seven days, ten days, twelve days, or fourteen days.

Methods of the present disclosure for treating or preventing a condition include inserting more than 5 implants to treat or prevent the condition for more than 2 weeks. Methods of the present disclosure for treating or preventing a condition include inserting more than 10 implants to treat or prevent the condition for more than 2 weeks. Methods of the present disclosure for treating or preventing a condition include inserting more than 25 implants to treat or prevent the condition for more than 2 weeks. Methods of the present disclosure for treating or preventing a condition include inserting more than 50 implants to treat or prevent the condition for more than 2 weeks. Methods of the present disclosure for treating or preventing a condition include inserting more than 100 implants to treat or prevent the condition for more than 2 weeks. Methods of the present disclosure for treating or preventing a condition include inserting more than 500 implants to treat or prevent the condition for more than 2 weeks. Methods of the present disclosure for treating or preventing a condition include inserting more than 1,000 implants to treat or prevent the condition for more than 2 weeks. Methods of the present disclosure for treating or preventing a condition include inserting more than 10,000 implants to treat or prevent the condition for more than 2 weeks. Methods of the present disclosure for treating or preventing a condition include inserting more than 100,000 implants to treat or prevent the condition for more than 2 weeks. Methods of the present disclosure for treating or preventing a condition include inserting more than 1,000,000 implants to treat or prevent the condition for more than 2 weeks. The polymer composition and ratios of each implant in these collections of implants can be varied between implants within a single dose such that an aggregate degradation profile of the collection of implants is achieved for delivery of the therapeutic agent for greater than 2 weeks, greater than 1 month, greater than 3 months, greater than 4 months, greater than 6 months, greater than 9 months and greater than 12 months.

Delivery of implants disclosed herein may include delivery through a 27 gauge needle or smaller. In some delivery methods, the needle is 28 gauge, 29 gauge, 30 gauge, 31 gauge, 32 gauge, 33 gauge, or 34 gauge needle. In other aspects, a 21 or 22 gauge needle is used. In other aspects, a 25 or 26 gauge needle is used.

The following non-limiting examples illustrate certain aspects of the present disclosure.

### EXAMPLES

### Example 1: Preparation of Polymer Matrix/Therapeutic Agent Blends

A series of polymer matrix/therapeutic agent blends were prepared prior to molding implants. All blends contained difluprednate as the therapeutic agent. **Table 1** details the composition of one series of blends.

**Table 1: Polymer Matrix/Therapeutic Agent Blend Ratios**

| ID | Polymer Matrix Target, % | | | | Therapeutic Agent Target, % |
|---|---|---|---|---|---|
| | PLGA | | PEG | PCL | Difluprednate |
| | DLG1A | 502H | PEG3300 | 14K | |
| 642-64-1 | 40.0 | | 10.0 | | 50.0 |
| 642-64-2 | | 60.2 | 9.8 | | 30.0 |
| 642-64-3 | | | | 70.0 | 30.0 |
| 642-64-4 | 50.0 | | | | 50.0 |

### Example 2: Fabrication of Molds

A series of molds of various dimensions were obtained for the PRINT^{®} particle replication technology from Envisia Therapeutics Inc., North Carolina.

Molds obtained included molds with cavity sizes and shapes as follows: a) a rod shape with dimensions of about 225 µm × about 225 µm × about 4,000 µm, b) a rod shape with dimensions of about 300 µm × about 300 µm × about 6,000 µm, and c) a rod shape with dimensions of about 400 µm × about 400 µm × about 6,000 µm.

### Example 3: Implant Fabrication

A series of implants were fabricated utilizing the polymer matrix/therapeutic agent blends of Example 1 and the molds of Example 2. Under aseptic conditions, a portion of polymer matrix/therapeutic agent blend was spread over a PET sheet and was heated on a hot plate for approximately 30 to 90 seconds until fluid. Once heated, the blend was covered with the mold of Example 2 which had the desired dimensions. Light pressure was applied using a hand roller to spread the blend over the mold area. The mold/blend laminate was then passed through a commercially available thermal laminator using the parameters in **Table** 2 below. The blend flowed into the mold cavities and assumed the shape of the mold cavities. The blend was allowed to cool to room temperature creating individual implants in the mold cavities. The mold was then removed leaving a two-dimensional array of implants on the PET film. Individual implants were removed from the PET film utilizing forceps.

**Table 2: Implant Fabrication Conditions**

| Process Parameter | |
|---|---|
| Hot Plate Temperature, °C | 80-180 |
| Hot Plate Time, seconds | 30-180 |

**Table 3** details the implants that were produced with the blends of Example 1 and the molds of Example 2 using the fabrication process of Example 3. The same ID used for the blend was also used for the resulting implant.

**Table 3: Implant Configurations**

| ID | Implant Dimensions |
|---|---|
| 642-64-1 | 225 µm X 225 µm X 4,000 µm |
| 642-64-2 | 225 µm X 225 µm X 4,000 µm |
| 642-64-3 | 225 µm X 225 µm X 4,000 µm |
| 642-64-4 | 225 µm X 225 µm X 4,000 µm |

### Example 4: Analysis of Difluprednate Content

Individual implants were placed into 2 mL HPLC vials and acetonitrile was added. The solution was diluted with an equal volume of water. The difluprednate concentration was determined using HPLC as described in Example 5 below. The mass of individual implants was also determined. Table 4 details the test results for the implants fabricated in Example 3.

**Table 4: Difluprednate Content for Implants**

| ID | Difluprednate, µg | | | Overall Implant, µg | | | Difluprednate |
|---|---|---|---|---|---|---|---|
| | AVE | STDEV | %RSD | AVE | STDEV | %RSD | % |
| 642-64-1 | 129.2 | 5.8 | 4.5 | 252.2 | 14.1 | 5.6 | 51.2 |
| 642-64-2 | 85.5 | 6.9 | 8.0 | 279.8 | 16.4 | 5.8 | 30.6 |
| 642-64-3 | 60.3 | 1.5 | 2.5 | 211.2 | 6.7 | 3.2 | 28.6 |
| 642-64-4 | 144.2 | 28.0 | 19.4 | 266.4 | 43.5 | 16.3 | 54.1 |

### Example 5: HPLC Method for Determination of Difluprednate

Mobile phase A was prepared by combining approximately 900 mL water, approximately 100 mL acetonitrile, and approximately 1 mL trifluoroacetic acid. Mobile phase B was prepared by combining approximately 900 mL acetonitrile, approximately 100 mL water, and approximately 1 mL trifluoroacetic acid. A series of standards were prepared by diluting a known difluprednate standard.

For analysis conditions, the column was a Phenomenex Luna Phenyl-hexyl, 3 µm, 100 X 4.6 mm, flow rate was 1.0 mL per minute, wavelength was 244 nm, temperature was 55°C, injection volume was 5 µL, and the run time was 14 minutes. The gradient is detailed in **Table 5.**

**Table 5: HPLC Gradient for Difluprednate Analysis**

| Time, minutes | Mobile Phase A, % | Mobile Phase B, % |
|---|---|---|
| 0 | 70 | 30 |
| 1 | 70 | 30 |
| 9 | 30 | 70 |
| 9.1 | 70 | 30 |
| 14 | 70 | 30 |

To determine therapeutic agent content in an implant, the measured µg/mL determined from the response associated with the standard curve was multiplied by the volume used to dissolve the implant.

### Example 6: In Vitro Difluprednate Release Studies

In vitro release of difluprednate was determined for the implants of Example 3. In this study, single implants were placed into HPLC vials and were incubated in a volume of 1X PBS (phosphate buffered saline) containing 1% sodium dodecyl sulfate media at 37°C. At each time point of interest, the media was removed for analysis. The media was then replaced with fresh media. The media that was removed was analyzed for difluprednate released via the HPLC method of Example 5.

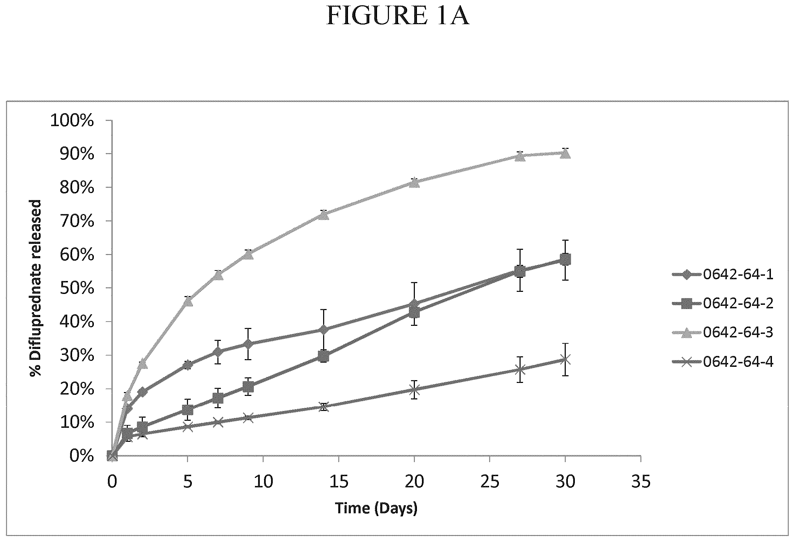

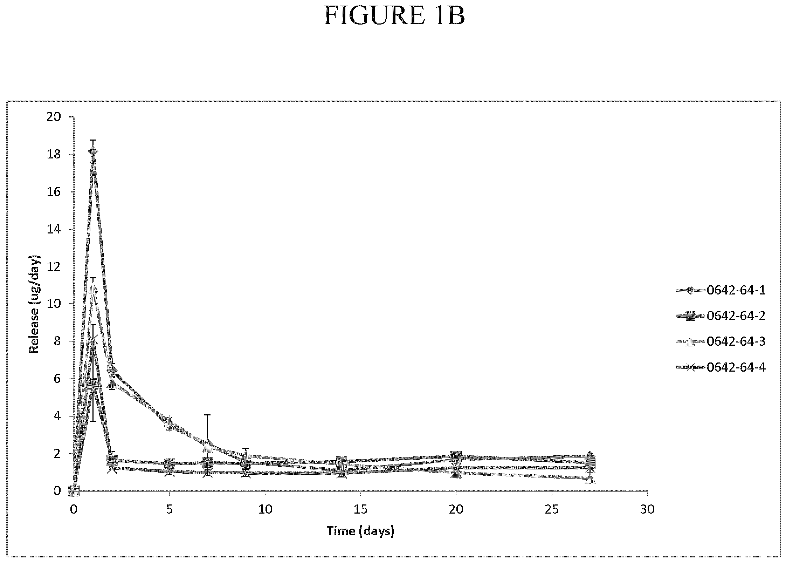

### FIGS. 1A and 1B depict the results of the study graphically.

### Example 7: Preparation of Polymer Matrix/Therapeutic Agent Blends

A series of polymer matrix/therapeutic agent blends were prepared prior to molding implants. All blends contained difluprednate as the therapeutic agent. **Table 6** details the composition of one series of blends.

**Table 6: Polymer Matrix/Therapeutic Agent Blend Ratios**

| ID | Polymer Matrix Target, % | | | Therapeutic Agent Target, % |
|---|---|---|---|---|
| | PEG | PCL | | Difluprednate |
| | PEG3300 | 2K | 14K | |
| 642-69-1 | | 50.0 | | 50.0 |
| 642-69-2 | | 25.0 | 25.0 | 50.0 |
| 642-69-3 | | | 50.0 | 50.0 |
| 642-69-4 | 10.0 | | 40.0 | 50.0 |
| 642-69-5 | 25.0 | | 25.0 | 50.0 |

**Table 7** details the implants that were produced with the blends of Example 7 and the molds of Example 2 using the fabrication process of Example 3. The same ID used for the blend was also used for the resulting implant.

**Table 7: Implant Configurations**

| ID | Implant Dimensions |
|---|---|
| 642-69-1 | 225 µm X 225 µm X 4,000 µm |
| 642-69-2 | 225 µm X 225 µm X 4,000 µm |
| 642-69-3 | 225 µm X 225 µm X 4,000 µm |
| 642-69-4 | 225 µm X 225 µm X 4,000 µm |
| 642-69-5 | 400 µm X 400 µm X 6,000 µm |

Implants were analyzed for difluprednate content according to Example 4 and Example 5. Results for the implants of Example 7 are shown in **Table 8.**

**Table 8: Difluprednate Content for Implants**

| ID | Difluprednate, µg | | | Overall Implant, µg | | | Difluprednate |
|---|---|---|---|---|---|---|---|
| | AVE | STDEV | %RSD | AVE | STDEV | %RSD | % |
| 642-69-1 | 99.2 | 3.0 | 3.0 | 206.0 | 4.5 | 2.2 | 48.1 |
| 642-69-2 | 105.0 | 4.6 | 4.4 | 206.5 | 6.7 | 3.3 | 50.1 |
| 642-69-3 | 115.1 | 6.5 | 5.7 | 227.0 | 7.6 | 3.3 | 51.6 |
| 642-69-4 | 102.9 | 2.7 | 2.6 | 220.0 | 8.3 | 3.8 | 46.7 |
| 642-69-5 | 630.5 | 56.3 | 8.9 | 1,197.8 | 95.3 | 8.0 | 51.7 |

### Example 8: In Vitro Difluprednate Release Studies

In vitro release of difluprednate was determined for the implants of Example 7. Implants were testing according to the method of Example 6.

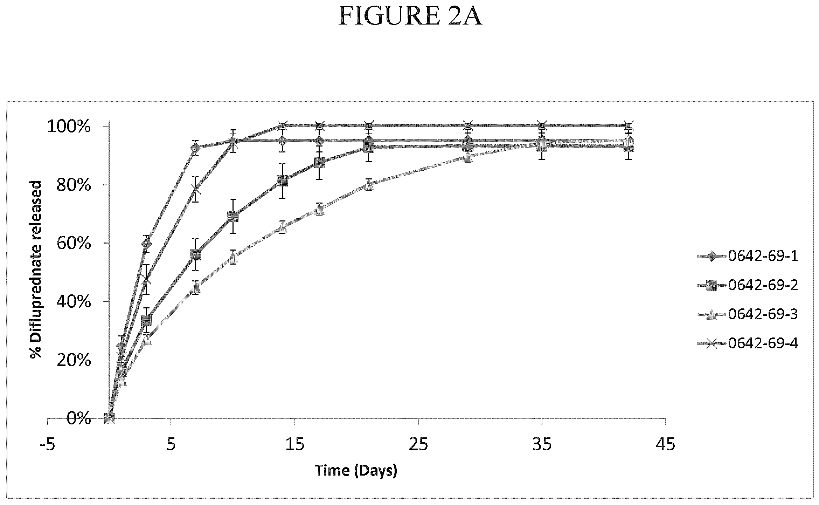

**FIGS. 2A** through **2F** detail particular results from studies utilizing a single implant.

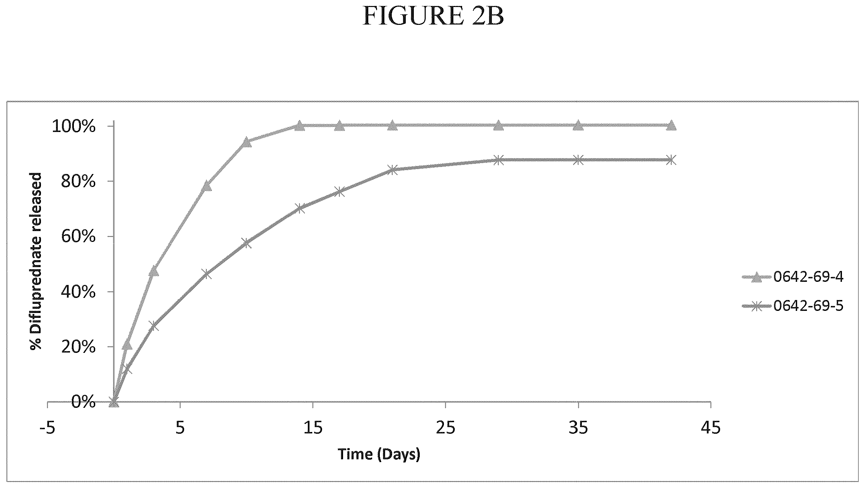

**FIG. 2A** shows the percent difluprednate released as a function of time (days) for four of the samples under study. **FIG. 2B** shows the percent of difluprednate released as a function of time (days) for two of the samples under study. The data demonstrates that for the blends under investigation, for example 642-69-4 and 642-69-5, a smaller implant containing about the same percentage therapeutic agent content releases the therapeutic agent more rapidly on a percentage basis when compared to a larger implant. Also, the data demonstrates that use of a higher molecular weight PCL slows the release of therapeutic agent. When combining a higher and lower molecular weight PCL, the release of therapeutic agent is intermediate to that of each respective PCL. To increase the release rate of higher molecular weight PCL samples, PEG can also be utilized. The data demonstrates that the percentage released for the therapeutic agent can be adjusted.

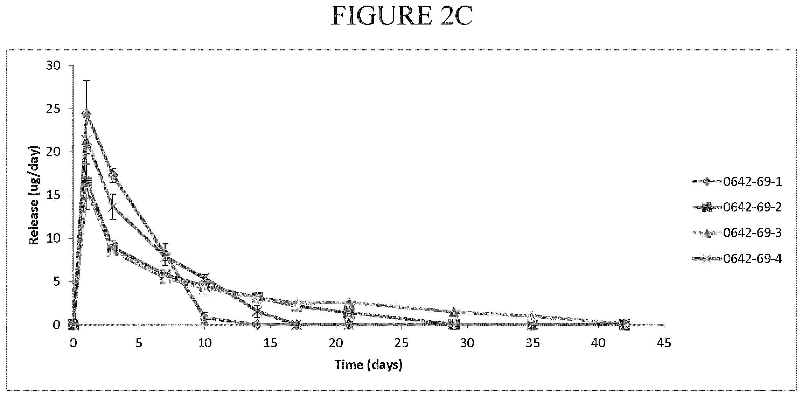

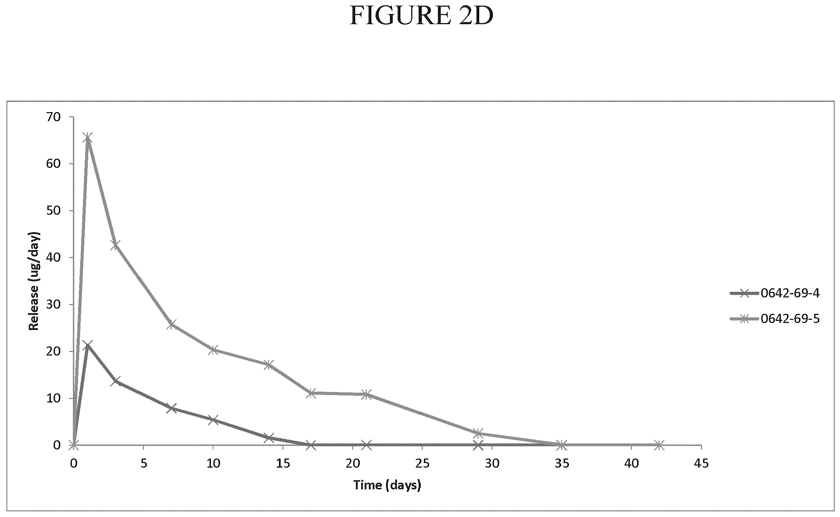

Further details of the study are shown in **FIGS. 2C** **and** **2D****,**
**FIG. 2C** **and** **2D** show the release rate (µg/day) of difluprednate as a function of time (days).

The data demonstrates that for the blends under investigation, for example 642-69-4 and 642-69-5, a smaller implant containing about the same percentage therapeutic agent but an overall lower therapeutic agent content releases the therapeutic agent for a shorter time period when compared to a larger implant. Also, the data demonstrates that use of a higher molecular weight PCL slows the release of therapeutic agent, allowing the implant to release therapeutic agent for a longer time span. When combining use of a higher and lower molecular weight PCL into the same implant configuration, the release rate of therapeutic agent is intermediate to that of each respective PCL. To increase the release rate of higher molecular weight PCL samples, PEG can also be utilized. The data demonstrates that the release rate for the therapeutic agent can be adjusted.

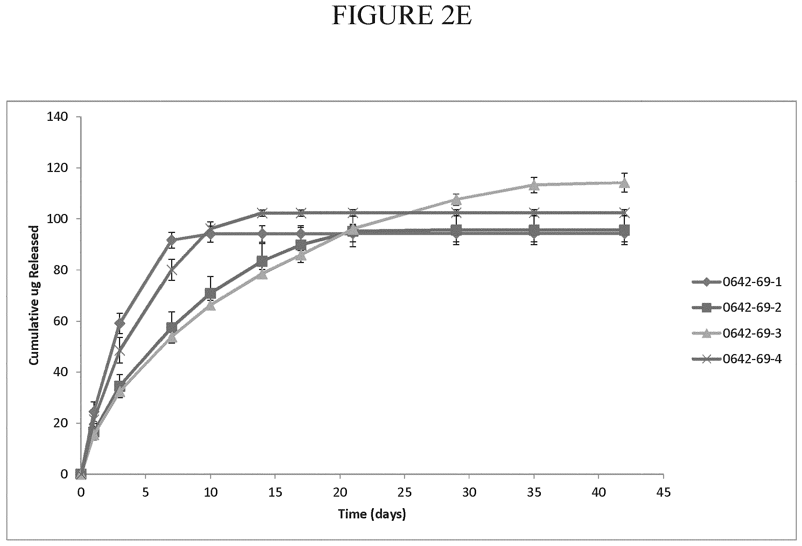

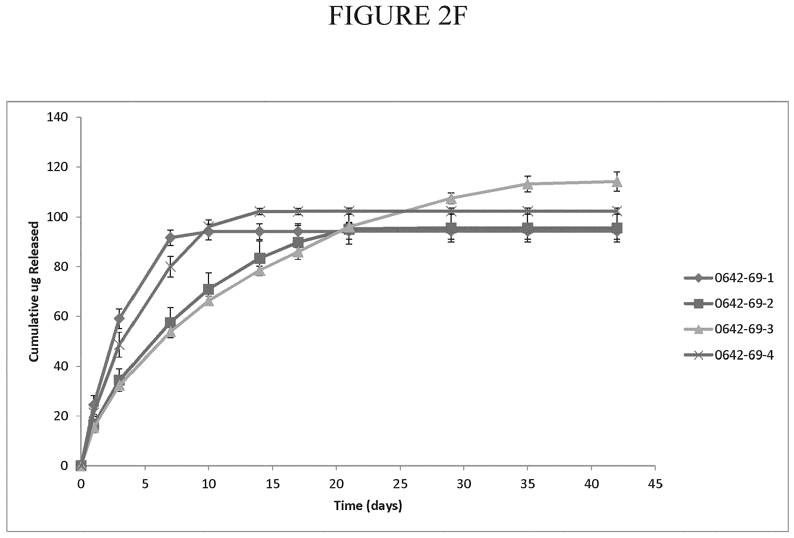

Further details of the study are shown in **FIG. 2E** and **2F****.**

**FIG. 2E** shows the cumulative therapeutic agent released as a function of time. The data in **FIG. 2F** shows a subset of the samples all having a 225 µm X 225 µm X 4,000 µm configuration.

### Example 9: Implants Utilized for In Vivo Studies

A twelve day in vivo study utilizing New Zealand White Rabbits was conducted to determine the ocular tolerability and also determine residual difluprednate in the implant at the study termination. **Table 9** details the IDs of the implants, number of implants utilized for dosing, and number of eyes dosed for the studies. Tables in the previous examples provide information as to composition and dimension for the implants. Topical administration of DUREZOL^{®} (difluprednate ophthalmic emulsion) (Alcon Laboratories, Inc.) was used as a positive control. The dosing paradigm for the positive control was one drop per eye, four times daily for the duration of the study. For a negative control the surgery was conducted, but no implant was used. Implants were placed either subconjunctivally or intracamerally. For subconjunctival placement, the implants were either directly injected into the subconjunctival tissue or a pocket was created in the subconjunctival region through a 1-2 mm incision in the conjunctiva at the place of interest. An appropriately sized needle was used to deliver the implant directly into the tissue or into the pocket. The pocket was able to contain up to three implants. For eyes containing greater than three implants, the appropriate number of pockets were created (i.e. two pockets to hold two implants each for a total of four implants delivered). For intracameral placement, implants were delivered through the limbus to the intracameral location using an appropriately sized needle as described elsewhere herein.

The Hackett-McDonald Scoring System was used to determine the Total Ocular Exam Score. A "perfect score" is equal to 0; however, a composite score that does not equal zero does not indicate a clinically unacceptable pharmaceutical composition. Residual difluprednate was determined in the retrieved implants using the method described in Example 4 and Example 5.

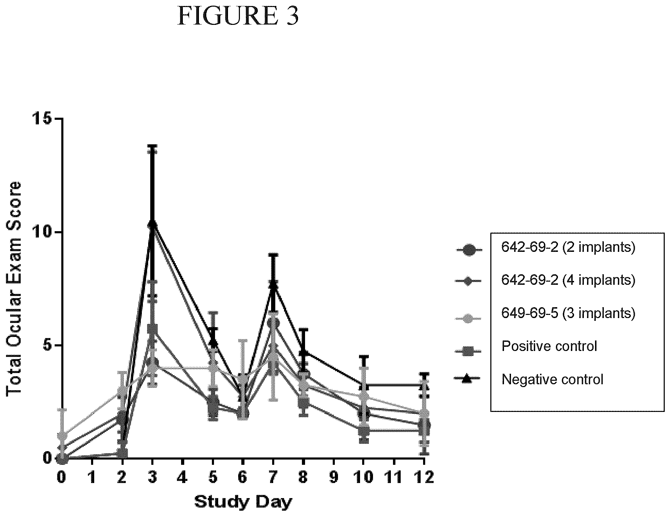

**FIG. 3** depicts the Total Ocular Exam Score for the implants under study. Transient spikes post-procedure were observed on Days 3 and 7. As expected, the negative control has the highest score throughout the study duration. However, at least one sample, 642-69-5 (3 implants) had a Total Ocular Exam Score below that of the positive control at least one time interval.

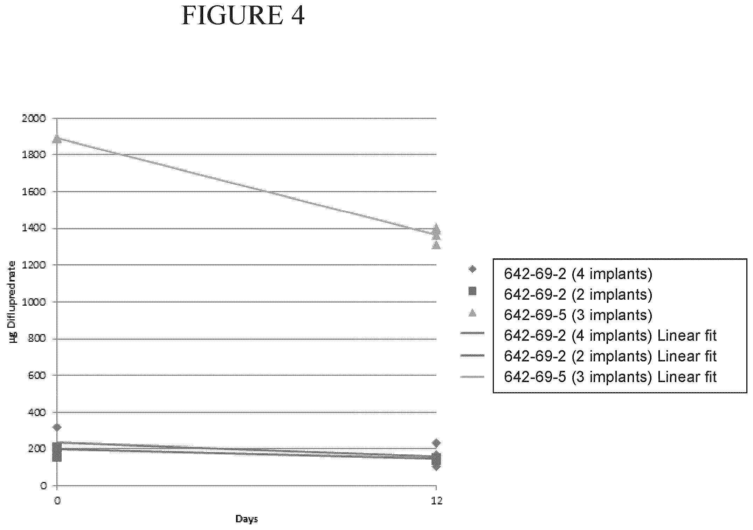

**Table 10** and **FIG. 4** depict results for the residual difluprednate determination. Note, animal 34L, one of the four implants fractured upon insertion. As a result, the theoretical dose is an estimate, not an actual value. At least two of the samples delivered a therapeutic agent dose with a relative standard deviation of less than 10% over the 12 day study. **FIG. 4** depicts the data graphically.

**Table 9: In Vivo Implants**

| ID | Implants Dosed/Eye # | Eyes Dosed # | Therapeutic Agent Content/Implant µg | Therapeutic Agent Total Content/Dose µg | Delivery Route |
|---|---|---|---|---|---|
| 642-69-2 | 4 | 4 | 105 | 420 | Subconjunctival |
| 642-69-2 | 2 | 4 | 105 | 210 | Intracameral |
| 642-69-5 | 3 | 4 | 636 | 1908 | Subconjunctival |
| Positive Control | Not Applicable | 4 | 60 | 60 | Topical drops |
| Negative Control | Not Applicable | 4 | 0 | 0 | No implant |

**Table 10: Delivered and Retrieved Difluprednate for In Vivo Study**

| ID | Animal | Therapeutic Agent Total Content/Dose µg | Therapeutic Agent Recovered µg | Therapeutic Agent Recovered % | Therapeutic Agent Delivered % |
|---|---|---|---|---|---|
| 642-69-2 | 34L | 385 | 130.4 | 33.9 | 66.1 |
| | 34R | 420 | 170.4 | 40.6 | 59.4 |
| | 35L | 420 | 106.4 | 25.3 | 74.7 |
| | 35R | 420 | 230.5 | 54.9 | 45.1 |
| | | | Average | 38.7 | 61.3 |
| | | | STDEV | 12.5 | 12.5 |
| 642-69-2 | 36L | 210 | 151.2 | 72.0 | 28.0 |
| | 36R | 210 | 144 | 68.6 | 31.4 |
| | 37L | 210 | 152.6 | 72.7 | 27.3 |
| | 37R | 210 | 141.3 | 67.3 | 32.7 |
| | | | Average | 70.1 | 29.9 |
| | | | STDEV | 2.6 | 2.6 |
| 642-69-5 | 38L | 1892 | 1402.4 | 74.1 | 25.9 |
| | 38R | 1892 | 1390.8 | 73.5 | 26.5 |
| | 39L | 1892 | 1365.3 | 72.2 | 27.8 |
| | 39R | 1892 | 1313.9 | 69.4 | 30.6 |
| | | | Average | 72.3 | 27.7 |
| | | | STDEV | 2.1 | 2.1 |

### Example 10: Polymer Matrix/Therapeutic Agent Blend Ratios

A series of polymer matrix/therapeutic agent blends were prepared prior to molding implants. All blends contained difluprednate as the therapeutic agent. Table 11 details the composition of one series of blends.

**Table 11: Polymer Matrix/Therapeutic Agent Blend Ratios**

| ID | Polymer Matrix Target, % | | | | | | Therapeutic Agent Target, % |
|---|---|---|---|---|---|---|---|
| | PEG | | PCL | | | Glycerol | Difluprednate |
| | PEG35K | PEG100K | 2K | 14K | 45K | | |
| 0053-1-1 | 34.3 | 14.7 | 8.8 | 8.8 | | 3.5 | 30.0 |
| 0053-1-2 | 14.7 | 6.3 | 22.8 | 22.8 | | 3.5 | 30.0 |
| 0053-1-3 | 22.1 | 9.5 | 17.5 | 17.5 | | 3.5 | 30.0 |
| 0053-1-4 | 22.1 | 9.5 | 17.5 | | 17.5 | 3.5 | 30.0 |
| 0053-1-5 | 14.7 | 6.3 | 22.8 | | 22.8 | 3.5 | 30.0 |
| 0053-1-6 | 52.5 | 14.0 | | | | 3.5 | 30.0 |
| 0053-4-1 | 34.3 | 14.7 | 8.8 | 8.8 | | 3.5 | 30.0 |
| 0053-4-2 | 14.7 | 6.3 | 22.8 | 22.8 | | 3.5 | 30.0 |
| 0053-4-3 | 22.1 | 9.5 | 17.5 | 17.5 | | 3.5 | 30.0 |
| 0053-4-4 | 52.5 | 14.0 | | | | 3.5 | 30.0 |
| 0053-4-5 | 24.5 | 10.5 | 31.5 | | | 3.5 | 30.0 |
| 0053-4-6 | 14.7 | 6.3 | 45.5 | | | 3.5 | 30.0 |
| 0053-4-7 | | | 66.5 | | | 3.5 | 30.0 |
| 0053-4-8 | 10.5 | 4.5 | 32.5 | | | 2.5 | 50.0 |
| 0053-4-9 | 5.3 | 2.3 | 40.0 | | | 3.5 | 50.0 |
| 0053-4-10 | | | 66.5 | | | 3.5 | 30.0 |
| 0053-4-11 | 14.7 | 6.3 | 45.5 | | | 3.5 | 30.0 |
| 0053-4-12 | 24.5 | 10.5 | 31.5 | | | 3.5 | 30.0 |
| 0053-4-13 | 27.0 | 11.6 | 14.0 | 14.0 | | 3.5 | 30.0 |

### Example 11: Implant Fabrication

**Table 12** details the implants that were produced with the blends of Example 10 and the molds of Example 2 using the fabrication process of Example 3. The same ID used for the blend was also used for the resulting implant.

**Table 12: Implant Configurations**

| ID | Implant Dimensions |
|---|---|
| 0053-1-1 | 225 µm X 225 µm X 4,000 µm |
| 0053-1-2 | 225 µm X 225 µm X 4,000 µm |
| 0053-1-3 | 225 µm X 225 µm X 4,000 µm |
| 0053-1-4 | 225 µm X 225 µm X 4,000 µm |
| 0053-1-5 | 225 µm X 225 µm X 4,000 µm |
| 0053-1-6 | 225 µm X 225 µm X 4,000 µm |
| 0053-4-1 | 400 µm X 400 µm X 6,000 µm |
| 0053-4-2 | 400 µm X 400 µm X 6,000 µm |
| 0053-4-3 | 400 µm X 400 µm X 6,000 µm |
| 0053-4-4 | 400 µm X 400 µm X 6,000 µm |
| 0053-4-5 | 400 µm X 400 µm X 6,000 µm |
| 0053-4-6 | 400 µm X 400 µm X 6,000 µm |
| 0053-4-7 | 400 µm X 400 µm X 6,000 µm |
| 0053-4-8 | 400 µm X 400 µm X 6,000 µm |
| 0053-4-9 | 400 µm X 400 µm X 6,000 µm |
| 0053-4-10 | 225 µm X 225 µm X 4,000 µm |
| 0053-4-11 | 225 µm X 225 µm X 4,000 µm |
| 0053-4-12 | 225 µm X 225 µm X 4,000 µm |
| 0053-4-13 | 400 µm X 400 µm X 6,000 µm |

### Example 12: Analysis of Difluprednate Content

Implants were analyzed for difluprednate content according to Example 4 and Example 5. The mass of individual implants was also determined. **Table 13** details the test results for the implants fabricated in Example 11.

**Table 13: Difluprednate Content for Implants**

| ID | Difluprednate, µg | | | Overall Implant, µg | | | Difluprednate |
|---|---|---|---|---|---|---|---|
| | AVE | STDEV | %RSD | AVE | STDEV | %RSD | % |
| 0053-1-1 | 73.9 | 4.1 | 5.5 | 222.1 | 22.5 | 10.1 | 33.3 |
| 0053-1-2 | 58.2 | 2.2 | 3.8 | 195.4 | 3.6 | 1.8 | 29.8 |
| 0053-1-3 | 59.4 | 1.8 | 3.0 | 209.9 | 5.1 | 2.4 | 28.3 |
| 0053-1-4 | 56.0 | 1.7 | 3.1 | 199.5 | 3.7 | 1.8 | 28.1 |
| 0053-1-5 | 57.5 | 1.8 | 3.1 | 192.0 | 3.5 | 1.8 | 30.1 |
| 0053-1-6 | 71.3 | 4.8 | 6.7 | 236.1 | 9.5 | 4.0 | 30.2 |
| 0053-4-1 | 296.1 | 13.6 | 4.6 | 1,255.2 | 93.2 | 7.4 | 24.3 |
| 0053-4-2 | 298.4 | 32.2 | 10.8 | 1,139.3 | 129.3 | 11.3 | 26.1 |
| 0053-4-3 | 275.1 | 48.3 | 17.6 | 1,057.6 | 127.9 | 12.1 | 26.5 |
| 0053-4-4 | 321.7 | 33.4 | 10.4 | 1,326.1 | 161.1 | 12.1 | 26.7 |
| 0053-4-5 | 325.2 | 33.7 | 10.4 | 1,172.0 | 119.0 | 10.2 | 27.8 |
| 0053-4-6 | 305.0 | 21.1 | 6.9 | 1,114.8 | 102.3 | 9.2 | 26.9 |
| 0053-4-7 | 264.0 | 18.7 | 7.1 | 1,002.3 | 95.0 | 9.5 | 26.0 |
| 0053-4-8 | 495.9 | 71.9 | 14.5 | 1,143.9 | 140.8 | 12.3 | 43.8 |
| 0053-4-9 | 474.1 | 45.9 | 9.7 | 1,100.5 | 113.4 | 10.3 | 45.0 |
| 0053-4-10 | 45.3 | 2.6 | 5.7 | 191.2 | 7.5 | 3.9 | 23.7 |
| 0053-4-11 | 54.2 | 2.6 | 4.8 | 202.5 | 8.0 | 3.9 | 26.8 |
| 0053-4-12 | 58.0 | 1.8 | 3.0 | 224.1 | 3.8 | 1.7 | 25.9 |
| 0053-4-13 | 292.7 | 20.7 | 7.1 | 1096.2 | 116.1 | 10.6 | 26.8 |

### Example 13: In Vitro Difluprednate Release Studies

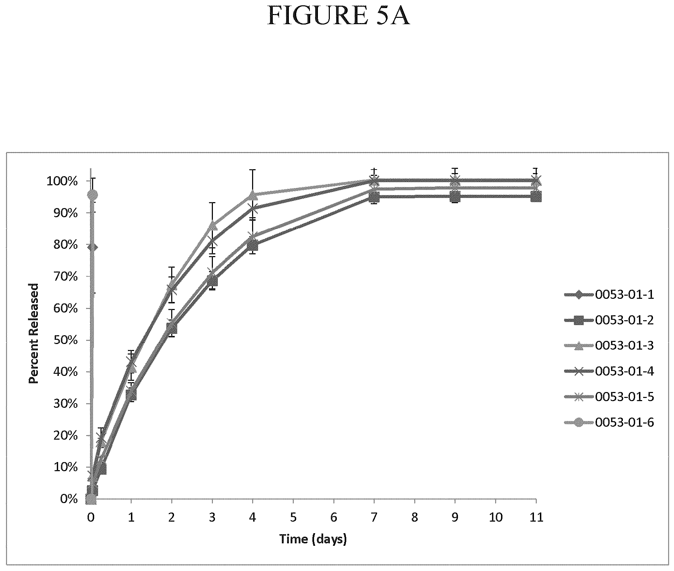

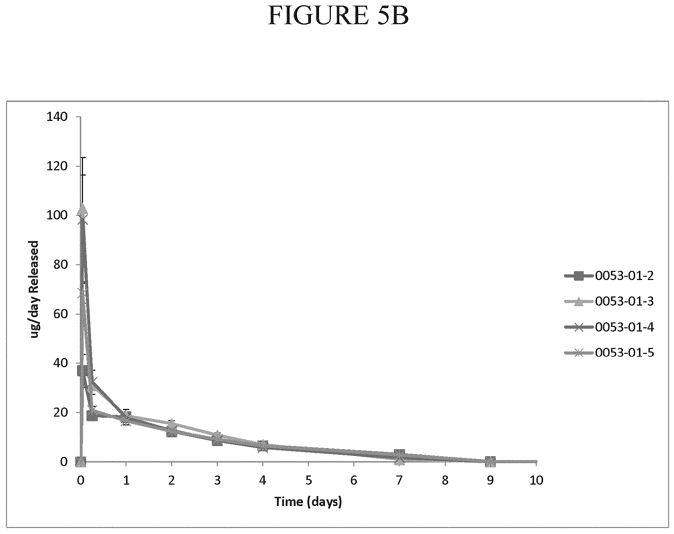

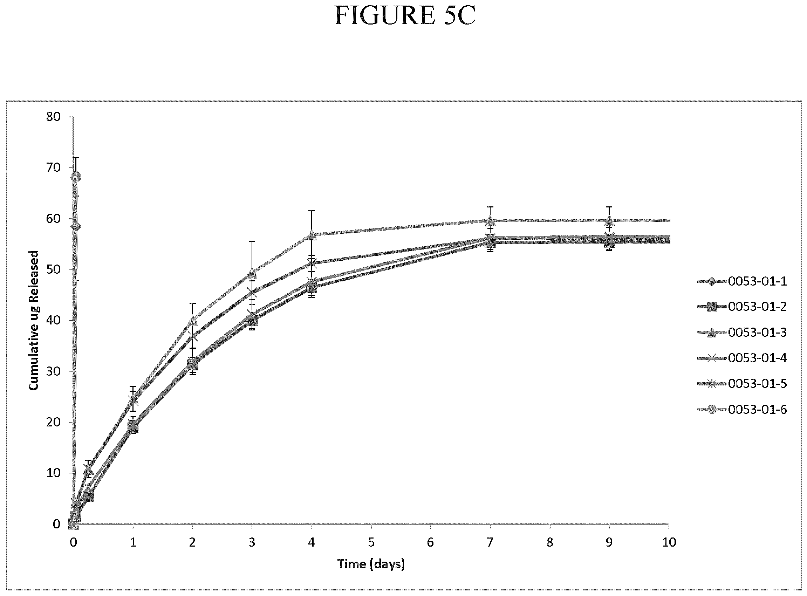

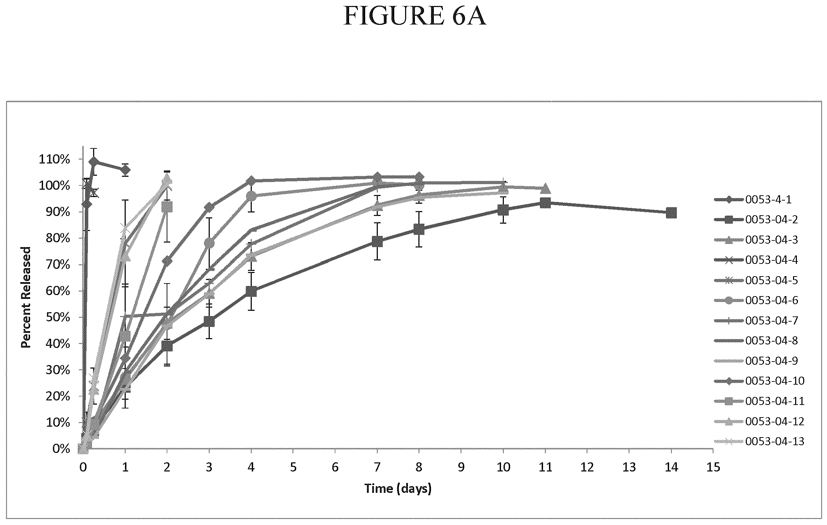

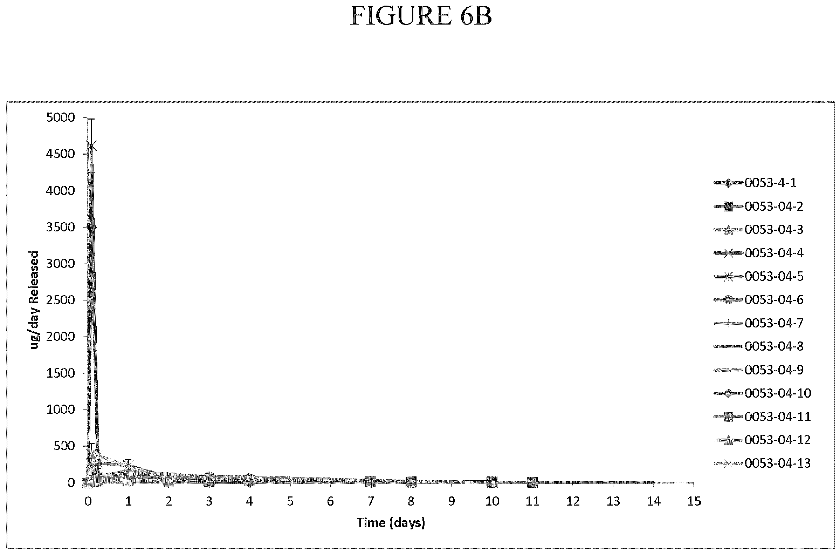

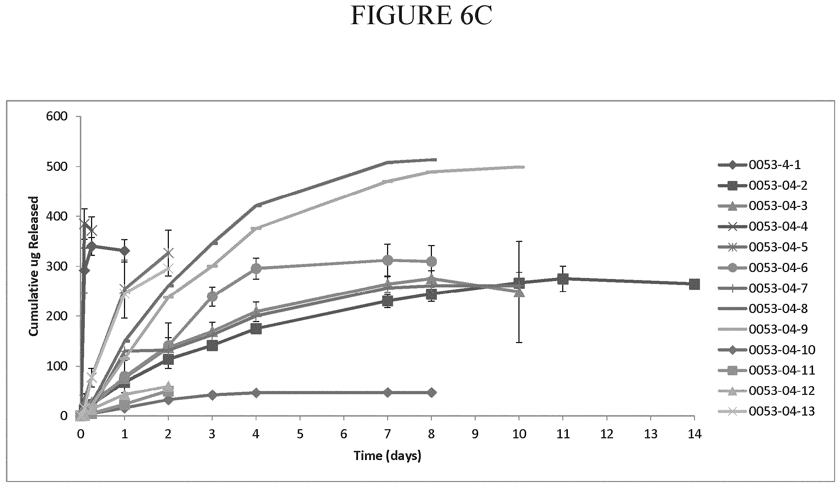

The in vitro release of difluprednate was determined for the implants of Example 11 according to the method of Example 6. Test results are shown in **FIG. 5A** through FIG. **5C****.** **FIG. 5A** shows the percent difluprednate released as a function of time (days) for samples under study. **FIG. 5B** shows the release rate (µg/day) for samples under study. Note that sample 0053-01-1 is not shown in this figure due to the rapid release of difluprednate. **FIG. 5C** shows the cumulative difluprednate (µg) released for samples under study. Test results are also shown in **FIG. 6A** through **FIG. 6C****.** **FIG. 6A** shows the percent difluprednate released as a function of time (days) for samples under study. **FIG. 6B** shows the release rate (µg/day) for samples under study. **FIG. 6C** shows the cumulative difluprednate (µg) released for samples under study.

All implants in **FIGS. 5A-5C** contained approximately 30wt% difluprednate and were about 225 µm X 225 µm X 4,000 µm in size. Most notable, is that use of increased levels of PEG35K or increased levels of PEG35K in conjunction with a PCL2K/PCL14K blend lead to rapid difluprednate release.

All samples in **FIG. 6A** through **6C** also contained approximately 30wt% difluprednate. Most samples were configured to about 400 µm X 400 µm X 6,000 µm in size, however, 00053-4-10, 00053-4-11, and 00053-4-12 were configured to about 225 µm X 225 µm X 4,000 µm. Samples 00053-4-5 and 00053-4-12; 00053-4-6 and 00053-4-11; 00053-4-7 and 00053-4-10 were similar composition pairs but differed in configuration.

### Example 14: Implants Utilized for In Vivo Studies

A thirteen day in vivo study utilizing New Zealand White Rabbits was conducted to determine the ocular tolerability and effect on induced ocular inflammation over the course of the study for a select group of samples in Example 11. **Table 14** details the IDs of the implants, number of implants utilized for dosing, and number of eyes dosed for the studies. Tables in the previous examples provide information as to composition and dimension for the implants. Topical administration of DUREZOL^{®} (difluprednate ophthalmic emulsion) (Alcon Laboratories, Inc.) was used as a positive control. The dosing paradigm was one drop per eye, four times daily for the duration of the study. For a negative control the surgery was conducted and a placebo implant (no difluprednate) was inserted.

Implants were placed subconjunctivally as described in Example 9. Two models were evaluated: paracentesis and corneal incision. In the paracentesis model, approximately 100 µL aqueous humor was withdrawn from samples on days 2 and 6. In the corneal incision model, 100 µL aqueous humor was withdrawn from animals on day 2. Additionally, corneal incision model groups had a 2.7 mm incision in the superior cornea sutured with 8-0 or 9-0 Vicryl on day 2.

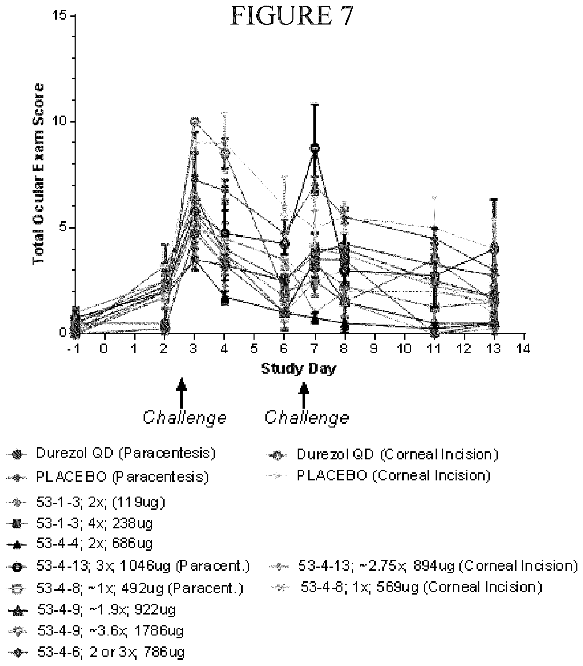

The Total Ocular Exam Score was also scored as described in Example 9. All corneal incision groups had any scoring related to inflammation surrounding sutures omitted from the Total Ocular Exam Score. **FIG. 7** depicts the results of the ocular examinations.

**Table 14: In Vivo Implants**

| ID | Implants Dosed/Eye # | Eyes Dosed # | Therapeutic Agent Content/Implant µg | Therapeutic Agent Total Content/Dose µg | Method of Delivery |
|---|---|---|---|---|---|
| Placebo Implant | 3 | | 0 | 0 | Subconjunctival Injection |
| Positive Control | 0 | | 50 | 50 | Topical Drops |
| 53-1-3 | 2 | | 59.5 | 118.9 | Subconjunctival Injection |
| 53-1-3 | 4 | | 59.5 | 237.8 | Subconjunctival Injection |
| 53-4-4 | 2 | | 343 | 686 | Subconjunctival Injection |
| 53-4-13 | 3 | | 349 | 1046 | Subconjunctival Injection |
| 53-4-8 | 1 | | 492 | 492 | Subconjunctival Injection |
| 53-4-9 | ~1.9 | | 485 | 922 | Subconjunctival Injection |
| 53-4-9 | ~3.6 | | 496 | 1786 | Subconjunctival Injection |
| 53-4-6 | 2 or 3 | | 393 | 786 | Subconjunctival Injection |
| Placebo Implant | 3 | | 0 | 0 | Subconjunctival Injection |
| Positive Control | 0 | | 50 | 50 | Topical Drops |
| 53-4-13 | 2.75 | | 325 | 894 | Subconjunctival Injection |
| 53-4-8 | 1 | | 569 | 569 | Subconjunctival Injection |

### Example 15: Polymer Matrix/Therapeutic Agent Blend Ratios

A series of polymer matrix/therapeutic agent blends were prepared prior to molding implants. All blends contained difluprednate as the therapeutic agent. **Table 15** details the composition of one series of blends.

**Table 15: Polymer Matrix/Therapeutic Agent Blend Ratios**

| ID | Polymer Matrix Target, % | | | | Therapeutic Agent Target, % |
|---|---|---|---|---|---|
| | PEG | | | Glycerol | Difluprednate |
| | PEG35K | PEG100K | PEGIMM | | |
| 00053.23-1 | | | 45.0 | 5.0 | 50.0 |
| 00053-23.2 | | | 50.0 | | 50.0 |
| 00053-23-3 | | | 50.0 | | 50.0 |
| 00053-23-4 | 49.0 | 21.0 | | | 30.0 |
| 00053-23-5 | 35.0 | 15.0 | | | 50.0 |
| 00053-23-6 | 35.0 | 15.0 | | | 50.0 |
| 00053-28-1 | 48.7 | 20.9 | | | 30.4 |
| 00053-28-2 | 34.9 | 15.0 | | | 50.1 |
| 00053-28-3 | | | 50 | | 50.0 |

### Example 16: Implant Fabrication

**Table 16** details the implants that were produced with the blends of Example 15, the molds of Example 2, and using the fabrication process of Example 3. The same ID used for the blend was also used for the resulting implant.

**Table 16 Implant Configurations**

| ID | Implant Dimensions |
|---|---|
| 00053.23-1 | 225 µm X 225 µm X 4,000 µm |
| 00053-23.2 | 225 µm X 225 µm X 4,000 µm |
| 00053-23-3 | 225 µm X 225 µm X 4,000 µm |
| 00053-23-4 | 225 µm X 225 µm X 4,000 µm |
| 00053-23-5 | 225 µm X 225 µm X 4,000 µm |
| 00053-23-6 | 225 µm X 225 µm X 4,000 µm |
| 00053-28-1 | 300 µm X 300 µm X 6,000 µm |
| 00053-28-2 | 300 µm X 300 µm X 6,000 µm |
| 00053-28-3 | 300 µm X 300 µm X 6,000 µm |

### Example 17: Analysis of Difluprednate Content

The difluprednate concentration of the implants was determined via detection of using HPLC as described in Example 5. The mass of individual implants was also determined. Table 17 details the test results for the implants fabricated in Example 16.

**Table 17: Difluprednate Content for Implants**

| ID | Difluprednate, µg | | | Overall Implant, µg | | | Difluprednate |
|---|---|---|---|---|---|---|---|
| | AVE | STDEV | %RSD | AVE | STDEV | %RSD | % |
| 00053.23-1 | 90.6 | 2.9 | 3.3 | 203.7 | 6.5 | 3.2 | 44.5 |
| 00053-23.2 | 93.7 | 4.2 | 4.4 | 227.6 | 11.5 | 5.1 | 41.2 |
| 00053-23-3 | 92.0 | 0.9 | 1.0 | 228.2 | 1.3 | 0.6 | 40.3 |
| 00053-23-4 | 51.1 | 1.1 | 2.1 | 218.4 | 1.5 | 0.7 | 23.4 |
| 00053-23-5 | 89.0 | 2.9 | 3.3 | 234.9 | 3.9 | 1.7 | 37.9 |
| 00053-23-6 | 91.8 | 2.5 | 2.7 | 245.3 | 2.9 | 1.2 | 37.4 |
| 00053-28-1 | 215.9 | 15.3 | 7.1 | 812.4 | 80.2 | 9.9 | 26.6 |
| 00053-28-2 | 384.3 | 12.0 | 3.1 | 821.4 | 22.2 | 2.7 | 46.8 |
| 00053-28-3 | 385.7 | 32.1 | 8.3 | 818.8 | 64.5 | 7.9 | 47.1 |

### Example 18: Implants Utilized for In Vivo Studies

A twenty-four day in vivo study utilizing New Zealand White Rabbits was conducted to determine the ocular tolerability and effect on induced inflammation over the course of the study for a select group of samples in Example 9. Table 18 details the IDs of the implants, number of implants utilized for dosing, and number of eyes dosed for the studies. Tables in the previous examples provide information as to composition and dimension for the implants. Topical administration of DUREZOL^{®} (difluprednate ophthalmic emulsion) (Alcon Laboratories, Inc.) was used as a positive control. The dosing paradigm for the positive control was one drop per eye, four times daily for the duration of the study. For a negative control the surgery was conducted and a placebo implant containing no difluprednate was inserted.

Implants were placed subconjunctivally as described in Example 9. Two models were evaluated: paracentesis and corneal incision. In the paracentesis model, approximately 100 µL aqueous humor was withdrawn from samples on days 2 and 6. In the corneal incision model, 100 µL aqueous humor was withdrawn from samples on day 2. Additionally, corneal incision model groups had a 2.7 mm incision in the superior cornea sutured with 8-0 or 9-0 Vicryl on day 2.

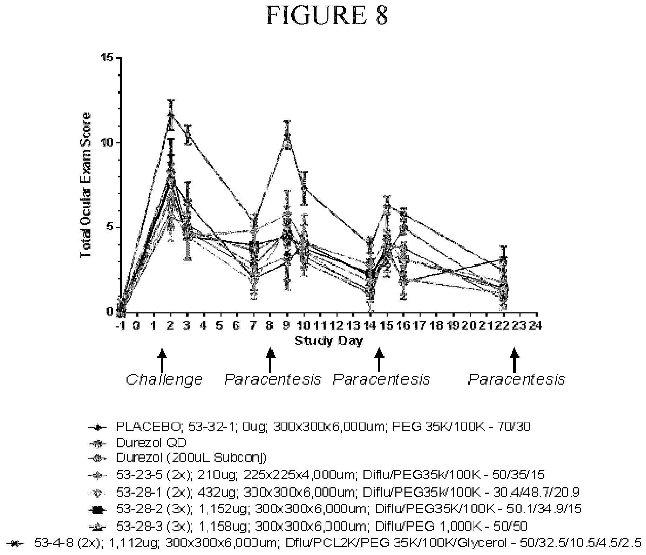

The Total Ocular Exam Score was also scored as described in Example 9. All corneal incision groups had any scoring related to inflammation surrounding sutures omitted from the Ocular Safety Index. FIG. 8 depicts the results of the ocular examinations.

**Table 18: In Vivo Implants**

| ID | Implants Dosed/Eye # | Eyes Dosed # | Therapeutic Agent Content/Implant µg | Therapeutic Agent Total Content/Dose µg |
|---|---|---|---|---|
| Placebo Implant | 2 | 6 | 0 | 0 |
| Positive Control (Topical) | 0 | 6 | 50 | 200 |
| Positive Control (Injected) | 0 | 6 | 50 | 50 |
| 53-23-5 | 2 | 6 | 105 | 210 |
| 53-28-1 | 2 | 6 | 216 | 432 |
| 53-28-2 | 3 | 6 | 384 | 1152 |
| 53-28-3 | 3 | 6 | 386 | 1158 |
| 53-4-8 | 2 | 6 | 556 | 1112 |

### Example 19: ENV905 Difluprednate Ophthalmic Implant Studies

The present example demonstrates an embodiment of the disclosure, termed ENV905, which is a difluprednate ophthalmic implant for the treatment of inflammation and pain associated with ocular surgery.

ENV905 (difluprednate) ophthalmic implant is an injectable difluprednate implant formulation using a biocompatible polyethylene glycol (PEG)-based drug delivery system. The implant is designed for ophthalmic administration via subconjunctival (SCJ) or intracameral (IC) injection, with a single dose administered following ocular surgery targeting duration of action of 28 days. The PEG-based drug delivery system is comprised of a blend of two PEGs that function as both diluent and binder, and was designed for rapid dissolution of the implant and concurrent release of difluprednate following administration.

The bioavailability and sustained therapeutic effect of ENV905 over 28 days is governed by multiple factors, including route of administration and the physicochemical properties of the drug substance difluprednate.

The present example illustrates the functionality of various formulations of ENV905 ocular implants. Included in the various embodiments of ENV905 ocular implants tested are ENV905-1 (also designated by sample ID No. **"115-8-7")** and ENV905-2 (also designated by sample ID No. **"115-16-1").**

Each formulation was designed according to its intended route of administration, either IC (ENV905-1) or SCJ (ENV905-2). These two embodiments are designed to decrease ocular inflammation and pain for 28 days following a single administration via SCJ or IC injection, with the intended range of doses from approximately 40 to 80 µg (IC) or 400 to 800 µg (SCJ) difluprednate.

### Characterization and Overview of ENV905

ENV905 is a biocompatible implant formulation containing the corticosteroid difluprednate in a PEG-based drug delivery system. ENV905 is formulated as a solid, rod-shaped implant of dimensions between 225-350 µm in width by 2,925-6,000 µm in length.

The aforementioned ENV905-1 formulation embodiment is designed for intracameral delivery into the anterior chamber of the eye. The IC implant ENV905-1 will enable targeted delivery of difluprednate directly into the anterior chamber, providing a potentially dose-sparing effect compared to DUREZOL^{®}. The IC ENV905-1 implant was designed to rapidly disintegrate in the anterior chamber, providing a therapeutic effect over approximately 28 days.

The aforementioned ENV905-2 formulation embodiment is designed for implantation in the subconjunctival space. The SCJ implant ENV905-2 was designed to rapidly disintegrate in the subconjunctival space, providing a therapeutic effect over approximately 28 days.

The two formulation embodiments differ in size and dose of difluprednate, while maintaining the same formulation characteristics of PEG excipients. The characteristics of these implants can be found, *inter alia,* in the below **Tables 19** and **20.**

**Table 19: ENV905-1: Intracameral Ophthalmic Implant Formulation (Nominal Ranges)**

| **ENV905-1 Intracameral Implant Formulation Nominal Dimensions: 225 µm x 225 µm x 2,925 µm** | | | | |
|---|---|---|---|---|
| **Component/Property** | | **Function** | **Amount (µg/implant)** | **% w/w** |
| | **Difluprednate** | Active | 24 to 56 | 15 to 35 |
| | **Total PEG (PEG 3,350** and **PEG 100,000)** | Diluent / Binder | 104 to 136 | 65 to 85 |
| | **PEG 3,350** | | 11.4 to 15.0 | 7.2 to 9.4 |
| | **PEG 100,000** | | 92.6 to 121.0 | 57.9 to 75.7 |
| | **Ratio of PEG 3,350/ PEG 100,000** | | 11% / 89% w/w | |
| | **Total Implant Mass** | - | 128 to 192 | - |

**Table 20: ENV905-2: Subconjunctival Ophthalmic Implant Formulation (Nominal Ranges)**

| **ENV905-2 Subconjunctival Implant Formulation Nominal Dimensions: 300 µm x 300 µm x 6,000 µm** | | | | |
|---|---|---|---|---|
| **Component/Property** | | **Function** | **Amount (µg/implant)** | **% w/w** |
| | **Difluprednate** | Active | 320 to 480 | 40 to 60 |
| | **Total PEG (PEG 3,350 and PEG 100,000)** | Diluent / Binder | 320 to 480 | 40 to 60 |
| | **PEG 3,350** | | 35.2 to 52.8 | 4.4 to 6.6 |
| | **PEG 100,000** | | 284.8 to 427.2 | 35.6 to 53.4 |
| | **Ratio of PEG 3,350/ PEG 100,000** | | 11% / 89% w/w | |
| | **Total Implant Mass** | - | 640 to 960 | - |

**Table 21: ENV905-3: Intracameral Ophthalmic Implant Formulation (Nominal Ranges)**

| **ENV905-3 Intracameral Implant Formulation Nominal Dimensions: 225 µm x 225 µm x 2,925 µm** | | | | |
|---|---|---|---|---|
| **Component/Property** | | **Function** | **Amount (µg/implant)** | **% w/w** |
| | **Difluprednate** | Active | 16 to 24 | 10 to 15 |
| | **Total PEG (PEG 3,350** and **PEG 100,000)** | Diluent / Binder | 136 to 144 | 85 to 90 |
| | **PEG 3,350** | | 15.0 to 15.8 | 9.4 to 9.9 |
| | **PEG 100,000** | | 121.0 to 128.2 | 75.7 to 80.1 |
| | **Ratio of PEG 3,350/ PEG 100,000** | | 11% / 89% w/w | |
| | **Total Implant Mass** | - | 152 to 168 | - |

**Table 22: ENV905-4: Intracameral Ophthalmic Implant Formulation (Nominal Ranges)**

| **ENV905-4 Intracameral Implant Formulation Nominal Dimensions: 225 µm x 225 µm x 2,925 µm** | | | | |
|---|---|---|---|---|
| **Component/Property** | | **Function** | **Amount (µg/implant)** | **% w/w** |
| | **Difluprednate** | Active | 8 to 12 | 5.0 to 7.5 |
| | **Total PEG (PEG 3,350 and PEG 100,000)** | Diluent / Binder | 148 to 152 | 92.5 to 95.0 |
| | **PEG 3,350** | | 16.3 to 16.7 | 10.2 to 10.5 |
| | **PEG 100,000** | | 131.7 to 135.3 | 82.3 to 84.6 |
| | **Ratio of PEG 3,350/ PEG 100,000** | | 11% / 89% w/w | |
| | **Total Implant Mass** | - | 156 to 164 | |

**Table 23: ENV905-5: Intracameral Ophthalmic Implant Formulation (Nominal Ranges)**

| **ENV905-5 Intracameral Implant Formulation Nominal Dimensions: 130 µm x 180 µm x 1,500 µm** | | | | |
|---|---|---|---|---|
| **Component/Property** | | **Function** | **Amount (µg/implant)** | **% w/w** |
| | **Difluprednate** | Active | 16 to 24 | 29.6 to 44.4 |
| | **Total PEG (PEG 3,350 and PEG 100,000)** | Diluent / Binder | 30 to 38 | 55.6 to 70.4 |
| | **PEG 3,350** | | 3.3 to 4.2 | 6.1 to 7.7 |
| | **PEG 100,000** | | 26.7 to 33.8 | 49.4 to 62.6 |
| | **Ratio of PEG 3,350/ PEG 100,000** | | 11% / 89% w/w | |
| | **Total Implant Mass** | - | 46 to 62 | |

ENV905 implants can be loaded into the needle of a single-use implant applicator and delivered directly into either the subconjunctival space or the anterior chamber. ENV905 was designed to deliver therapeutic levels of difluprednate for approximately 28 days in certain aspects.

ENV905 implants, of various configurations, have been well tolerated in efficacy and tolerability studies in albino rabbits following administration by their respective routes of delivery. This generalization will be supported by the below data.

Following subconjunctival insertion, ENV905-2 implants remain at the insertion site, cause no apparent discomfort, and rapidly disintegrate. Following IC insertion, ENV905-1 implants localize to the inferior iridocorneal angle upon insertion, remain largely immobile, and quickly disintegrate. ENV905 has been shown to decrease ocular inflammation in New Zealand white (NZW) rabbit models of post-operative inflammation, with excellent safety and tolerability profiles following both SCJ and IC administration.

### Potential Mechanism of Action for ENV905 Embodiments

Without wishing to be bound to a particular mechanistic theory of action, the following description provides one possible mechanism of action for the ENVA905 ocular implants disclosed herein.

Corticosteroids inhibit the inflammatory response to a variety of inciting agents. They inhibit edema, fibrin deposition, capillary dilation, leukocyte migration, capillary proliferation, fibroblast proliferation, deposition of collagen, and scar formation associated with inflammation.

Corticosteroid activity is mediated by intracellular activation of GR. Binding of the corticosteroid ligand results in translocation of the ligand-bound GR from the cell cytosol into the nucleus, where it functions as a transcription factor and binds to the glucocorticoid response elements in the promoter regions of responsive genes or interacts directly with other transcription factors. The actions results in consequent anti-inflammatory effects due to down-regulation of pro-inflammatory molecule production.

In the eye, corticosteroids are thought to act by the induction of phospholipase A2 inhibitory proteins, collectively called lipocortins. It is postulated that these proteins control the biosynthesis of potent mediators of inflammation such as prostaglandins and leukotrienes by inhibiting the release of their common precursor arachidonic acid. Arachidonic acid is released from membrane phospholipids by phospholipase A2. Difluprednate is structurally similar to other corticosteroids (DUREZOL^{®} Package Insert). DUREZOL^{®} is a topical corticosteroid that is indicated for the treatment of inflammation and pain associated with ocular surgery as well as the treatment of endogenous anterior uveitis. DUREZOL^{®} is formulated as a 0.05% difluprednate sterile preserved emulsion, and the treatment regimen is instillation of one drop into the conjunctival sac 4 times daily for 14 days, followed by a tapering regimen as clinically indicated.

The ENV905-1 and ENV905-2 embodiments can decrease ocular inflammation and/or pain for 28 days following a single administration via SCJ or IC injection, by effectively delivering difluprednate to targeted areas of the eye, rather than via the problematic topical application required for DUREZOL^{®}.

### Nonclinical Pharmacology of Difluprednate in Ocular Formulations

Difluprednate, a corticosteroid pro-drug of the active metabolite 6α,9-difluoroprednisolone 17-butyrate (DFB), is marketed as a 0.05% sterile preserved emulsion for topical ocular delivery. DUREZOL^{®} is administered as a 4 times per day drop (approximately 100 µg per day) in patients recovering from ocular surgery or patients with endogenous anterior uveitis.

Corticosteroid eye drops have been in use since the 1950s for controlling ocular inflammation. Extensive clinical experience attests to the efficacy of these compounds for treating anterior segment inflammation (Gaudio 2004). Controlling inflammation is one of the key objectives following ocular surgery. Difluprednate is a potent corticosteroid that is regularly used by physicians following ocular surgery in the US standard of care. A glucocorticoid (GC) receptor-binding test was performed to evaluate GC receptor-binding activity (GCRBA) of glucocorticoids. Difluprednate's active metabolite DFB had the highest affinity for the GR with the lowest inhibition constant (Kᵢ) when compared with prednisolone, betamethasone, fluorometholone, and dexamethasone **(Table 21)** (Tajika 2011b). The greater binding affinity may be attributed to the unique molecular structure of difluprednate. Difluprednate is a derivative of prednisolone, but differs substantially due to structural modifications. The addition of two fluorines and a C-17 butyrate directly increase the affinity of difluprednate for the GR (Donnenfeld 2011).

**Table 21: Kᵢ Values of Difluprednate, Its Metabolite, and Various Glucocorticoids**

| **Compound** | | **Kᵢ Value (mol/L)** |
|---|---|---|
| | Difluprednate (DFBA) | 7.8 x 10⁻¹⁰ ± 3.6 x 10⁻¹¹ |
| | Desacetyl Difluprednate (DFB) | 6.1 x 10⁻¹¹ ± 1.2 x 10⁻¹¹ |
| | Prednisolone | 3.4 x 10⁻⁹ ± 7.3 x 10⁻¹⁰ |
| | Betamethasone | 1.7 x 10⁻⁹ ± 3.2 x 10⁻¹⁰ |
| | Dexamethasone | 1.9 x 10⁻⁹ ± 2.3 x 10⁻¹⁰ |

Studies of topical difluprednate in rabbits show that it is effective in models of post-operative inflammation when applied 4 times daily (QID; DUREZOL^{®} NDA 22-212). Difluprednate has been studied in humans, and the efficacy and safety of topical ocular administration of DUREZOL^{®} is established (Korenfeld 2009, Foster 2010).

### Proposed Manufacturing Protocol of ENV905

Based upon the data contained in the present disclosure, the inventors anticipate large scale production of the ENV905 product. Both PEG 3,350 and PEG 100,000 will be manufactured in a current Good Manufacturing Practices (cGMP)-compliant process and comply with current National Formulary (NF) compendia specifications (USP 37-NF 32 Monographs for Polyethylene Glycol and Polyethylene Oxide). The ENV905 implant will be compounded and packaged in a low bioburden environment and may be terminally sterilized by gamma irradiation.

### Description of Manufacturing Process and Process Controls for ENV905

Manufacture of ENV905 (difluprednate) ophthalmic implant is a well-controlled, multistep process used to produce implants composed of micronized difluprednate contained within a biocompatible PEG drug delivery system.

The process begins with the fabrication and sterilization of the mold template that will be used to form the final implant geometry. The mold is formed by patterning a series of cavities into an ultraviolet (UV) curable polymer material. The resulting mold is a flexible sheet with an array of cavities of uniform size and shape. The mold sheet is then sterilized by gamma irradiation at Steris Isomedix, Inc. prior to use. A polyethylene terephthalate (PET) backing sheet used in the molding process is also sterilized by gamma irradiation at Steris Isomedix.

Micronized difluprednate is first compounded with the two PEGs at a specified ratio to create a homogeneous blend. The blend is then heated above the melting point of the PEGs to allow for the PEGs to freely flow creating a homogenous solid dispersion with the micronized difluprednate. The ENV905 (difluprednate) ophthalmic implant is then formed by thermally molding the resulting difluprednate/PEG blend into the desired geometrical shape using the pre-sterilized mold and backing sheet. This manufacturing step is performed in an International Standard for Organization (ISO) 5 environment. The final implant is prism-shaped with a size defined by the mold sheet cavities. The formed implants are then manually transferred in an ISO 5 environment into a pre-sterilized container closure. The packaged implants will then be terminally sterilized via gamma irradiation.

### Implant Mold Fabrication

The final shape of the ENV905 (difluprednate) ophthalmic implant is defined by a molding process which uses a polymer mold template which consists of a polymer coating (referred to as SAE-04) that is applied to a backing sheet of PET. The SAE-04 material is a UV-curable silicone that can be patterned to form mold cavities in the desired shape and size of the implant.

The mold-making process begins with a master template fabricated on a silicon wafer through a process known in the microelectronics industry as photolithography. This master template consists of an array of uniformly sized and shaped features having nominally the same size and geometry as the final implant.

Through a series of replication steps referred to as mold making, the master template is then used to create a polymer mold template. First, the PET backing sheet is coated with a thin layer of an acrylate polymer to serve as an adhesive promoting tie-layer. This acrylate layer ensures that the SAE-04 mold adheres to the PET backing layer. Liquid SAE-04 monomer is premixed with a photo-initiator referred to as TMP (diphenyl(2,4,6-trimethylbenzoyl)phosphine oxide) to drive the polymerization reaction during mold making. A small volume of SAE-04 solution is dispensed onto the PET backing sheet to form a film, and is covered with the master template. The sample is then exposed to UV light (λ_{avg}=265 nm) for 60 to 120 seconds to polymerize the mold film while in contact with the pattern on the master template. This creates a series of rectangular cavities in the mold film. The solidified mold and PET backing layer are then peeled off of the master template to create a free-standing mold.

The mold is then inspected visually for macroscopic defects and mold cavity dimensions are verified using optical profilometry to measure the cavity depth. The mold is then double-bagged in self-sealing sterilization pouches and shipped to an external vendor for sterilization using gamma irradiation.

### Preparation of Difluprednate/PEGs Blend for ENV905

All manufacturing procedures are performed at ambient temperature unless otherwise specified. Micronized difluprednate, PEG 3,350, and PEG 100,000 are all received as dry powders. The individual components are weighed and mixed at a given ratio. They are first thoroughly mixed as a dry powder blend by mechanical agitation. To ensure a homogenous blend, the dry powder blend is then heated above the melting points of the polymers. The polymers can then freely flow and form a homogeneous solid dispersion with the micronized difluprednate. The homogenous difluprednate/PEG blend solidifies as it cools down back to room temperature.

### Preparation of Molded Implant

Individual implants are formed by heating the difluprednate/PEG blend and allowing it to flow into the cavities of the mold. **Table 24** includes proposed processing parameters. This process takes place in two BSC hoods (ISO 5 environment). The difluprednate/PEG blend is placed on the PET sheet and placed on a hot plate for 120-180 seconds. Once heated, the blend is covered with the Fluorocur^{®} (FCR) mold sheet. Light pressure is applied using a hand roller to laminate the two sheets together. The mold/blend laminate is then passed through a commercially available thermal laminator at a specified temperature, speed, and pressure. Under these conditions the difluprednate/PEG blend is able to flow into the mold cavities, filling the cavities, and assuming the shape of the mold cavities. The laminate containing the difluprednate/PEG blend in the mold cavities is then allowed to cool back down to room temperature. The difluprednate/PEG blend then solidifies forming individual, discrete implants. The mold can then be removed, leaving a two-dimensional array of discrete implants on the PET film.

**Table 24: ENV905 Implant Fabrication Conditions**

| **Process Parameter** | | **Proposed Range** |
|---|---|---|
| | Hot Plate Temp (°C) | 120 - 160 |
| | Hot Plate Time (sec) | 120 - 180 |
| | Laminator Temp (°F) | 260 - 320 |
| | Laminator Speed (ft/min) | 0.1 - 1 |
| | Laminator Pressure (psi) | 40 - 80 |
| | Number of Lamination Passes | 4 - 8 |
| | Cooling time (min:sec) | NLT 3:00 |

### Implant Packaging

It is envisioned that in an embodiment the packaging for the ENV905 implants will be a blister pack comprised of a thermoformed film with each well containing individual dosage units, foil or foil laminate lidding, and a pressure-seal coating. Individual implants will be manually removed from the PET backing sheet using stainless steel tweezers. Alternatively, sterile vials may be used. Each implant will be visually inspected for macroscopic defects before placing it in its final packaging. It is envisioned that the ENV905 implants will be preloaded into an ENV905 Implant Applicator, with the needle hub assembly of the Applicator functioning as a container closure for the implants.

### Sterility Assurance

Terminal moist heat sterilization is not a viable method due to the fact that ENV905 is a solid dosage form designed to disintegrate in aqueous environments. Therefore, studies to evaluate gamma irradiation were conducted on early ENV905 formulations to determine if ionizing radiation was a viable option for a terminally sterilized drug product. ENV905 implants were exposed to gamma irradiation with a dose of 46 kGy per ISO 11137-2:2012 (Sterilization of Health Care Products - Radiation - Part 2: Establishing the Sterilization Dose). The implant formulations used for these studies consisted of a blend of difluprednate and PEGs. The compositions and sizes of the implant formulations used in the study are provided in **Table 25** below.

**Table 25: Difluprednate Implant Formulations (ENV-11-115-8)**

| **Formulation ID** | **Implant Dimensions (µm)** | **Mean Difluprednate Content (µg) (n =10)** | **SD (µg)** | **% RSD** | **PEG Ratio (PEG 3,350 / PEG 100,000)** |
|---|---|---|---|---|---|
| ENV-1I-115-8-3 | 225 x 225 x 4,000 | 97.2 | 2.6 | 2.7 | q.s. ad 11% / 89% |
| ENV-1I-115-8-5 | 225 x 225 x 2,925 | 74.1 | 1.3 | 1.7 | q.s. ad 11% / 89% |
| ENV-1I-115-8-7 | 225 x 225 x 2,925 | 38.9 | 1.0 | 2.5 | q.s. ad 11% / 89% |

Implants were individually packaged in HPLC vials with air headspace and polytetrafluoroethylene (PTFE)-lined screw caps. Ten individual implants (n = 10) were tested for difluprednate assay. The results are summarized in **Table 26.**

**Table 26: Impact of Gamma Irradiation (46 kGy) on Difluprednate Assay on Difluprednate/PEG Implant Formulations**

| **Formulation** | **Radiation Dose (kGy)** | **Mean Difluprednate Content (µg/implant) (n = 10 implants)** | **SD (µg)** | **Difluprednate Assay (% Area)** | **Degradation (% Area)** |
|---|---|---|---|---|---|
| Neat API | None | - | - | 97.98 | - |
| | 46 | - | - | 97.13 | 0.85 |
| Implant ENV-1I-115-8-3 | None | 97.2 | 2.6 | 97.19 | - |
| | 46 | 101.2 | 5.5 | 96.80 | 0.39 |
| Implant ENV-1I-115-8-5 | None | 74.1 | 1.3 | 97.54 | - |
| | 46 | 73.1 | 5.1 | 97.01 | 0.53 |
| Implant ENV-1I-115-8-7 | None | 38.9 | 1.0 | 97.92 | - |
| | 46 | 40.1 | 1.4 | 97.31 | 0.61 |

Gamma irradiation under standard sterilization conditions (46 kGy) resulted in 0.39% to 0.61% degradation of difluprednate in the drug product, and 0.85% degradation of neat difluprednate.

The effect of gamma irradiation disintegration time and difluprednate content in media upon full disintegration of the implants was also evaluated. Implants were individually packaged in HPLC vials with air headspace and PTFE-lined screw caps. Five (n=5) implants were tested for difluprednate assay in the media after a prescribed time of 5 minutes to achieve full disintegration. The results are summarized in **Table 27.**

**Table 27: Impact of Gamma Sterilization on In Vitro Difluprednate Disintegration Test. Disintegration Medium: 0.01 M Phosphate Buffered Saline, 1% SDS; Temperature = 20°C**

| **Formulation** | **Radiation Dose (kGy)** | **Mean Difluprednate Content Measured in Media upon Full Implant Disintegration (µg/implant) (n =5 implants)** | **SD (µg)** |
|---|---|---|---|
| ENV-1I-115-8-3 | None | 103.3 | 4.3 |
| | 46 | 104.4 | 5.0 |
| ENV-1I-115-8-5 | None | 73.2 | 3.3 |
| | 46 | 72.4 | 4.8 |
| ENV-1I-115-8-7 | None | 40.7 | 1.1 |
| | 46 | 40.6 | 0.8 |

Gamma irradiation did not statistically impact the amount of difluprednate recovered in the media after full disintegration. Furthermore, gamma irradiation did not have an impact on the time required to achieve full disintegration in media (t ≤5 minutes).

Based on these preliminary sterilization studies described above, the inventors contemplate that in some embodiments they will terminally sterilize ENV905 drug product using gamma irradiation. A sterility assurance level of 10⁻⁶ or better will be demonstrated for the developed cycle.

### Raw Materials and Proposed Excipient Specifications for ENV905

The raw materials and proposed specifications for the PEG excipients used to manufacture ENV905 (difluprednate) implants are summarized in **Tables 28-31** below. The PEG excipients will be in conformance with NF compendial specifications.

**Table 28: List of Raw Materials for ENV905**

| **Material Name (product number)** | **Purpose** |
|---|---|
| Difluprednate | Active |
| Poly(ethylene glycol) 3,350 | Diluent |
| Poly(ethylene glycol) 100,000 | Diluent/Binder |

**Table 29: List of Raw Materials for Mold Fabrication**

| **Material Name (product number)** | **Purpose** |
|---|---|
| Polyethylene terephthalate (PET), 5 mil (Melinex 453) | Substrate Backing Layer |
| SR9012 (tri-functional acrylate ester) | Mold Tie Layer Component |
| DPT; (Diphenyl(2,4,6-trimethylbenzoyl) phosphine oxide/ 2-hydroxy-2-methylpropiophenone) | Mold Tie Layer Component |
| Fluorocur-SAE-04; Acrylated Polysiloxanes; Siloxanes and Silicones, di-Me, hydrogen terminated, reaction products with acrylic acid and 2-ethyl-2- [(2-propenyloxy)methyl] -1, 3-propanol | FCR Mold Resin Component |
| TMP; Diphenyl(2,4,6-trimethylbenzoyl)phosphine oxide (CAS# 75980-60-8) | FCR Mold Resin Component |

**Table 30: Proposed Specifications for Poly(ethylene glycol); 3,350 MW**

| **Test** | **Proposed Specification** | **Method Description** |
|---|---|---|
| Physical Appearance | White to off - white powder | Visual |
| Average Molecular Weight | 90.0 to 110.0% of nominal value | Titrimetric (NF Monograph) |
| Residue on Ignition | NMT 0.1% | USP<281> |
| Heavy Metals | NMT 5 ppm | USP<231> |
| Free Ethylene Dioxide | NMT 10 µg/g | Headspace GC - FID (NF Monograph) |
| Free 1,4-Dioxane | NMT 10 µg/g | Headspace GC - FID (NF Monograph) |
| pH (5.0 g of Polyethylene Glycol in 100 mL of carbon dioxide-free water) | 4.5 to 7.5 | USP<791> |
| Completeness and Color of Solution | Colorless and Clear | NF Monograph |
| Viscosity | 9 to 15 cPs (25% w/w, H₂O at 25 °C) | Viscometry |

| Microbial Limits | | |
|---|---|---|
| Total microbial count | ≤ 2000 CFU/g | USP<61> |
| Yeasts and molds | ≤ 200 CFU/g | |
| Tests for specified microorganisms | Absence of S. aureus, E. coli, Salmonella, P. aeruginosa | USP<62> |

**Table 31: Proposed Specifications for Poly(ethylene glycol); 100,000 MW**

| **Test** | **Proposed Specification** | **Method Description** |
|---|---|---|
| Physical Appearance | White to off - white powder | Visual (NF Monograph) |
| Infrared Absorption | Spectrum consistent with reference standard | USP<197K> |
| Viscosity | 12 to 50 cPs (c=5%, H₂O, at 25 °C) | Viscometry |
| Heavy Metals | NMT 10 ppm | USP<231> Method II |
| Silicon Dioxide Residue on Ignition | NMT 3% | NF Monograph |
| Non-Silicon Dioxide Residue on Ignition | NMT 2% | NF Monograph |
| Free Ethylene Dioxide | NMT 0.001% | Headspace GC - FID (NF Monograph) |
| Loss On Drying | NMT 1.0% | USP<731> |

| Microbial Limits | | |
|---|---|---|
| Total microbial count | ≤ 2000 CFU/g | USP<61> |
| Yeasts and molds | ≤ 200 CFU/g | |
| Tests for specified microorganisms | Absence of S. aureus, E. coli, Salmonella, P. aeruginosa | USP<62> |

### Nonclinical Pharmacology of ENV905

Multiple pharmacology studies of various formulations of ENV905 have been conducted in albino rabbit models of ocular post-operative inflammation.

Animal models included corneal incision, lens phacoemulsification, and anterior chamber paracentesis. The effect of ENV905, placebo implant, or positive control DUREZOL^{®} on induced ocular inflammation was assessed over approximately 1 month, and the results are discussed in this section.

The anti-inflammatory effects of topical difluprednate have been established, and the kinetics of the deacetylation of difluprednate to its active metabolite desacetyl difluprednate (DFB) in vivo are known (Tajika 2011a, Tajika 2011b).

However, the pharmacology of extended release SCJ or IC delivery of difluprednate has not been studied, although delivery of other corticosteroids to these compartments demonstrates a reduction in ocular inflammation (Croasdale 1999, Deileman 2011, Koval 2014).

### Nonclinical Pharmacology of ENV905 - General Experimental Protocol

Four non-Good Laboratory Practices (GLP) efficacy and tolerability studies of ENV905 formulations have been completed in NZW rabbits **(Table 32).**

On Day 1, animals received either a bilateral corneal incision, which mimics the incision encountered during cataract surgery (studies ENV905-PRE-003, ENV905-PRE-006, and ENV905-PRE-008), or lens phacoemulsification (study ENV905-PRE-004), and were given a single bilateral administration of ENV905 or placebo implant concurrent with the surgical procedure.

DUREZOL^{®} administered topically 4 times per day starting at the time of surgery served as the positive control. The dosing paradigm for DUREZOL^{®} was one drop per eye, four times daily for the duration of the study. For a negative control, the surgery was conducted and a placebo implant (difluprednate-free) was inserted.

Animals were observed for general tolerability endpoints, and received ophthalmic exams at regular intervals during the study. As these models generally induce ocular inflammation for a limited amount of time, additional inflammation induction in the form of paracentesis (removal of an aliquot of aqueous humor) were conducted at approximately weekly intervals to enable assessment of the duration of anti-inflammatory effect of ENV905.

Specifically, for the ENV905-PRE-006 study, anterior chamber paracentesis was conducted on Days 9, 15, and 22 to induce ocular inflammation. Eyes were examined on Days -2 (baseline), 2, 3, 4, 9, 10, 11, 15, 16, 17, 22, 23, and 24 by a board certified veterinary ophthalmologist and were graded using the Hackett and McDonald Ophthalmic Exam Scoring System. *See,* Hackett, RD and McDonald, TO. Ophthalmic Toxicology and Assessing Ocular Irritation. Dermatoxicology, 5th edition. Ed. FN Marzulli and HI Maibach. Washington, DC: Hemisphere Publishing Corporation. 1996; 299-305 and 557-566.

Multiple formulations of ENV905 have been assessed in the pharmacology studies, and both routes of administration, subconjunctival and intracameral, were employed **(Tables 33-35).**

Specifically, ENV905 implants were assessed for effect on induced ocular inflammation in NZW rabbits following a single SCJ or IC dose administration on Day 1 **(Table 32** and **33).** Custom implant applicators with 21 G to 27 G needles were used to administer the implants. Placebo implant formulations were used as negative controls, and DUREZOL^{®} dosed QID throughout the study served as the positive control group.

Ocular inflammation was induced by one of two methods: a 2.7 mm clear corneal incision approximately 1 mm from the limbus; a phacoemulsification procedure involving a surgical 2.7 mm clear corneal incision at the superior limbus, a central curvilinear capsularexis, and removal of the lens cortex and nucleus by phacoemulsification and aspiration. Additionally, as the induced ocular inflammation was relatively short lived and in order to assess effect on inflammation at later time points, animals also received anterior chamber paracentesis at predetermined time points, involving a 30 G needle placed through the limbus and removal of aqueous humor, at pre-determined time points during each study. General tolerability endpoints included body weights and daily clinical observations. A board-certified veterinary ophthalmologist performed ophthalmic exams and McDonald-Shadduck scoring to assess ocular inflammation.

**Table 32: Summary of Completed Pharmacology Studies of ENV905**

| **Model** | **Duration (Days)** | **Species** | **No. of Animal s/ Group** | **Regulatory Status** | **Study Number** |
|---|---|---|---|---|---|
| Corneal Incision and Paracentesis | 24 | NZW Rabbit | 2-4 | Non-GLP | ENV905-PRE-003 |
| | 25 | NZW Rabbit | 2-4 | Non-GLP | ENV905-PRE-006 |
| | 31 | NZW Rabbit | 4 | Non-GLP | ENV905-PRE-008 |
| Phacoemulsification and Paracentesis | 28 | NZW Rabbit | 2-4 | Non-GLP | ENV905-PRE-004 |

**Table 33: ENV905 Formulations Tested in Pharmacology Studies in New Zealand White Rabbits**

| **ENV905 Formulation ID** | **PEG Ratio** | **Implant Dimensions (µm)** | **Number of Implants/ Eye** | **Total Dose (µg difluprednate/ Eye)** | **Route of Administration** | **Study Number** |
|---|---|---|---|---|---|---|
| ENV-11-53-32-1 | PEG 35K / PEG 100K (70/30) | 300 x 300 x 6,000 | 2 | 0 | SCJ | ENV905-PRE-003 |
| ENV-11-53-23-5 | PEG 35K / PEG 100K (70/30) | 225 x 225 x 4,000 | 2 | 210 | SCJ | ENV905-PRE-003 |
| ENV-11-53-28-1 | PEG 35K / PEG 100K (70/30) | 300 x 300 x 6,000 | 2 | 432 | SCJ | ENV905-PRE-003 |
| ENV-11-53-28-2 | PEG 35K / PEG 100K (70/30) | 300 x 300 x 6,000 | 3 | 1,152 | SCJ | ENV905-PRE-003 |
| ENV-11-53-28-3 | PEG 1,000K (100) | 300 x 300 x 6,000 | 3 | 1,158 | SCJ | ENV905-PRE-003 |
| ENV-11-115-3 | PEG 35K / PEG 100K (70/30) | 225 x 225 x 4,000 | 2 | 0 | SCJ | ENV905-PRE-004 |
| ENV-11-115-4 | PEG 35K / PEG 100K (70/30) | 300 x 300 x 6,000 | 2 | 0 | SCJ | ENV905-PRE-004 |
| ENV-11-115-1 | PEG 35K / PEG 100K (70/30) | 225 x 225 x 4,000 | 2 | 202 | SCJ or IC | ENV905-PRE-004 |
| ENV-11-115-2 | PEG 35K / PEG 100K (70/30) | 300 x 300 x 6,000 | 1 or 3 | 413 or 1,239 | SCJ | ENV905-PRE-004 |
| ENV-11-115-8-10 | PEG 100K/ PEG 3,350 (89/11) | 225 x 225 x 2,925 | 1 | 0 | IC | ENV905-PRE-006 |
| ENV-11-115-23-1 | PEG 100K/ PEG 3,350 (89/11) | 300 x 300 x 6,000 | 1 | 0 | SCJ | ENV905-PRE-006 |
| ENV-11-115-8-3 | PEG 100K/ PEG 3,350 (89/11) | 225 x 225 x 4,000 | 2 | 204 | IC | ENV905-PRE-006 |
| ENV-11-115-8-5 | PEG 100K/ PEG 3,350 (89/11) | 225 x 225 x 2,925 | 1 | 74 | IC | ENV905-PRE-006 |
| ENV-11-115-8-7 | PEG 100K/ PEG 3,350 (89/11) | 225 x 225 x 2,925 | 1 | 40 | IC | ENV905-PRE-006 |
| ENV-11-115-16-1 | PEG 100K/ PEG 3,350 (89/11) | 300 x 300 x 6,000 | 1 | 390 | SCJ | ENV905-PRE-006 |
| ENV-11-115-37-1 | PEG 100K/ PEG 3,350 (89/11) | 225 x 225 x 2,925 | 1 | 0 | IC | ENV905-PRE-008 |
| ENV905-2I-178-20-4 | PEG 100K/ PEG 3,350 (89/11) | 225 x 225 x 2,925 | 1 | 10 | IC | ENV905-PRE-008 |
| ENV905-2I-178-20-1 | PEG 100K/ PEG 3,350 (89/11) | 225 x 225 x 2,925 | 1 | 20 | IC | ENV905-PRE-008 |
| ENV905-21-0178-25 | PEG 100K/ PEG 3,350 (89/11) | 225 x 225 x 2,925 | 1 | 44 | IC | ENV905-PRE-008 |

**Table 34: Polymer Matrix/Therapeutic Agent Blend Ratio For ENV905-PRE-006 and ENV905-PRE-008 Implants**

| Study No. | ID | Polymer Matrix Target, % | | Therapeutic Agent Target, % |
|---|---|---|---|---|
| | | PEG 100K | PEG 3350 | |
| ENV905-PRE-006 | ENV-11-115-8-10 | 89.0 | 11.0 | 0.0 |
| | ENV-11-115-23-1 | 89.0 | 11.0 | 0.0 |
| | ENV-11-115-8-3 | 44.5 | 5.5 | 50.0 |
| | ENV-11-115-8-5 | 44.5 | 5.5 | 50.0 |
| | ENV-11-115-8-7 | 66.75 | 8.25 | 25.0 |
| | ENV-11-115-16-1 | 44.5 | 5.5 | 50.0 |
| ENV905-PRE-008 | ENV-11-115-37-1 | 89.0 | 11.0 | 0 |
| | ENV905-2I-178-20-4 | 89.0 | 11.0 | 8 |
| | ENV905-21-178-20-1 | 89.0 | 11.0 | 14 |
| | ENV905-21-0178-25 | 89.0 | 11.0 | 26.4 |

**Table 35: Implants Administered in ENV905-PRE-006 and ENV905-PRE-008**

| Study No. | ID | No. Implants Dosed/Eye | No. Eyes Dosed | Therapeutic Agent Content/ Implant (µg) | Therapeutic Agent Dose/ Eye (µg) | Therapeutic Agent Dose/ Animal (µg) |
|---|---|---|---|---|---|---|
| ENV905-PRE-006 | Positive Control | 0 | 8 | 0 | 0 | 0 |
| | ENV-11-115-8-10 | 1 | 4 | 0 | 0 | 0 |
| | ENV-11-115-23-1 | 1 | 4 | 0 | 0 | 0 |
| | ENV-11-115-8-3 | 2 | 8 | 102.2 | 204.4 | 408.8 |
| | ENV-11-115-8-5 | 1 | 8 | 74.3 | 74,3 | 148.6 |
| | ENV-11-115-8-7 | 1 | 8 | 40.2 | 40.2 | 80.4 |
| | ENV-11-115-16-1 | 1 | 8 | 390.3 | 390.3 | 780.6 |
| ENV905-PRE-008 | ENV-11-115-37-1 | 1 | 8 | 0 | 0 | 0 |
| | ENV905-2I-178-20-4 | 1 | 8 | 10 | 10 | 20 |
| | ENV905-2I-178-20-1 | 1 | 8 | 20 | 20 | 40 |
| | ENV905-21-0178-25 | 1 | 8 | 44 | 44 | 88 |

### Nonclinical Pharmacology of ENV905 - Results

Dose administration via the SCJ or IC route was conducted successfully, and there were no adverse findings related to the dosing procedure noted in any study. Ocular inflammation was successfully induced via corneal incision or phacoemulsification, as observed by ophthalmic exam, and clinical findings related to the surgical procedure included conjunctival hyperemia and chemosis, iritis, and aqueous and cellular flare; these findings generally decreased in severity by Day 7. Anterior chamber paracentesis was conducted at the predetermined time points, and induced findings similar in nature, but diminished in severity, compared with those observed following the surgical procedures. Positive control DUREZOL^{®} dampened the inflammatory response following surgery or paracentesis as expected.

### SCJ ENV905 Results

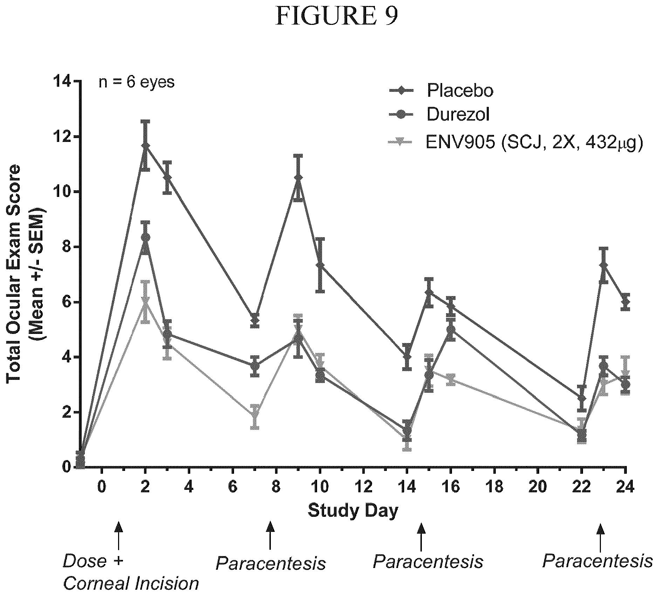

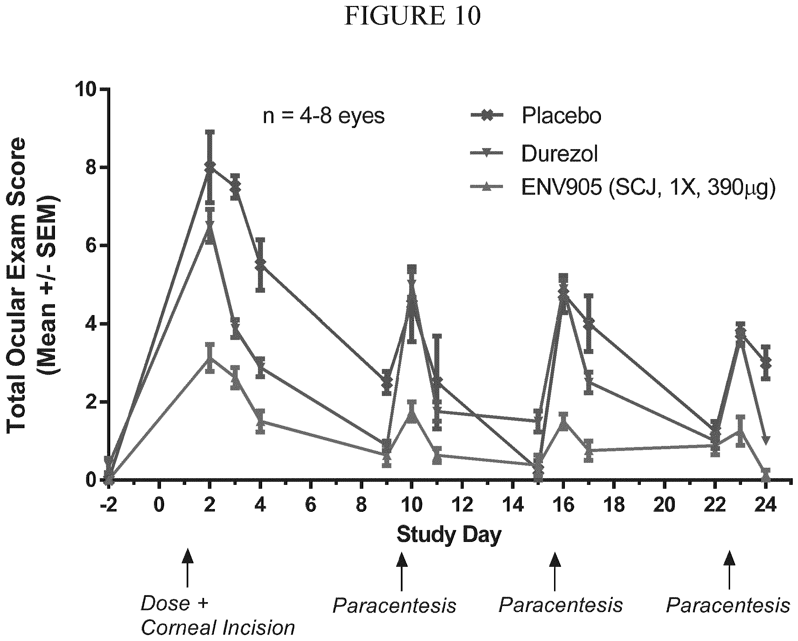

SCJ ENV905, dosed at approximately 400 µg of difluprednate per eye, reduced inflammation following corneal incision at a level similar or superior to that observed with QID DUREZOL^{®} (~100 µg difluprednate per day, assumes 50 µL/drop) **(****FIG. 9** and **FIG. 10****).**

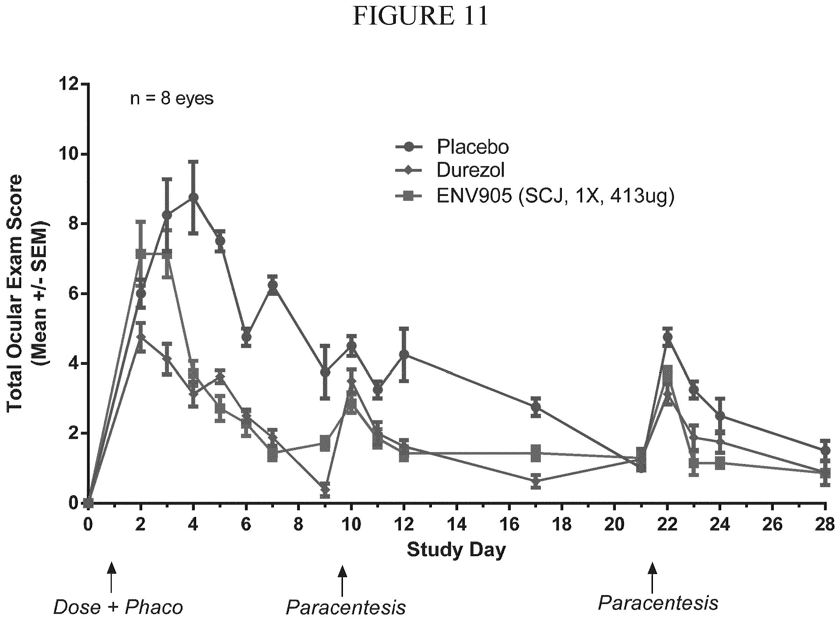

Following the more invasive lens phacoemulsification surgery, SCJ ENV905 at ~400 µg difluprednate per eye did not reduce inflammation at 24 and 48 hours post-surgery when compared with QID DUREZOL^{®}, but was equivalent in efficacy to DUREZOL^{®} at all other time points **(****FIG. 11****).**

Note that DUREZOL^{®} dosing was initiated at the time of surgery in all studies, contrary to the package label instructions which recommend beginning DUREZOL^{®} administration 24 hours post-surgery. Additionally, sufficient difluprednate following subconjunctival administration was present following a single administration on Day 1 to enable reduction of inflammation via paracentesis at each subsequent induction time point through Day 24/28 (FIGS. **9-11****).**

### IC ENV905 Results

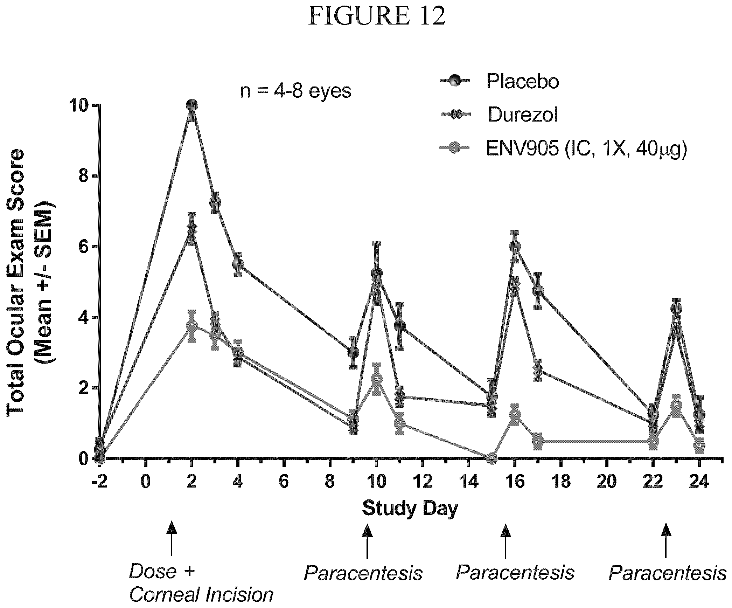

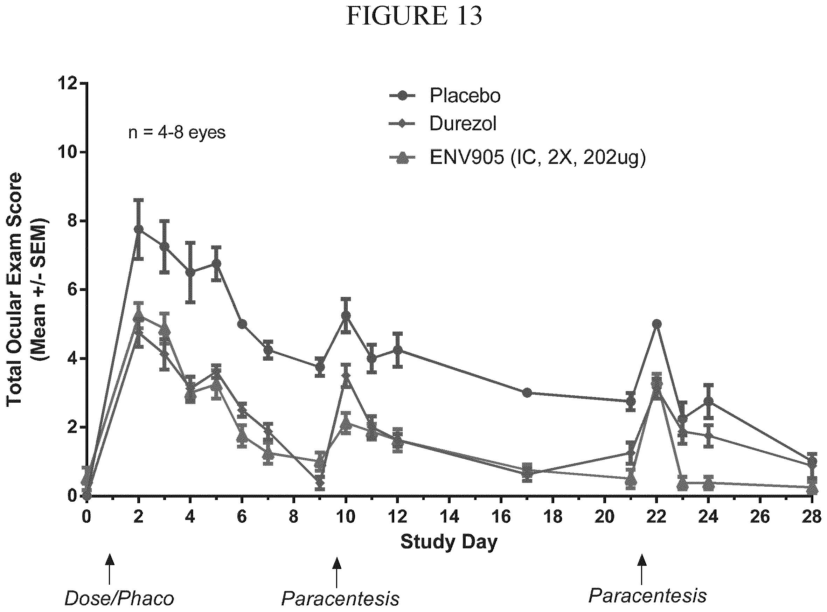

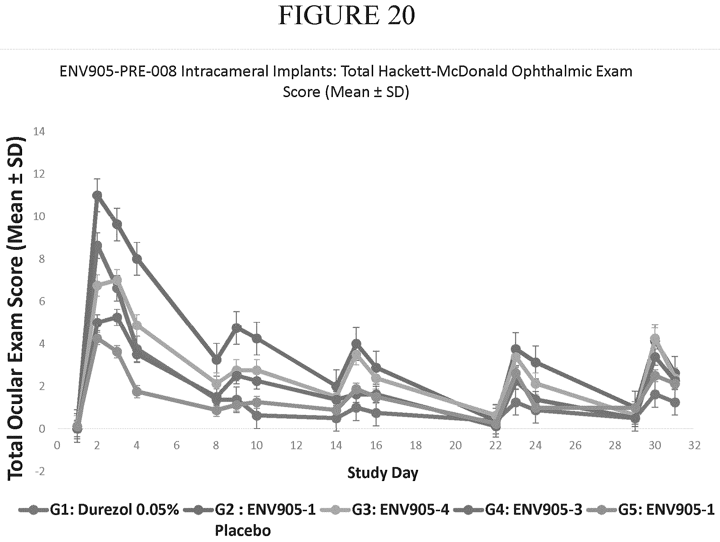

IC ENV905, dosed as a single administration of either 10, 20, 40 or 44 µg of difluprednate via one implant per eye, decreased the level of induced inflammation following corneal incision in a manner which was similar or superior to that observed with QID DUREZOL^{®} (~100 µg difluprednate per day, assumes 50 µL/drop) at most time points throughout the 24-day or 31-day study period **(****FIG. 12** and **FIG. 20****).** IC ENV905, dosed as a single administration of ~200 µg of difluprednate via two implants per eye, was equivalent to QID DUREZOL^{®} at the early time points following phacoemulsification surgery, and was generally equivalent or superior to DUREZOL^{®} at the later time points **(****FIG. 13****).**

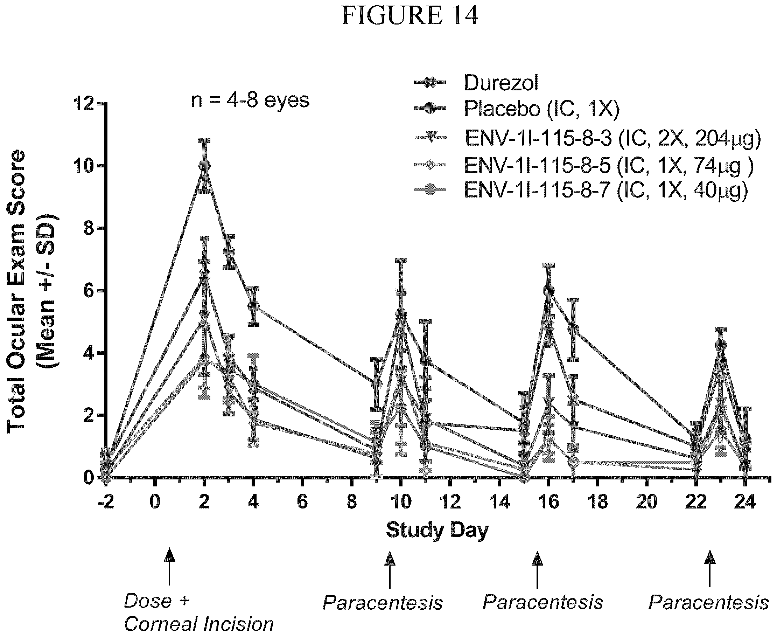

**FIG. 14** illustrates the Total Hackett-McDonald Ophthalmic Exam Score (Mean ± SD) for all ENV905 intracameral implants tested in the ENV905-PRE-006 study.

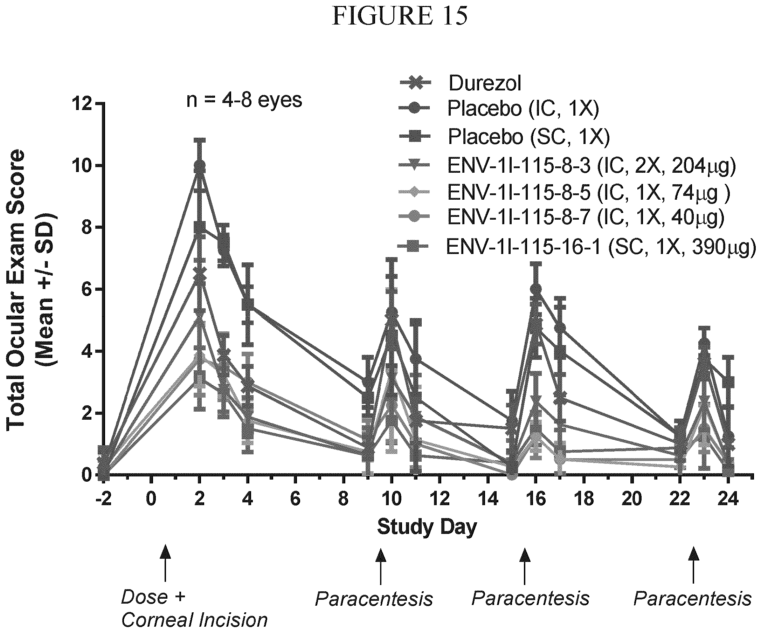

**FIG. 15** illustrates the Total Hackett-McDonald Ophthalmic Exam Score (Mean ± SD) for all ENV905 intracameral and subconjunctival implants tested in the ENV905-PRE-006 study.

**FIG. 20** illustrates the Total Hackett-McDonald Ophthalmic Exam Score (Mean ± SEM) for all ENV905 intracameral implants tested in the ENV905-PRE-008 study.

### ENV905 In Vivo Animal Model Test Conclusions

Data from the aforementioned non-GLP pharmacology studies of various formulations of ENV905 indicate that, whether administered via the SCJ or IC routes, ENV905 decreases ocular inflammation following ocular surgery in the rabbit, at dose levels ranging from 40 to 400 µg of difluprednate per eye. A similar effect is expected in humans.

No adverse effects were noted in any study, and the implants were well tolerated in this species.

A significant dose sparing effect was observed with both SCJ and IC ENV905, with excellent anti-inflammatory effects observed at doses of difluprednate that were significantly less than the dose of difluprednate via topical DUREZOL^{®}.

### Pharmacokinetics of ENV905

Pharmacokinetics (often abbreviated PK) is the pharmacological study of how an administered drug is absorbed, distributed, metabolized, and eliminated by the body. *See,* Rowland and Tozer, "Clinical Pharmacokinetics and Pharmacodynamics: Concepts and Applications," 4th Ed., 2011, Lippincott Williams & Wilkins publishers, which is incorporated herein by reference in its entirety for all purposes.

Systemic and ocular PK was assessed as part of the ENV905 pharmacology studies discussed above and two ocular pharmacokinetic studies.

Matrixes included aqueous humor, cornea, trabecular meshwork, iris/ciliary body, bulbar conjunctiva, subconjunctival dose site (SCJ only), retina, and plasma. Ocular matrixes were analyzed for both the parent molecule, difluprednate (DFBA), as well as the active metabolite, desacetyl difluprednate (DFB). Plasma was analyzed for DFB only, as the conversion to the active metabolite occurs within the ocular tissue prior to systemic absorption. Systemic exposure to DFB was minimal and was generally below the limit of quantitation by Day 7. Concentrations of DFBA and DFB in ocular tissue were dose dependent as well as dependent on the route of administration.

### Bioanalytical Methods

Bioanalytical methods for difluprednate (DFBA), the active metabolite (DFB), and their internal standard DFB-d₆ were developed and qualified in albino rabbit matrixes by Intertek Laboratories (San Diego, CA) using liquid-liquid extraction and high performance liquid chromatography and tandem mass spectrometry (LC-MS/MS). Methods in rabbit aqueous humor, cornea, trabecular meshwork, iris/ciliary body, bulbar conjunctiva, subconjunctival dose site (SCJ only), retina, and plasma (DFB only) were qualified for range of reliable response, selectivity, carryover assessment, and precision and accuracy.

**Table 36: ENV905 Formulations Tested for Pharmacokinetics in Tolerability, Pharmacology, and Pharmacokinetics Studies in New Zealand White Rabbits.**

| **ENV905 Formulation ID** | **PEG Ratio** | **Implant Dimensions (µm)** | **Number of Implants/ Eye** | **Total Dose (µg difluprednate/ Eye)** | **Route of Administration** | **Study Number** | **Matrices Analyzed** |
|---|---|---|---|---|---|---|---|
| ENV-11-115-3 | PEG 35K / PEG 100K (70/30) | 225 x 225 x 4,000 | 2 | 0 | SCJ | ENV905-PRE-004 | Aqueous Humor, Conjunctiva |
| ENV-11-115-4 | PEG 35K / PEG 100K (70/30) | 300 x 300 x 6,000 | 2 | 0 | SCJ | ENV905-PRE-004 | Aqueous Humor, Conjunctiva |
| ENV-11-115-1 | PEG 35K / PEG 100K (70/30) | 225 x 225 x 4,000 | 2 | 202 | SCJ or IC | ENV905-PRE-004 | Aqueous Humor, Conjunctiva |
| ENV-11-115-2 | PEG 35K / PEG 100K (70/30) | 300 x 300 x 6,000 | 1 or 3 | 413 or 1,239 | SCJ | ENV905-PRE-004 | Aqueous Humor, Conjunctiva |
| ENV-11-115-4 (Placebo) | PEG 35K / PEG 100K (70/30) | 300 x 300 x 6,000 | 2 | 0 | SCJ | ENV905-PRE-005 | Plasma |
| ENV-11-53-28-1¹ | PEG 35K / PEG100K (70/30) | 300 x 300 x 6,000 | 2 | 432 | SCJ | ENV905-PRE-005 | Plasma |
| ENV-11-115-2 | PEG 35K / PEG100K (70/30) | 300 x 300 x 6,000 | 2 | 826 | SCJ | ENV905-PRE-005 | Plasma |
| ENV-11-115-8-10 | PEG 100K/ PEG 3,350 (89/11) | 225 x 225 x 2,925 | 1 | 0 | IC | ENV905-PRE-006 | Aqueous Humor |
| ENV-11-115-23-1 | PEG 100K/ PEG 3,350 (89/11) | 300 x 300 x 6,000 | 1 | 0 | SCJ | ENV905-PRE-006 | Aqueous Humor |
| ENV-11-115-8-3 | PEG 100K/ PEG 3,350 (89/11) | 225 x 225 x 4,000 | 2 | 204 | IC | ENV905-PRE-006 | Aqueous Humor |
| ENV-11-115-8-5 | PEG 100K/ PEG 3,350 (89/11) | 225 x 225 x 2,925 | 1 | 74 | IC | ENV905-PRE-006 | Aqueous Humor |
| ENV-11-115-8-7 | PEG 100K/ PEG 3,350 (89/11) | 225 x 225 x 2,925 | 1 | 40 | IC | ENV905-PRE-006 | Aqueous Humor |
| ENV-11-115-16-1 | PEG 100K/ PEG 3,350 (89/11) | 300 x 300 x 6,000 | 1 | 390 | SCJ | ENV905-PRE-006 | Aqueous Humor |
| ENV905-1 | PEG 100K/ PEG 3,350 (89/11) | 225 x 225 x 2,925 | 3 | 121.5 | IC | ENV905-ADME-001 | Plasma Aqueous Humor, Cornea/TM, Iris/Ciliary Body, Conjunctiva, Retina |
| ENV905-2 | PEG 100K/ PEG 3,350 (89/11) | 300 x 300 x 6,000 | 3 | 1207.5 | SCJ | ENV905-ADME-001 | Plasma Aqueous Humor, Cornea/TM, Iris/Ciliary Body, Conjunctiva, Retina |
| ENV905-1 | PEG 100K/ PEG 3,350 (89/11) | 225 x 225 x 2,925 | 1, 2, or 3 | 42.8, 85.6, or 128.4 | IC | ENV905-ADME-002 | Plasma Aqueous Humor, Cornea, Trabecular Meshwork, Iris/Ciliary Body, Conjunctiva, Retina |
| ENV905-1 | PEG 100K/ PEG 3,350 (89/11) | 225 x 225 x 2,925 | 1, 2, or 3 | 44, 88, or 132 | IC | ENV905-TOX-001 | Plasma |

### Plasma Concentration Data from Tolerability Study ENV905-PRE-005

Animals received a single, bilateral administration of SCJ ENV905 (432 or 826 µg difluprednate per eye) or placebo implant.

There was no detectable DFB in plasma from placebo animals (LLOQ 0.05 ng/mL).

Following subconjunctival administration of ENV905, DFB in plasma peaked on Day 2 (24 hours post-dose), with concentrations of 0.68 and 0.72 ng/mL for 432 and 826 µg difluprednate per eye (864 and 1,652 µg difluprednate per animal), respectively. *See,* **Table 37.**

Systemic exposure to DFB decreased rapidly through Days 4 and 7, and was undetectable at later time points. In comparison to a previously published study, DFB Cₘₐₓ in plasma following topical DUREZOL^{®} administration was approximately 10 ng/mL at 30 minutes post-dose (Tajika 2011a).

These data demonstrate decreased systemic exposure to DFB following ENV905 administration when compared with QID DUREZOL^{®}.

**Table 37: Concentration of DFB in Rabbit Plasma Following Subconjunctival ENV905**

| **Study Number** | **Formulation (µg DFBA)** | **Study Day** | **Parameter** | **Concentration of DFB (ng/mL)** |
|---|---|---|---|---|
| ENV905-PRE-005 | ENV1I-53-28-1 (432) | 2 | Mean | 0.678 |
| | | | SD | 0.238 |
| | | 4 | Mean | 0.116 |
| | | | SD | 0.0278 |
| | | 7 | Mean | BLQ |
| | | | SD | NA |
| | | 14 | Mean | BLQ |
| | | | SD | NA |
| | | 29 | Mean | BLQ |
| | | | SD | NA |
| ENV905-PRE-005 | ENV1I-115-2 (826) | 2 | Mean | 0.720 |
| | | | SD | 0.232 |
| | | 4 | Mean | 0.206 |
| | | | SD | 0.0872 |
| | | 7 | Mean | 0.0415 |
| | | | SD | 0.0542 |
| | | 14 | Mean | BLQ |
| | | | SD | NA |
| | | 29 | Mean | BLQ |
| | | | SD | NA |

### Aqueous Humor Concentration Data from Pharmacology Studies

Aqueous humor (AH) was obtained in the pharmacology studies described above, as part of the inflammation induction method of anterior chamber paracentesis.

An approximately 100 µL sample was obtained by the veterinary ophthalmologist using a 30 G needle inserted at the limbus. Samples were analyzed for DFBA and DFB in studies ENV905-PRE-004 and ENV905-PRE-006. Sampling days varied slightly across studies due to ophthalmologist availability.

In ENV905-PRE-004, animals received a single bilateral administration of ENV905, and AH was sampled on Days 9, 21, and 28. SCJ ENV905 doses were 413 or 1,239 µg of difluprednate per eye, and the IC dose was 202 µg difluprednate per eye. There was no detectable DFBA or DFB in AH obtained from placebo animals. Following a dose of 413 µg of difluprednate per eye, DFB was detectable on Day 9 and below the lower limit of quantification (LLOQ) at later time points. Following the 1,239 µg difluprednate per eye dose, DFB was detectable at all time points through Day 28. *See,* **Tables 38-39.**

In ENV905-PRE-006, animals received a single bilateral administration of ENV905, and AH was sampled on Days 9, 15, and 24. The SCJ dose was 390 µg difluprednate per eye, and IC doses were 40 and 74 µg difluprednate per eye. There was no detectable DFBA or DFB in AH obtained from placebo animals. SCJ ENV905 demonstrated no detectable DFB on Day 9, but was detectable at low concentrations on Days 15 and 24. IC ENV905 had very few samples with detectable DFB concentrations, potentially due to sampling only on Day 9 or later, while robust efficacy was seen in all animals throughout following this route of dose administration. *See,* **Tables 38-39.**

**Table 38: Concentration of DFBA and DFB in Rabbit Aqueous Humor Following Subconjunctival ENV905**

| **Study Number** | **Formulation (µg DFBA)** | **Study Day** | **Parameter** | **Concentratio n of DFBA (ng/mL)** | **Concentration of DFB (ng/mL)** |
|---|---|---|---|---|---|
| ENV905-PRE-004 | ENV-1I-115-1 (413) | 9 | Mean | BLQ | 0.039 |
| | | | SD | NA | 0.088 |
| | | 21 | Mean | BLQ | BLQ |
| | | | SD | NA | NA |
| | | 28 | Mean | BLQ | BLQ |
| | | | SD | NA | NA |
| ENV905-PRE-004 | ENV-1I-115-1 (1,239) | 9 | Mean | BLQ | 0.138 |
| | | | SD | NA | 0.171 |
| | | 21 | Mean | BLQ | 0.056 |
| | | | SD | NA | 0.112 |
| | | 28 | Mean | BLQ | 0.0436 |
| | | | SD | NA | 0.0825 |
| ENV905-PRE-006 | ENV-1I-115-16-1 (390) | 9 | Mean | BLQ | BLQ |
| | | | SD | NA | NA |
| | | 15 | Mean | BLQ | 0.0368 |
| | | | SD | NA | 0.0735 |
| | | 24 | Mean | BLQ | 0.0206 |
| | | | SD | NA | 0.0412 |

**Table 39: Concentration of DFBA and DFB in Rabbit Aqueous Humor Following Intracameral ENV905**

| **Study Number** | **Formulation (µg DFBA)** | **Study Day** | **Parameter** | **Concentration of DFBA (ng/mL)** | **Concentration of DFB (ng/mL)** |
|---|---|---|---|---|---|
| ENV905-PRE-004 | ENV-1I-115-1 (202) | 9 | Mean | BLQ | BLQ |
| | | | SD | NA | NA |
| | | 21 | Mean | BLQ | BLQ |
| | | | SD | NA | NA |
| | | 28 | Mean | BLQ | BLQ |
| | | | SD | NA | NA |
| ENV905-PRE-006 | ENV-1I-115-8-7 (40) | 9 | Mean | BLQ | BLQ |
| | | | SD | NA | NA |
| | | 15 | Mean | BLQ | 0.0278 |
| | | | SD | NA | 0.0555 |
| | | 24 | Mean | BLQ | BLQ |
| | | | SD | NA | NA |
| ENV905-PRE-006 | ENV-1I-115-8-5 (74) | 9 | Mean | BLQ | BLQ |
| | | | SD | NA | NA |
| | | 15 | Mean | BLQ | 0.0278 |
| | | | SD | NA | 0.0555 |
| | | 24 | Mean | BLQ | 0.0143 |
| | | | SD | NA | 0.0287 |

### Conjunctiva Concentration Data from Pharmacology Study ENV905-PRE-004

Conjunctiva samples were taken at study termination in ENV905-PRE-004 to gain an understanding of how much DFBA and DFB remained at 28 days post-dose.

The formulations assessed in this study are not fully representative of the clinical candidate formulations that are planned for continued development, as they differ in excipient content. There was no detectable DFBA or DFB in the placebo samples. Detectable DFBA and DFB was present in 2 out of 8 samples at 28 days following the 413 µg difluprednate implant, and 4 out of 8 samples at 28 days following 1,239 µg difluprednate implant. Analysis of conjunctiva for concentration of DFBA and DFB following dosing of the clinical candidate formulations of ENV905 has not been conducted. *See,* **Table 40.**

**Table 40: Concentration of DFBA and DFB in Rabbit Superior Conjunctiva Following Subconjunctival ENV905**

| **Study Number** | **Formulation (µg DFBA)** | **Study Day** | **Parameter** | **Concentration of DFBA (ng/g)** | **Concentration of DFB (ng/g)** |
|---|---|---|---|---|---|
| ENV905-PRE-004 | ENV-1I-115-1 (413) | 28 | Mean | 292.6 | 27380¹ |
| | | | SD | 827.3 | 77426 |
| ENV905-PRE-004 | ENV-1I-115-1 (1,239) | 28 | Mean | 26900 | 368878.5² |
| | | | SD | 56997 | 569916.1 |

| | | | | | |
|---|---|---|---|---|---|
| 1. 2 out of 8 samples were quantifiable, all other samples were BLQ 2. 4 out of 8 samples were quantifiable, all other samples were BLQ | | | | | |

### Ocular and Systemic Concentration Data from Pharmacokinetic Study ENV905-ADME-001

Plasma samples were collected from all surviving animals on Days 1 (2h), 2 (24h), 4, 7, 14, 21, 28, 42, 56 and 84. Ocular tissues from two animals per group per time point were collected on Days 14, 28, 42, 56, and 84. Ocular matrixes collected included: aqueous humor, cornea (enriched with trabecular meshwork), iris/ciliary body, bulbar conjunctiva, subconjunctival dose site (SCJ only), and retina.

The formulations assessed in this study are representative of the clinical candidate formulations that are planned for continued development.

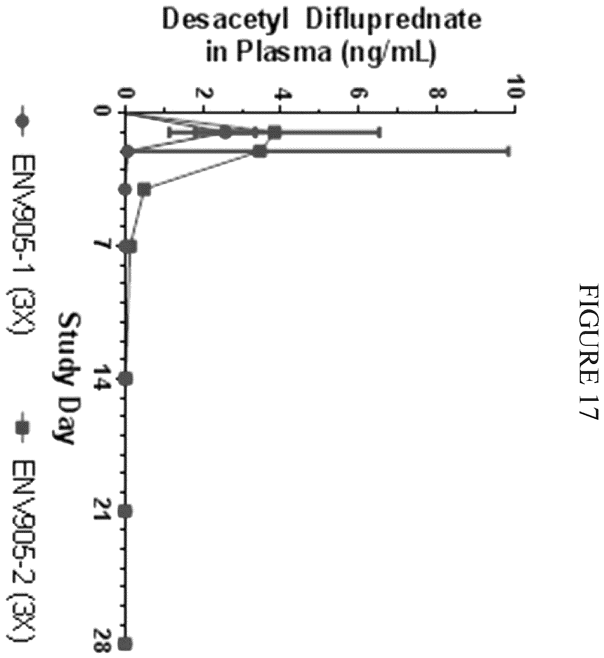

Plasma exposure to Desacetyl Difluprednate (DFB) following intracameral administration of ENV905-1 was noted at minimal concentrations on Days 1 and 2, and was not quantifiable by Day *4. See,* **FIG. 17** and **Table 41.**

Following SCJ delivery of ENV905-2, plasma exposure to Desacetyl Difluprednate (DFB) was also minimal and was quantifiable at very low levels through Day 14. *See,* **FIG. 17** and **Table 41.**

**Table 41: Concentration of DFB in Rabbit Plasma Following Intracameral or Subconjunctival ENV905**

| **Study Number** | **Formulation (µg DFBA/ Implant)** | **No. of Implants / Eye** | **Study Day** | **Parameter** | **Concentration of DFB (ng/mL)** |
|---|---|---|---|---|---|
| ENV905-ADME-001 | ENV905-1 (40.5) IC Delivery | 3 | 1 (2h) | Mean | 2.583 |
| | | | | SD | 0.779 |
| | | | 2 (24h) | Mean | 0.062 |
| | | | | SD | 0.049 |
| ENV905-ADME-001 | ENV905-2 (402.5) | 3 | 1 (2h) | Mean | 3.850 |
| | | | | SD | 2.703 |
| | | | 2 (24h) | Mean | 3.475 |
| | SCJ Delivery | | | SD | 6.387 |
| | | | 4 | Mean | 0.493 |
| | | | | SD | 0.09 |
| | | | 7 | Mean | 0.134 |
| | | | | SD | 0.044 |
| | | | 14 | Mean | 0.017 |
| | | | | SD | 0.028 |

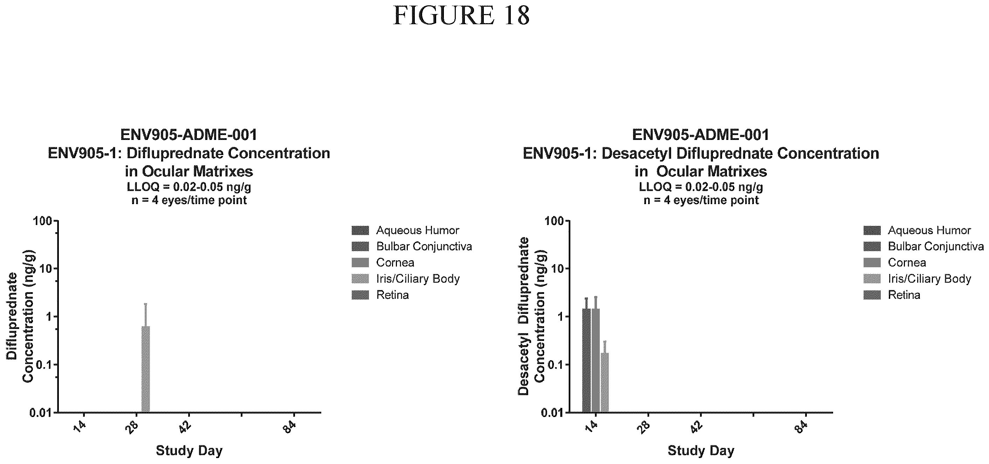

Following IC delivery of ENV905-1, there was no quantifiable exposure to Difluprednate (DFBA) except in 1 iris/ciliary body sample on Day 28. All other samples were below the limit of quantitation (BLQ). Exposure to Desacetyl Difluprednate (DFB) was minimal on Day 14 in bulbar conjunctiva, TM-enriched cornea, and iris/ciliary body, and was not quantifiable at Day 28. *See,* **FIG. 18****.**

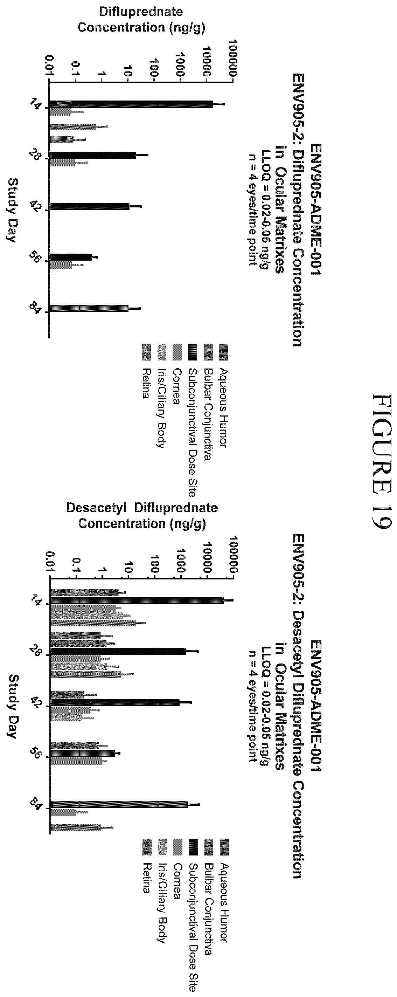

Following SCJ delivery of ENV905-2, Difluprednate (DFBA) was quantifiable at the dose site and cornea through Day 56, and was noted in the aqueous humor and retina at early time points. Desacetyl Difluprednate (DFB) was observed at the dose site and cornea through Day 84, in the bulbar conjunctiva through Day 56, in the iris/ciliary body through Day 42, in the retina through Day 28, and in the aqueous humor at early time points. *See,* **FIG. 19****.** Desacetyl Difluprednate (DFB) was also observed in the retina through Day 28 and again in Day 84, but not at Days 42 and 56. *See,* **FIG. 19****.**

### Ocular and Systemic Pharmacokinetics from Pharmacokinetic Study ENV905-ADME-002

New Zealand White male rabbits (two per group per terminal time point) were given a single bilateral administration of one, two, or three ENV905 (difluprednate) Ophthalmic Implants (ENV905-1) via intracameral delivery. For ENV905-1, the total dose delivered was 42.8, 85.6, or 128.4 µg difluprednate per eye and 85.6, 171.2, or 256.8 µg difluprednate per animal, for the one, two, or three implant groups, respectively. A separate group of animals were administered topical DUREZOL^{®} according to the clinical standard of care paradigm for comparison purposes. DUREZOL^{®} dose administration was conducted as follows: one drop per eye four times per day on Days 1-14, one drop per eye twice daily on Days 15-21, and one drop per eye once daily on Days 22-28, resulting in a total dose of up to 1925 µg difluprednate per eye and 3850 µg difluprednate per animal over the course of the study.

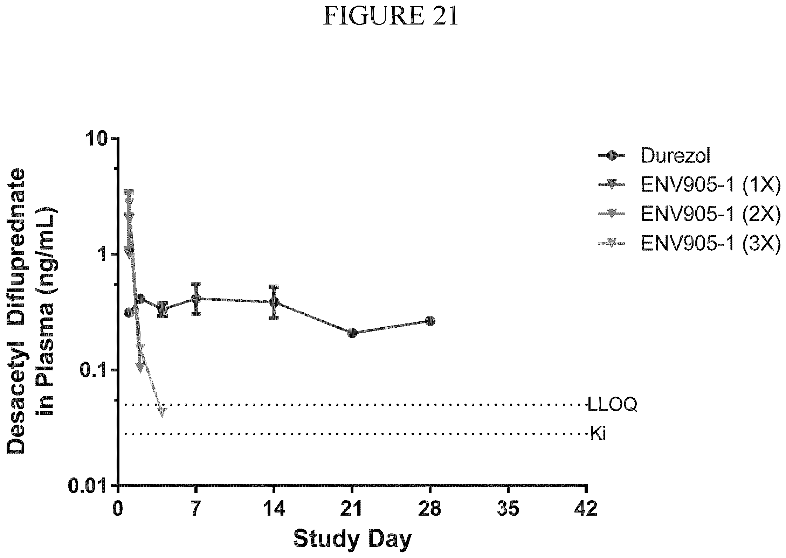

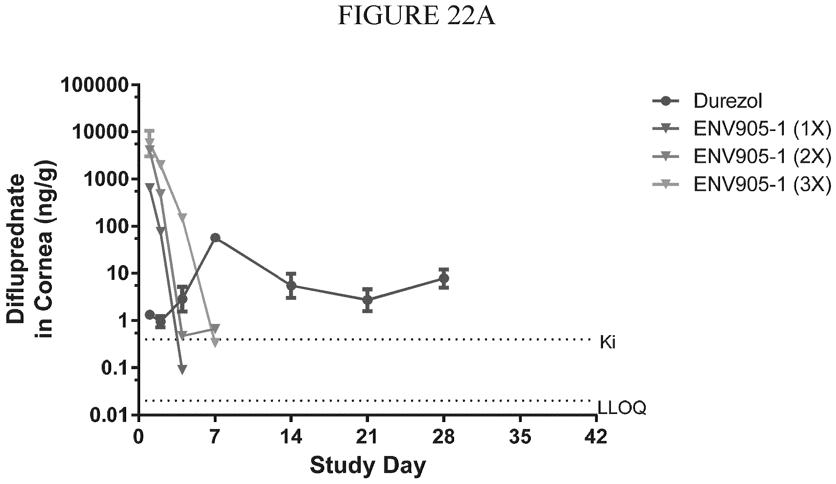

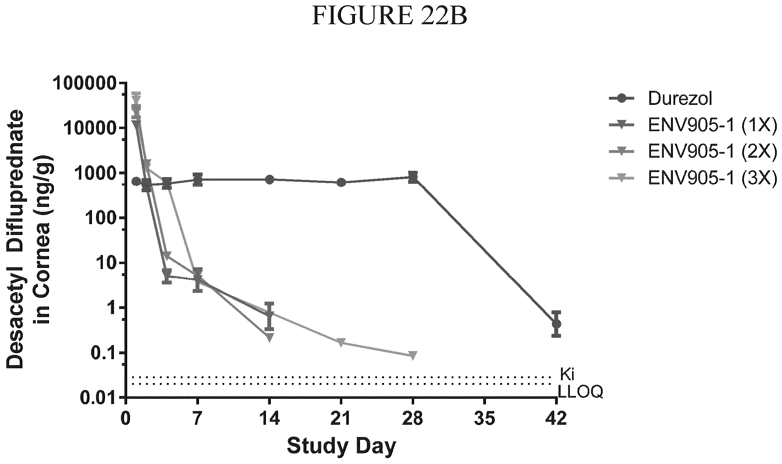

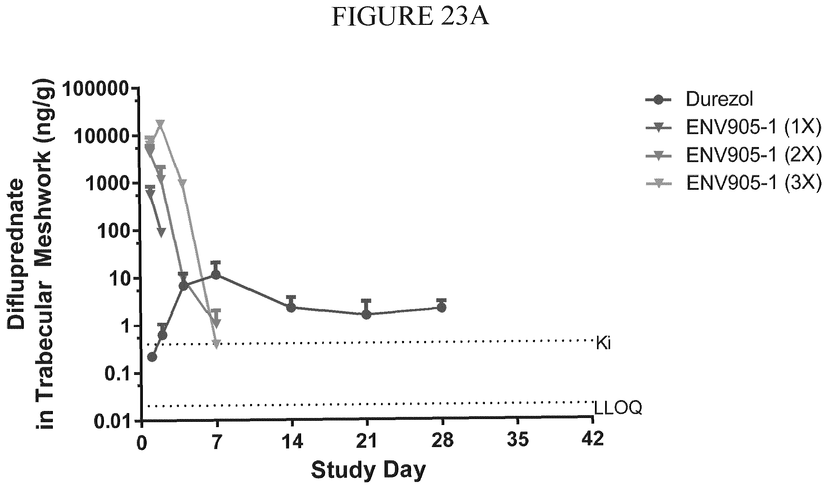

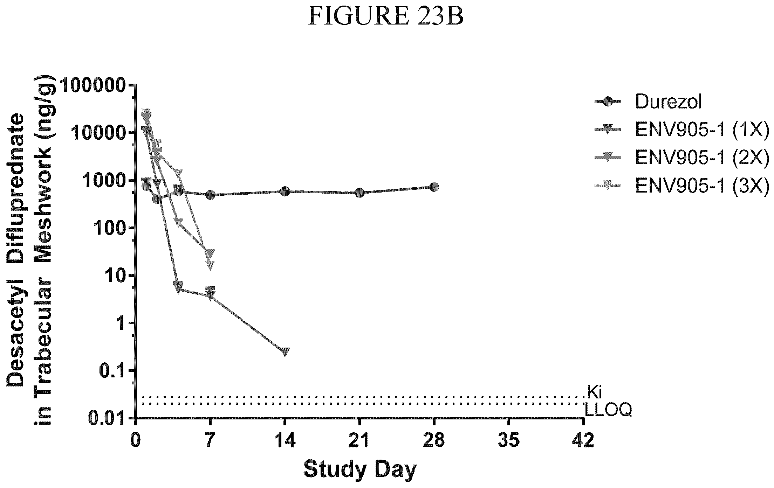

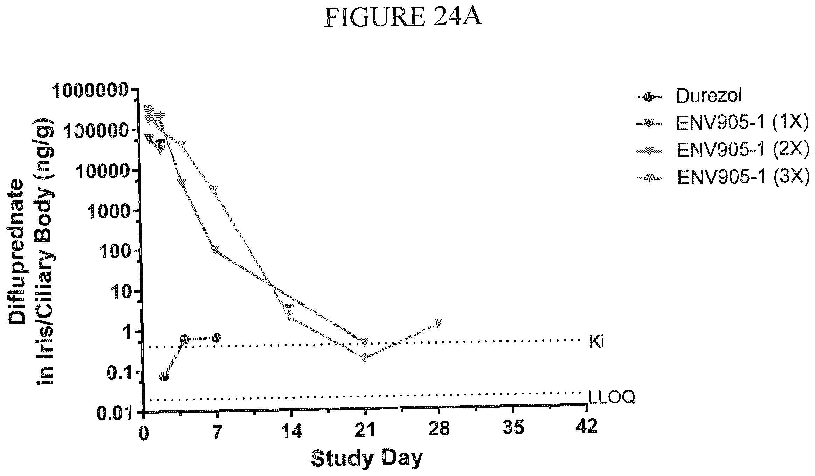

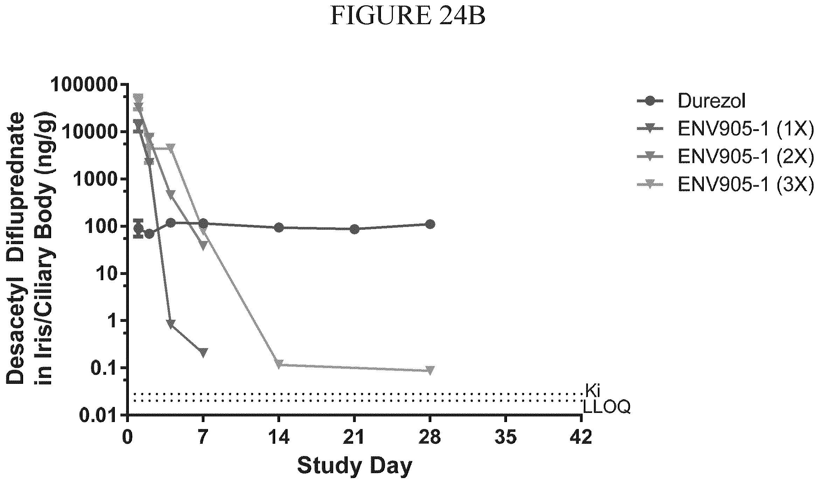

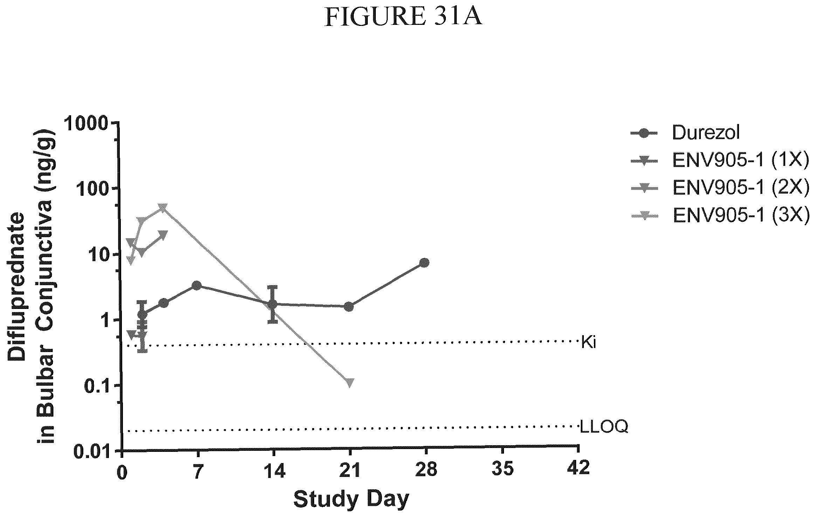

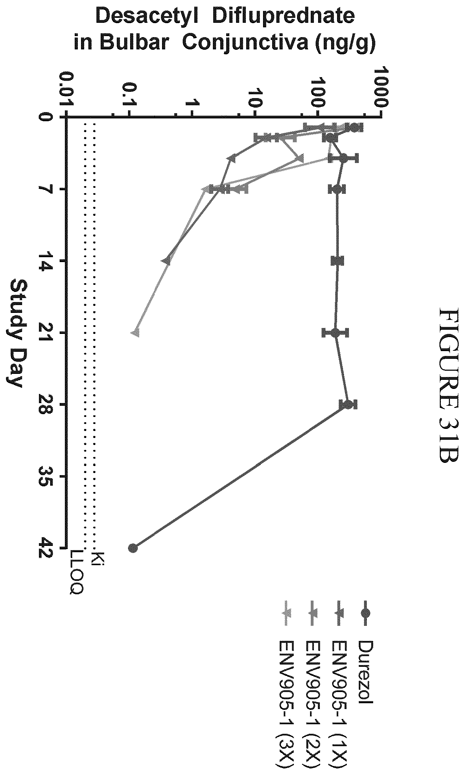

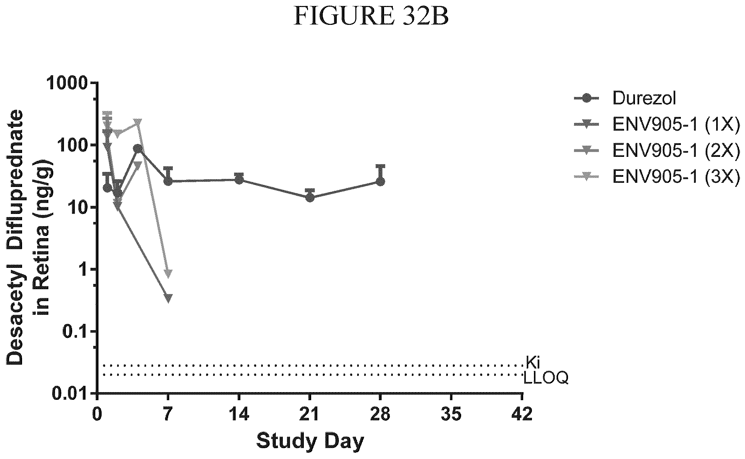

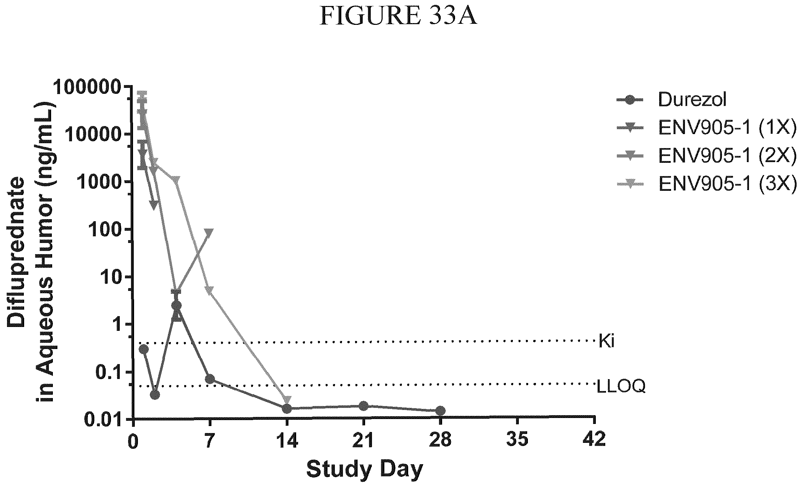

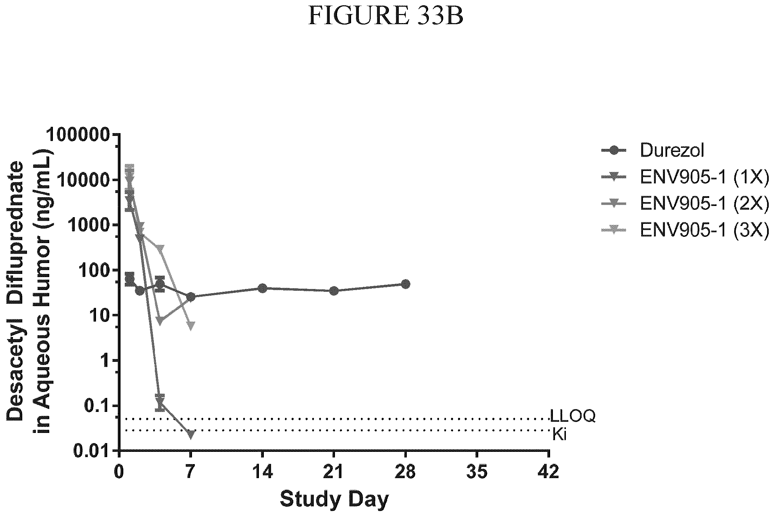

Plasma and ocular matrixes (aqueous humor, bulbar conjunctiva, cornea, trabecular meshwork, iris/ciliary body, and retina) were collected on Days 1 (2 hours post dose), 2 (24 hours post dose), 4, 7, 14, 21, 28, and 42, and analyzed for exposure to Difluprednate (DFBA) and the active metabolite Diacetyl Difluprednate (DFB). Samples for animals in the DUREZOL^{®} group were obtained on the same days, but were always collected at 30 minutes following the last dose administration of the day, so as to capture the peak exposure following topical administration. Data from these studies are provided in **Table 42,** and in **FIG. 21-24****,** and **31-33.** **FIG. 21** shows the levels of DFB in the plasma (ng/mL); **FIG. 22A** and **B** compares the levels of Difluprednate to DFB in the cornea (ng/mL), respectively; **FIG. 23A** and **B** compares the levels of Difluprednate to DFB in the trabecular meshwork (ng/mL), respectively; **FIG. 24A** and **B** compares the levels of Difluprednate to DFB in the iris/ciliary body (ng/mL), respectively; **FIG. 31A** and **B** compares the levels of Difluprednate to DFB in the bulbar conjunctiva (ng/mL), respectively; **FIG. 32A** and **B** compares the levels of Difluprednate to DFB in the retina (ng/mL), respectively; and **FIG. 33A** and **B** compares the levels of Difluprednate to DFB in the aqueous humordif (ng/mL), respectively.

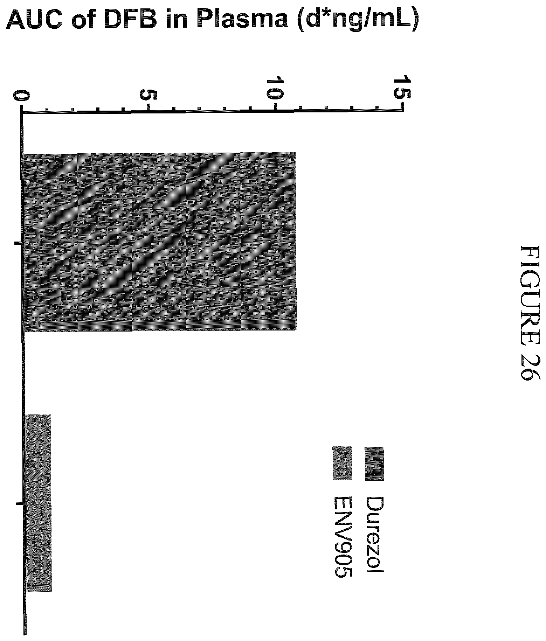

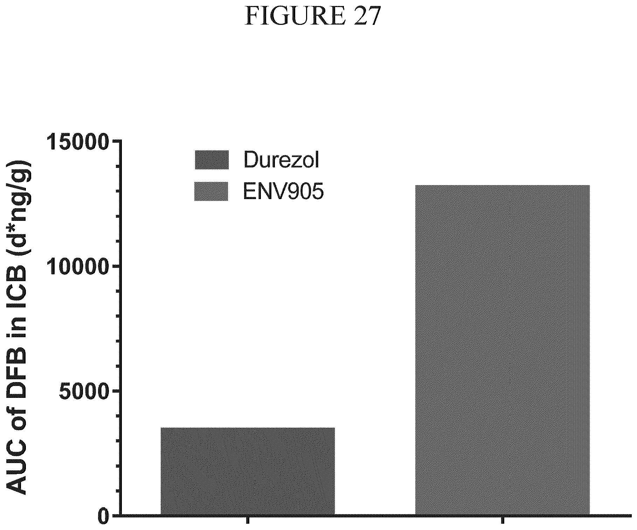

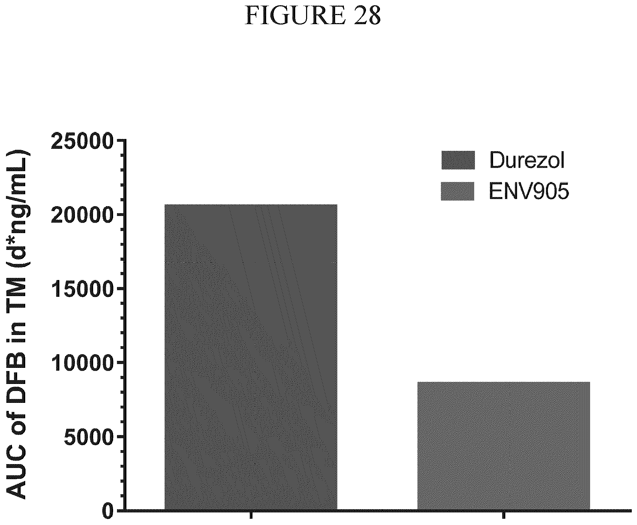

Topical DUREZOL^{®} and a single bilateral administration of one, two, or three ENV905-1 implants per eye were well tolerated in the male NZW rabbit in this study for 42 days. Exposure to parent DFBA and metabolite DFB following topical DUREZOL^{®} administration according to the clinical standard of care (1925 µg/eye) was consistent in plasma (assessed for DFB only) and in ocular matrixes across the dosing period when measured at 30 minutes post-dose, with the greatest exposure to DFB being in the cornea and trabecular meshwork (AUCₗₐₛₜ 19996 and 15530 d*ng/g, respectively). Exposure to the active metabolite DFB following administration of one ENV905-1 implant per eye (42.8 µg/eye) was highest in iris/ciliary body (AUCₗₐₛₜ 13174 d*ng/g; *See* **FIG. 24****),** to a lesser extent in cornea and trabecular meshwork (AUCₗₐₛₜ 9329 and 8571 d*ng/g, respectively; *See* **FIG. 22** and **23****,** respectively), with the least exposure in the bulbar conjunctiva and retina (AUCₗₐₛₜ 135 and 92.1 d*ng/g, respectively; *See* **FIG. 31** and **32****).** and this distribution paradigm was generally mirrored in the two and three implants per eye groups as well, with an increase in exposure observed as the dose increased. ENV905-1 Cₘₐₓ was on Day 1, and samples were generally not quantifiable after the first 7-14 days of the study. *See* **FIG. 21****.** Generally, the shape of the exposure over time curve was shifted left for ENV905 when compared with DUREZOL^{®}, indicating increased exposure at earlier time points. *See,* e.g., **FIG. 21****.** A direct comparison of AUC (d*ng/mL) for ENV905-1 (low) and DUREZOL^{®} observed in plasma is shown in **Figure 26****,** in iris/ciliary body is show in **Figure 27****,** and in trabecular meshwork is shown in **Figure 28****.** These data demonstrate that following ENV905-1 administration, absorption and distribution within the ocular matrixes is targeted to the anterior segment at physiologically relevant concentrations, and the concentration of difluprednate is greatest in the tissues most relevant to the therapeutic indication. Additionally, the duration of difluprednate exposure in ocular matrixes is generally limited to the most relevant time points for inflammation suppression for this indication and is particularly limited in the tissues linked to corticosteroid safety concerns. Administration of ENV905 allows for significant dose-sparing when compared with the standard of care regimen for topical DUREZOL^{®} due to the location of administration as well as the release kinetics of ENV905.

**Table 42: Pharmacokinetics from ENV905-ADME-002**

| **Test Article** | **Matrix** | **Parent: Difluprednate (DFBA)** | | | | | | **Metabolite: Desacetyl Difluprednate (DFB)** | | | | | |
|---|---|---|---|---|---|---|---|---|---|---|---|---|---|
| | | Cₘₐₓ (ng/mL or g) | Tₘₐₓ (d) | T_{1/2} (d) | Cₗₐₛₜ (ng/mL or g) | Tₗₐₛₜ (d) | AUCₗₐₛₜ (d*ng/mL or g) | Cₘₐₓ (ng/mL or g) | Tₘₐₓ (d) | T_{1/2} (d) | Cₗₐₛₜ (ng/mL or g) | Tₗₐₛₜ (d) | AUCₗₐₛₜ (d*ng/mL or g) |
| DUREZOL^{®} (up to 1925 µg/eye, 3850 µg/animal) | Plasma | NA | NA | NA | NA | NA | NA | 0.41 | 2 | NC | 0.26 | 28 | 8.83 |
| | Aqueous Humor | 2.43 | 4 | 10.2 | 0.01 | 28 | 5.24 | 64 | 1 | NC | 49.2 | 28 | 1047 |
| | Bulbar Conjunctiva | 6.52 | 28 | NC | 6.52 | 28 | 66.5 | 379 | 1 | 1.8 | 0.11 | 42 | 6528 |
| | Cornea | 56.8 | 7 | NC | 7.81 | 28 | 310 | 810 | 28 | NC | 0.43 | 42 | 19996 |
| | Trabecular Meshwork | 11.4 | 7 | 8.9 | 2.11 | 28 | 100 | 765 | 1 | NC | 717 | 28 | 15530 |
| | Iris/ Ciliary Body | 0.65 | 7 | NC | 0.65 | 7 | 2.64 | 118 | 4 | NC | 109 | 28 | 2683 |
| | Retina | 33.4 | 4 | 6.3 | 2.78 | 28 | 160 | 87.5 | 4 | NC | 25.8 | 28 | 749 |
| ENV905-1 (42.8 µg/eye, 85.6 µg/animal) | Plasma | NA | NA | NA | NA | NA | NA | 0.99 | 1 | NC | 0.99 | 1 | 0.495 |
| | Aqueous Humor | 3595 | 1 | NC | 305 | 2 | 3131 | 3420 | 1 | 0.3 | 0.022 | 7 | 3323 |
| | Bulbar Conjunctiva | 0.575 | 1 | NC | 0.551 | 2 | 0.85 | 106 | 1 | 1.9 | 0.37 | 14 | 135 |
| | Cornea | 641 | 1 | 0.2 | 0.088 | 4 | 609 | 11283 | 1 | 3.2 | 0.65 | 14 | 9329 |
| | Trabecular Meshwork | 544 | 1 | NC | 88.3 | 2 | 523 | 9435 | 1 | 0.5 | 0.23 | 14 | 8571 |
| | Iris/ Ciliary Body | 58125 | 1 | NC | 30020 | 2 | 71599 | 13115 | 1 | 0.4 | 0.2 | 7 | 13174 |
| | Retina | 30.1 | 1 | NC | 0.77 | 2 | 23.1 | 89.9 | 1 | 0.8 | 0.33 | 7 | 92.1 |
| ENV905-1 (85.6 µg/eye, 171.2 µg/animal) | Plasma | NA | NA | NA | NA | NA | NA | 1.98 | 1 | NC | 0.1 | 2 | 1.62 |
| | Aqueous Humor | 25065 | 1 | NC | 77 | 7 | 21679 | 9273 | 1 | 0.7 | 24 | 7 | 8621 |
| | Bulbar Conjunctiva | 18.8 | 4 | NC | 18.8 | 4 | 49.1 | 377 | 1 | 1.2 | 4.76 | 7 | 447 |
| | Cornea | 4029 | 1 | 0.5 | 0.665 | 7 | 3815 | 22750 | 1 | 0.5 | 0.21 | 14 | 19984 |
| | Trabecular Meshwork | 4215 | 1 | 0.5 | 1.04 | 7 | 4934 | 18050 | 1 | 0.7 | 28 | 7 | 18695 |
| | Iris/ Ciliary Body | 181650 | 2 | 1.4 | 0.43 | 21 | 361171 | 32275 | 1 | 0.6 | 37.9 | 7 | 38503 |
| | Retina | 363 | 4 | NC | 363 | 4 | 409 | 137 | 1 | NC | 45.5 | 4 | 169 |
| | Plasma | NA | NA | NA | NA | NA | NA | 2.75 | 1 | 0.5 | 0.04 | 4 | 2.44 |
| | Aqueous Humor | 46050 | 1 | 0.7 | 0.023 | 14 | 41629 | 11158 | 1 | 0.6 | 5.57 | 7 | 10427 |
| ENV905-1 (128.4 µg/eye, 256.8 µg/animal) | Bulbar Conjunctiva | 48.7 | 4 | NC | 0.098 | 21 | 175 | 233 | 1 | 0.9 | 1.63 | 7 | 716 |
| | Cornea | 5640 | 1 | 0.4 | 0.335 | 7 | 7777 | 39875 | 1 | 3.7 | 0.08 | 28 | 33262 |
| | Trabecular Meshwork | 16703 | 2 | 0.3 | 0.38 | 7 | 25656 | 24825 | 1 | 0.6 | 15.8 | 7 | 29174 |
| | Iris/ Ciliary Body | 243775 | 1 | 1.3 | 1.13 | 28 | 460790 | 41700 | 1 | 0.7 | 0.09 | 28 | 49403 |
| | Retina | 3011 | 4 | NC | 0.485 | 7 | 4508 | 222 | 4 | 0.8 | 0.83 | 7 | 764 |

### Toxicology Summary for ENV905

Difluprednate, a corticosteroid prodrug of the active metabolite DFB, is marketed as a 0.05% sterile preserved emulsion for topical ocular delivery. DUREZOL^{®} is administered as a 4 times per day drop (approximately 100 µg per day) in patients recovering from ocular surgery or patients with endogenous anterior uveitis. The toxicology profile of difluprednate was investigated under NDA 22-212. Effects noted in nonclinical studies of difluprednate included decreases in body weight and adrenal effects, were deemed to be due to the pharmacologic action of the molecule, and are well known glucocorticosteroid effects (DUREZOL^{®} Package Insert).

The disclosed ENV905 (difluprednate) Ophthalmic Implant is an injectable difluprednate implant formulation using a biocompatible polyethylene glycol (PEG)-based drug delivery system. The implant is designed for ophthalmic administration via subconjunctival (SCJ) or intracameral (IC) injection, with a single dose administered following ocular surgery targeting duration of action of 28 days. The PEG-based drug delivery system is comprised of a blend of two PEGs that function as both diluent and binder, and was designed for rapid dissolution of the implant and concurrent release of difluprednate. The bioavailability and sustained therapeutic effect of ENV905 over 28 days is governed by multiple factors, including route of administration and the physicochemical properties of the drug substance difluprednate. ENV905 is formulated for the treatment of inflammation and pain associated with ocular surgery. ENV905 is formulated as a solid, rod-shaped implant of dimensions between 225-350 µm in width by 2,925-6,000 µm in length. The implants are loaded into the needle of a single-use implant applicator and delivered into either the subconjunctival space or the anterior chamber where they will quickly disintegrate.

As shown in **Table 43,** multiple formulations of ENV905 administered by either SCJ or IC insertion were assessed for tolerability in the pharmacology studies conducted in NZW rabbits described above. Tolerability endpoints included body weights, daily clinical observations, ophthalmic exams conducted by a board-certified veterinary ophthalmologist, and ocular observations during enucleation at sacrifice.

**Table 43: ENV905 Formulations Tested in Tolerability and Toxicology Studies in New Zealand White Rabbits**

| **ENV905 Formulation ID** | **PEG Ratio** | **Implant Dimensions (µm)** | **Number of Implants/ Eye** | **Total Dose (µg difluprednate/ Eye)** | **Route of Administration** | **Study Number** |
|---|---|---|---|---|---|---|
| ENV-1I-115-4 (Placebo) | PEG 35K / PEG 100K (70/30) | 300 x 300 x 6,000 | 2 | 0 | SCJ | ENV905-PRE-005 |
| ENV-1I-53-28-1¹ | PEG 35K / PEG100K (70/30) | 300 x 300 x 6,000 | 2 | 432 | SCJ | ENV905-PRE-005 |
| ENV-1I-115-2 | PEG 35K / PEG100K (70/30) | 300 x 300 x 6,000 | 2 | 826 | SCJ | ENV905-PRE-005 |
| ENV905-1 Placebo | PEG 100K/ PEG 3,350 (89/11) | 225 x 225 x 2,925 | 3 | 0 | IC | ENV905-PRE-008 |
| ENV905-5 Placebo | PEG 100K/ PEG 3,350 (89/11) | 130 x 180 x 1,500 | 3 | 0 | IC | ENV905-PRE-008 |
| ENV905-5 | PEG 100K/ PEG 3,350 (89/11) | 130 x 180 x 1,500 | 3 | 60 | IC | ENV905-PRE-008 |
| ENV905-1 Placebo | PEG 100K/ PEG 3,350 (89/11) | 225 x 225 x 2,925 | 3 | 0 | IC | ENV905-TOX-001 |
| ENV905-1 | PEG 100K/ PEG 3,350 (89/11) | 225 x 225 x 2,925 | 1, 2, or 3 | 44, 88, or 132 | IC | ENV905-TOX-001 |

There were no observed adverse effects following a single administration of ENV905 (range 40 to 1,239 µg difluprednate per eye) or placebo implant noted in any of the aforementioned pharmacology studies.

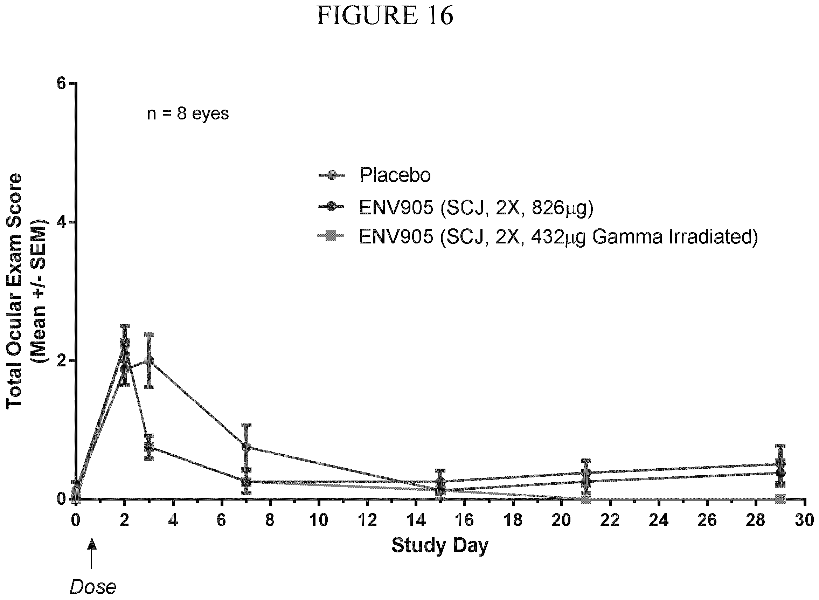

A non-GLP tolerability study of SCJ ENV905 was conducted in NZW rabbits. Male rabbits were administered a single bilateral SCJ insertion of ENV905 or placebo implant and were followed for 28 days. Tolerability endpoints included body weights, daily clinical observations, ophthalmic exams, and ocular histopathology at sacrifice. Ophthalmic exams and histopathology assessment were conducted by a board-certified veterinary ophthalmologist.ENV905 (0, 432, or 826 µg difluprednate per eye) was well tolerated in the NZW rabbit for 28 days, no adverse effects were noted, and no evidence of ocular toxicity in clinical or histologic observation was observed over 29 days. *See,* e.g., FIG. 16.

A non-GLP tolerability study of IC ENV905 was conducted in NZW rabbits. New Zealand White rabbits (4 males / group) were administered a single bilateral intracameral injection of three ENV905 active or placebo implants, or received a single bilateral intracameral injection without implants (sham), and were followed for 7 days. The eyes were examined on Days 0 (baseline), 1 (8 hours post-dose), and on Days 2 (~24 hours post-dose), 3 (~48 hours post-dose), 4, 5, 6, and 7 by a board-certified veterinary ophthalmologist for signs of inflammation using the Hackett-McDonald scoring system, and animals were observed daily. Central and inferior corneal thickness was measured using an ultrasonic pachymeter at baseline, approximately 8 hours post-injection, and on Days 2, 3, 4, 5, 6, and 7. *See* FIG. 25A and 25B, respectively. Non-contact specular microscopy (NCSM) images were also taken on Days 0 (baseline), 1 (~8 hours post-dose), and on Days 2, 3, 4, 5, 6, and 7. ENV905-1 and ENV905-5 implants were well tolerated. However, the ENV905-1 placebo implants were associated with consistently increased central and inferior corneal thickness and slightly higher histopathology scores compared to ENV905-5 placebo and active implants or sham. *See* **FIG. 25A** and **25B****,** respectively. These results indicates that the corneal thickening effect observed with the ENV905-5 placebo is similar to that observed with the sham (injection), and more corneal thickening occurs with higher polymer matrix content (ENV905-1 placebo). Both ENV905-1 (active) and ENV905-5 (active) show a reduction in corneal thickening. ENV905-5 placebo implants did not induce any additional inflammation or corneal effects when compared with sham, and the corneal thinning observed in the ENV905-5 active group was associated with ocular corticosteroid administration as expected and was not considered adverse. *See* **FIG. 25A** and **25B****,** respectively.

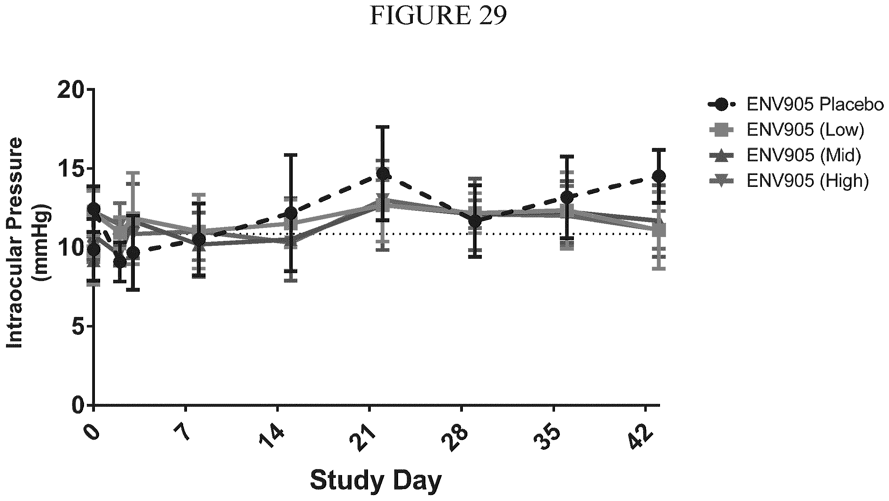

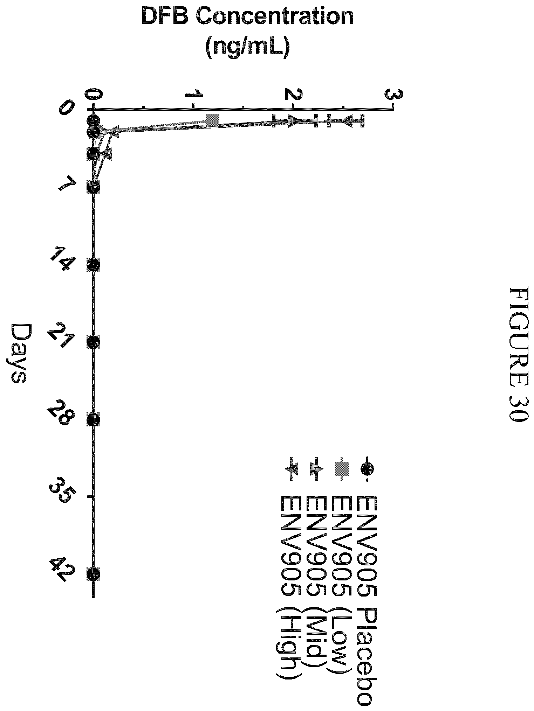

A GLP toxicology study of IC ENV905 was conducted in NZW rabbits. Male and female rabbits received a single administration of ENV905-1 and its relevant placebo, and were followed for 42 days. Assessment of toxicity was based on mortality, clinical observations, qualitative food consumption, body weight, ophthalmic examinations (slit lamp biomicroscopy and indirect ophthalmoscopy), intraocular pressure (IOP) measurements, pachymetry, electroretinography (ERG; scotopic tests), and clinical and anatomic pathology. Blood samples were collected for toxicokinetic evaluations. Male and female rabbits were each given three ENV905-1 Placebo ophthalmic implants; or one, two, or three ENV905-1 (difluprednate) ophthalmic implants, each containing 44.0 µg difluprednate/implant in the right and left eyes via intracameral injection using ENV905-1 Applicators (dose levels of 44.0 (Low), 88.0 (Mid), or 132.0 (High) µg/eye). No test article-related clinical findings or effects on mortality, clinical observations, qualitative food consumption, body weight, or body weight change were noted. Ophthalmic findings and IOP changes in the active ENV905-1 groups were limited to clinically beneficial effects on the procedure-associated inflammation (*See* **FIG. 29****);** signs of inflammation noted in the placebo group were generally resolved by Day 3. Changes in axial corneal thickness and in ERG were not clinically important. Clinical pathology changes in all groups given ENV905-1 implants included mildly increased glucose; moderately decreased white blood cell and absolute lymphocyte, eosinophil, and basophil counts; and mildly decreased calcium, which resolved during the study period. Test article-related microscopic findings and organ weight changes in systemic tissues evaluated were limited to pharmacologic effects of the test article in the liver and thymus at the interim sacrifice and are known glucocorticoid effects; these were absent at the terminal sacrifice. Test article-related microscopic observations in the bulbar conjunctiva and eyes consisted of decreases in the severity and incidence of mixed cell inflammation and heterophilic infiltrates. No ENV905-1-related organ weight changes or microscopic findings (systemic or ocular tissues) were present at the terminal sacrifice, indicating findings had recovered by Day 43. No ENV905-1-related macroscopic observations were noted at the interim or terminal sacrifice. Plasma concentration of Deacetyl Difluprednate (DFB, active metabolite) peaked at 2 hours post-dose (the first time point). *See* **FIG. 30****.** ENV905-1 (High) had a Cₘₐₓ of 2.5 ng/mL. DFB concentrations were not quantifiable following Day 4 for ENV905-1 (High). Due to the mild severity of findings and the lack of impact on the health and wellbeing of animals given 132.0µg/eye, effects at this dose were not considered adverse. Thus, the no observed adverse effect level (NOAEL) is 132.0 µg/eye.

Thus, the tolerability profile of ENV905 and placebo implant has been demonstrated in multiple non-GLP pharmacology studies, a single non-GLP tolerability study, and a single GLP toxicology study, and no findings have been observed to date that preclude the continued development of ENV905.

### Known Toxicology of Difluprednate (DUREZOL^{®})

The toxicology profile of difluprednate was investigated under DUREZOL^{®} NDA 22-212. In multiple studies performed in rodents and non-rodents, subchronic and chronic toxicity tests of difluprednate showed systemic effects such as suppression of body weight gain; a decrease in lymphocyte count; atrophy of the lymphatic glands and adrenal gland; and for local effects, thinning of the skin; all of which were due to the pharmacologic action of the molecule and are well known glucocorticosteroid effects. Most, if not all of these effects were reversible after drug withdrawal. The no-observed-effect-level (NOEL) for the subchronic and chronic toxicity tests were consistent between species and ranged from 1-1.25 µg/kg/day (DUREZOL^{®} Package Insert).

Difluprednate was not genotoxic in vitro in the Ames test, and in cultured mammalian cells CHL/IU (a fibroblastic cell line derived from the lungs of newborn female Chinese hamsters). An in vivo micronucleus test of difluprednate in mice was also negative. Treatment of male and female rats with subcutaneous difluprednate up to 10 µg/kg/day prior to and during mating did not impair fertility in either gender. Long-term studies have not been conducted to evaluate the carcinogenic potential of difluprednate (DUREZOL^{®} Package Insert).

Difluprednate is categorized as Pregnancy Category C. Difluprednate has been shown to be embryotoxic (decrease in embryonic body weight and a delay in embryonic ossification) and teratogenic (cleft palate and skeletal anomalies) when administered subcutaneously to rabbits during organogenesis at a dose of 1-10 µg/kg/day. The NOEL for these effects was 1 µg/kg/day, and 10 µg/kg/day was considered to be a teratogenic dose that was concurrently found in the toxic dose range for fetuses and pregnant females. Treatment of rats with 10 µg/kg/day subcutaneously during organogenesis did not result in any reproductive toxicity, nor was it maternally toxic. At 100 µg/kg/day after subcutaneous administration in rats, there was a decrease in fetal weights and delay in ossification, and effects on weight gain in the pregnant females (DUREZOL^{®} Package Insert).

### Known Toxicology of PEG Excipients

High molecular weight PEGs, also known as polyethylene oxide (PEO) are polymers of ethylene oxide with a formula of HO-(CH₂-CH₂-O)ₙ-H, where n is the number (average) of ethylene glycol groups present in the molecule.

PEGs are not single chemical entities, but are mixtures of various polymer chain lengths, and for some of the higher molecular weight materials they can also be branched (Morpurgo 2004).

PEG is commonly used in cosmetics, PEGylated biologics, and in numerous approved and marketed therapeutics (Inactive Ingredients Database). These polymers are largely excreted unchanged in the urine, with hepatobiliary clearance representing a relatively minor pathway (Webster 2009). PEGs have been used extensively in approved products across multiple routes of administration including oral, topical, ophthalmic, rectal, vaginal, intrasynovial, intra-articular, intramuscular, and intravenous. PEGs are generally considered to have low toxicity and have been tested across multiple routes of administration. PEGs with a wide range of molecular weights have been shown to be safe (Herold 1982, Smyth 1947, Smyth 1950, Smyth 1970, Webster 2009). However, significant systemic exposure to PEGs via some routes of administration (mainly topical application to burn victims) has resulted in renal toxicity (Herold 1982). Studies of intravenous PEG have shown that urinary clearance decreased with increasing PEG molecular weight, similar to the tissue clearance, whereas liver clearance increased with increasing PEG molecular weight (Yamaoka 1993). After intravenous, intramuscular, and subcutaneous administration, PEGs are generally excreted almost completely within 24 hours, mainly in the urine (MAK collection). Furthermore, intravenous administration of PEG 4,000,000 g/mol MW at dose levels of up to 10 mg/kg had no evident toxicological effect (Smyth 1970). PEGs are classified as Pregnancy Category C.

ENV905 ocular implant formulations may contain, in certain embodiments, approximately 11.4 or 52.8 µg of PEG 3,350 g/mol MW and 92.6 or 427.2 µg of PEG 100,000 g/mol MW per implant for the intracameral or subconjunctival implants, respectively.

Based on current thinking for the proposed dose levels in the GLP toxicology studies (up to 4 implants dosed bilaterally, if feasible), animals could receive up to 422.4 µg PEG 3,350 and 3,417.6 µg PEG 100,000 per animal, while the exposure in the placebo implant groups could be as much as twice that as the placebo implants will be equal in mass to the ENV905 implants but will not contain active pharmaceutical ingredient (API).

Systemic exposure to molecules administered via the IC or SCJ routes is generally minimal, although exposure to PEG using these routes is unknown. Clinical pathology parameters, including renal factors BUN and creatinine, as well as liver enzyme levels, will be measured predose and at sacrifice in all animals in the GLP toxicology study to assess the effect of potential systemic exposure to PEG; gross pathology at necropsy and organ weights will also be monitored. Systemic histopathology is not planned.

### ENV905 Implant Applicator

In embodiments, the ENV905 ocular implant administrations have been performed using an ENV905 implant applicator; this sterile applicator is comprised of either a 21 G to 22 G (for ENV905-2), or a 25 G to 27 G (for ENV905-1, ENV905-3, ENV905-4, and ENV905-5) single-lumen hypodermic needle and stainless steel pushrod attached to a standard 1-mL syringe.

ENV905 implants can be injected using the ENV905 ocular implant applicator into the subconjunctival space or anterior chamber of the eye using said injection tool.

The sterile implant applicator and the sterile ENV905 implants, in some aspects, can be packaged separately and loaded in a sterile field in an operating room prior to administration to patients scheduled for cataract surgery.

In some embodiments, ENV905 implants will be preloaded into the sterile applicator, with the needle hub assembly of the implant applicator functioning as a container closure for the implants.

### ENV905 Implant Applicator Design

In embodiments, the applicator will be supplied as a single-use, sterile, needle-based instrument for delivery of the ENV905 implants into the subconjunctival space or anterior chamber. In aspects, the applicator can utilize a 510K-approved, 21 G, 22 G, 23 G, 24 G, 25 G, 26 G, or 27 G, single-lumen hypodermic needle to deliver the ENV905 implant(s). A stainless steel metal shaft actuated via scroll wheel will advance the rod-shaped ENV905 implants from the lumen of the needle. The ENV905 implant applicator, in some embodiments, will be terminally sterilized via gamma irradiation following assembly and packaging. In some aspects, the ENV905 implant applicator will be packaged in a Tyvek^{™} pouch and terminally sterilized via gamma irradiation in accordance with a validated sterilization method.

### Prophetic Example 1: ENV905 in Cataract Surgery

The following example is prophetic in nature and describes experiments to be performed and predicted results, rather than work actually conducted or results actually achieved. Clinical efficacy and safety is evaluated in randomized, active comparator controlled study in which subjects after cataract extraction followed by intraocular lens implantation are treated with DUREZOL (difluprednate ophthalmic emulation 0.05%) or ENV905 containing smaller dose of difluprednate vs. what is administered to the patient via DUREZOL over the treatment period of 4 weeks after the surgery. DUREZOL is administered as indicated 4 times daily beginning 24 hours after surgery and continuing throughout the first 2 weeks of the postoperative period, followed by 2 times daily for a week and then a taper based on the response. The presence of complete clearing (a cell count of 0) is assessed 8 and 15 days post-surgery. All adverse events are tracked and evaluated, including adverse event of intraocular pressure (IOP) elevation. The primary efficacy results based on presence of complete clearing as described above show non-inferior control of ocular inflammation in patients dosed with ENV905.

The adverse event of IOP elevation is often characterized as IOP elevation by more than 10 mmHg. It has been shown for DUREZOL that the adverse event of IOP elevation occurs approximately in the range of 5-10% percent of the patients. ENV905, while demonstrating non-inferior treatment effect, will demonstrate improved safety and lesser incidence of adverse event of IOP elevation over DUREZOL's incidence of 5-10%. Thus, it is expected that the incidence of adverse events of IOP elevation is lower in patients dosed with ENV905 compared to patients dosed with DUREZOL.

### Prophetic Example 2: ENV905 in Endogenous Anterior Uveitis

The following example is prophetic in nature and describes experiments to be performed and predicted results, rather than work actually conducted or results actually achieved. Clinical efficacy and safety is evaluated in randomized, active comparator controlled study in which subjects who present with endogenous anterior uveitis are assigned to DUREZOL (difluprednate ophthalmic emulation 0.05%) or ENV905 containing smaller dose of difluprednate vs. what is administered to the patient via DUREZOL over the treatment period. DUREZOL is administered as indicated 4 times daily continuing throughout 2 weeks. The presence of complete clearing (a cell count of 0) is assessed at 8 and at 15 days. All adverse events are tracked and evaluated, including adverse event of intraocular pressure (IOP) elevation. The primary efficacy results based on presence of complete clearing as described above show non-inferior control of ocular inflammation in patients dosed with ENV905.

The adverse event of IOP elevation is often characterized as IOP elevation by more than 10 mmHg. It has been shown for DUREZOL that the adverse event of IOP elevation occurs approximately in the range of 5-10% percent of the patients. ENV905, while demonstrating non-inferior treatment effect, will demonstrate improved safety and lesser incidence of adverse event of IOP elevation over DUREZOL's incidence of 5-10%. Thus, it is expected that the incidence of adverse events of IOP elevation is lower in patients dosed with ENV905 compared to patients dosed with DUREZOL.

### LIST OF ABBREVIATIONS

| **Abbreviation** | **Definition** |
|---|---|
| % RSD | relative standard deviation |
| % v/v | % volume/volume |
| % w/w | % weight/weight |
| °C | degrees Celsius |
| °F | degrees Fahrenheit |
| µg | microgram |
| µL | microliter |
| µm | micrometer |
| λ_{avg} | average wavelength |
| AH | aqueous humor |
| API | active pharmaceutical ingredient |
| AUCₗₐₛₜ | area under the plasma concentration-time curve from time zero to the time of the last quantifiable concentration |
| BCVA | best corrected visual acuity |
| BID | twice daily |
| BLQ | below limit of quantitation |
| BSC | biological safety cabinet |
| c | concentration |
| CAS # | chemical abstracts service number |
| CDER | Center for Drug Evaluation and Research |
| CFU | colony-forming unit |
| cGMP | current Good Manufacturing Practices |
| Cₘₐₓ | maximal concentration |
| CMC | chemistry, manufacturing, and controls |
| cPs | centipoise |
| Da | daltons |
| DFB | 6α,9-difluoroprednisolone 17-butyrate (desacetyl difluprednate) |
| DFBA | 6α, 9-difluoro-11β,17,21-trihydroxypregna-1,4-diene-3,20-dione 21 acetate 17-butyrate (difluprednate) |
| DMF | Drug Master File |
| DPT | (diphenyl(2,4,6-trimethylbenzoyl) phosphine oxide/2-hydroxy-2-methylpropiophenone) |
| DTOP | Division of Transplant and Ophthalmology Products |
| ETDRS | Early Treatment Diabetic Retinopathy Study |
| EU | endotoxin units |
| FCR | Fluorocur^{®} (mold) |
| FDA | United States Food and Drug Administration |
| FID | flame ionization detector |
| ft | foot |
| FTIR | Fourier transform infrared spectrometry |
| g | gram |
| G | gauge |
| GC | gas chromatography |
| GLP | Good Laboratory Practices |
| GR | glucocorticoid receptor |
| GCRBA | glucocorticoid receptor-binding activity |
| H | half |
| HPLC | high performance liquid chromatography |
| HPLC RT | high performance liquid chromatography retention time |
| IC | intracameral |
| ICH | International Conference on Harmonization |
| IND | Investigational New Drug |
| IOP | intraocular pressure |
| ISO | International Standard for Organization |
| K | thousand (number) |
| kGy | kilogray |
| Kᵢ | inhibition constant |
| L | liter |
| l.c. | label claim |
| LC-MS/MS | liquid chromatography and tandem mass spectrometry |
| LLOC | lower limit of quantification |
| M | molar |
| ME | masked extension |
| mg | milligram |
| min | minute |
| mm | millimeter |
| MTD | maximum tolerated dose |
| MW | average molecular weight |
| N, n, or No. | number |
| NA | not applicable |
| NDA | New Drug Application |
| NF | National Formulary |
| ng | nanogram |
| NLT | not less than |
| nm | nanometer |
| NMT | not more than |
| NOEL | no-observed-effect-level |
| NZW | New Zealand white (rabbit) |
| PD | pharmacodynamic |
| PEG | polyethylene glycol |
| PEO | polyethylene oxide |
| PET | polyethylene terephthalate |
| PK | pharmacokinetic |
| ppm | parts per million |
| psi | pounds per square inch |
| PTFE | polytetrafluoroethylene |
| q.s. ad | quantities sufficient to make |
| QC | quality control |
| QID | four times daily |
| RH | relative humidity |
| RT | retention time |
| SAE-04 | mold resin component |
| SCJ | subconjunctival |
| SD | standard deviation |
| SDS | sodium dodecyl sulfate |
| sec | seconds |
| T or t | time |
| TBD | to be determined |
| Tₘₐₓ | time of Cₘₐₓ |
| TMP | diphenyl(2,4,6-trimethylbenzoyl)phosphine oxide |
| US | United States |
| USP | United States Pharmacopoeia |
| USP-NF | United States Pharmacopoeia - National Formulary |
| UV | ultraviolet |
| VA | visual acuity |

### SUMMARY OF EMBODIMENTS OF OCULAR IMPLANT FORMULATIONS

| | Dimension of implant | Volume of implant | AI in implant (µg) | AI in implant (%w/w) | PEG (µg) in implant (3,350/100,000) | PEG % w/w in implant (3,350/100,000) | Total implant (µg) | Needle (ga) |
|---|---|---|---|---|---|---|---|---|
| ENV905-1 | 225 x 225 x 2925 | 148,078,125 | 24-56 | 15-35 | 104-136 (11.4-15.0/92.6-121.0) | 65-85 (7.2-9.4/57.9-75.7) | 128-192 | 27 |
| ENV905-3 | | | 16-24 | 10-15 | 136-144 (15-15.8/121-128.2) | 85-90 (9.4-9.9/75.7-80.1) | 152-168 | |
| ENV905-4 | | | 8-12 | 5-7.5 | 148-152 (16.3-16.7/131.7-135.3) | 92.5-95.0 (10.2-10.5/82.3-84.6) | 156-164 | |
| ENV905-2 | 300 x 300 x 6000 | 540,000,000 | 320-480 | 40-60 | 320-480 (35.2-52.8/284.8-427.2) | 40-60 (4.4-6.6/35.6-53.4) | 640-960 | 21, 22 |
| ENV905-5 | 130 x 180 x 1500 | 35,100,000 | 16-24 | 29.6-44.4 | 30-38 (3.3-4.2/26.7-33.8) | 55.6-70.4 (6.1-7.7/49.4-62.9) | 46-62 | 25, 26, 27 |

### INCORPORATION BY REFERENCE

All references, articles, publications, patents, patent publications, and patent applications cited herein, are hereby incorporated by reference in their entireties for all purposes.

### REFERENCES CITED

Burns E, Mulley GP. Practical problems with eye-drops among elderly ophthalmology outpatients. Age and Ageing. 1992;21:168-170.

Cheng CL, Chee SP, Tan DT. Patient reliability in the administration of topical ocular medication. Singapore Med J. 2001 Jun;42(6):252-4.

Chennamaneni SR, Mamalis C, Archer B, Oakey Z, Ambati BK. Development of a novel bioerodible dexamethasone implant for uveitis and postoperative cataract inflammation. J Control Release. 2013:167;53-59.

Croasdale CR, Brightbill FS. Subconjunctival corticosteroid injections for nonnecrotizing anterior scleritis. Arch Ophthalmol. 1999 Jul;117(7):966-8.

Dieleman M, Wubbels RJ, van Kooten-Noordzij M, de Waard PW. Single perioperative subconjunctival steroid depot versus postoperative steroid eyedrops to prevent intraocular inflammation and macular edema after cataract surgery. J Cataract Refract Surg. 2011;37:1589-1597.

Donnenfeld ED. Difluprednate for the prevention of ocular inflammation postsurgery: An update. Clin Ophthalmol. 2011;5:811-6.

DUREZOL® Package Insert. Alcon Laboratories, Inc. Fort Worth, Texas. 2013.

Foster CS, Davanzo R, Flynn TE, McLeod K, Vogel R, Crockett RS. DUREZOL® (Difluprednate Ophthalmic Emulsion 0.05%) compared with Pred Forte 1% ophthalmic suspension in the treatment of endogenous anterior uveitis. J Ocul Pharmacol Ther. 2010 Oct;26(5):475-83.

Gaudio PA. A review of evidence guiding the use of corticosteroids in the treatment of intraocular inflammation. Ocul Immunol Inflamm. 2004 Sep;12(3):169-92.

Hackett, RD and McDonald, TO. Ophthalmic Toxicology and Assessing Ocular Irritation. Dermatoxicology, 5th edition. Ed. FN Marzulli and HI Maibach. Washington, DC: Hemisphere Publishing Corporation. 1996; 299-305 and 557-566.

Herold DA, Rodeheaver GT, Bellamy WT, Fitton LA, Bruns DE, Edlich RF. Toxicity of topical polyethylene glycol. Toxicol Appl Pharmacol. 1982;65(2):329-335.

FDA. Inactive Ingredients Database. 24 October 2013. Accessed 12 December 2014. http://www.accessdata.fda.gov/scripts/cder/iig/index.cfmhttp://www.accessdata.fda.gov/scripts/c der/iig/index.cfm

The International Pharmaceutical Excipients Council of the Americas. IPEC-FDA OGD Meeting. 09 December 2011. Accessed 12 December 2014.

Korenfeld MS, Silverstein SM, Cooke DL, Vogel R, Crockett. Difluprednate ophthalmic emulsion 0.05% for postoperative inflammation and pain. J Cataract Refract Surg. 2009 Jan;35(1):26-34.

Koval MS, Moster MR, Freidl KB, Waisbourd M, Jain SG, Ichhpujani P, Myers JS, Pro MJ. Intracameral triamcinolone acetonide in glaucoma surgery: a prospective randomized controlled trial. Am J Ophthalmol. 2014 Aug;158(2):395-401.

Morpurgo M, Veronese FM. "Conjugates of peptides and proteins to polyethylene glycols." Bioconjugation Protocols: Strategies and Methods. Ed. CM Niemeyer. Totowa, NJ: Humana Press, 2004. Print.

Polyethylene glycol [MAK Value Documentation, 1998]. The MAK Collection for Occupational Health and Safety. 2012; 248-270.

Rowland and Tozer, "Clinical Pharmacokinetics and Pharmacodynamics: Concepts and Applications," 4th Ed., 2011, Lippincott Williams & Wilkins publishers.

Shell JW. Ophthalmic drug delivery systems. Surv Ophthalmol. 1984:29;117-128.

Smyth, HF, Carpenter, CP, Shaffer, C B. The toxicity of high molecular weight polyethylene glycols; chronic oral and parenteral administration. J Am Pharm Assoc. 1947;36(5): 157-160.

Smyth, HF, Carpenter, CP, Weil, CS. The toxicology of the polyethylene glycols. J Am Pharm Assoc. 1950;39(6): 349-354.

Smyth HF, Weil CS, Woodside MD, Knaak JB, Sullivan LJ, Carpenter CP. Experimental toxicity of a high molecular weight poly(ethylene oxide). Toxicol Appl Pharmacol. 1970;16:442-445.

Tajika T, Isowaki A, Sakaki H. Ocular distribution of difluprednate ophthalmic emulsion 0.05% in rabbits. J Ocul Pharmacol Ther. 2011 Feb;27(1):43-9.

Tajika T, Waki M, Tsuzuki M, Kida T, Sakaki H. Pharmacokinetic features of difluprednate ophthalmic emulsion in rabbits as determined by glucocorticoid receptor-binding bioassay. J Ocul Pharmacol Ther. 2011 Feb;27(1):29-34.

Webster R, Elliott V, Park BK, Walker D, Hankin M., Taupin P. "PEG and PEG conjugates toxicity: towards an understanding of the toxicity of PEG and its relevance to PEGylated biologicals." PEGylated protein drugs: Basic science and clinical applications. Ed. FM Veronese. Basel: Birkhauser, 2009. Print.

Winfield AJ, Jessiman D, Williams A, Esakowitz L. A study of the causes of non-compliance by patients prescribed eyedrops. Brit J Ophthalmol, 1990:74;477-480.

Yamaoka T, Tabata Y, Ikada Y. Distribution and tissue uptake of poly(ethylene glycol) with different molecular weights after intravenous administration to mice. J Pharm Sci. 1994 Apr;83(4):601-606.

### Embodiments

1. An extended treatment rapid delivery system for treating post-operative ocular inflammation in an eye of a patient in need thereof, comprising:
   A) a biocompatible polymer matrix based ocular implant rapid delivery vehicle; and
   B) at least one therapeutic agent homogeneously dispersed within the polymer matrix,

   wherein said ocular implant rapid delivery vehicle is formulated to substantially disintegrate within 24 hours of being placed into the eye, and
   wherein said disintegration of the ocular implant rapid delivery vehicle results in substantially all of the at least one therapeutic agent being released into the eye within 24 hours, and
   wherein the at least one therapeutic agent provides a therapeutic effect to the eye of the patient for at least 2 days, and
   wherein said ocular implant rapid delivery vehicle is formulated to reduce post-operative corneal thickening in a patient in need thereof.
2. The extended treatment rapid delivery system of item 1, wherein the at least one therapeutic agent provides a therapeutic effect to the eye of the patient for at least 3 days.
3. The extended treatment rapid delivery system of item 1, wherein the at least one therapeutic agent provides a therapeutic effect to the eye of the patient for at least 5 days.
4. The extended treatment rapid delivery system of item 1, wherein the at least one therapeutic agent provides a therapeutic effect to the eye of the patient for at least 7 days.
5. The extended treatment rapid delivery system of item 1, wherein the at least one therapeutic agent provides a therapeutic effect to the eye of the patient for at least 14 days.
6. The extended treatment rapid delivery system of item 1, wherein the at least one therapeutic agent provides a therapeutic effect to the eye of the patient for at least 28 days.
7. The extended treatment rapid delivery system of item 1, wherein the at least one therapeutic agent has a low solubility under normal human eye physiological conditions.
8. The extended treatment rapid delivery system of item 1, wherein the at least one therapeutic agent has a water solubility of about 0.0097 mg/mL.
9. The extended treatment rapid delivery system of item 1, wherein the biocompatible polymer matrix comprises poly(ethylene glycol).
10. The extended treatment rapid delivery system of item 1, wherein the biocompatible polymer matrix comprises a mixture of two or more poly(ethylene glycol) polymers of different molecular weight.
11. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and one of said polymers has a molecular weight of about 100,000.
12. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 10-20 weight percent of the total polymer and one of said polymers has a molecular weight of about 100,000 and is present in an amount of about 80-90 weight percent of the total polymer.
13. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 11 weight percent of the total polymer and one of said polymers has a molecular weight of about 100,000 and is present in an amount of about 89 weight percent of the total polymer.
14. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 7-10 weight percent of the ocular implant and one of said polymers has a molecular weight of about 100,000 and is present in an amount of about 57-76 weight percent of the ocular implant, and wherein the at least one therapeutic agent is present in an amount of about 15-35 weight percent of the ocular implant.
15. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 4-7 weight percent of the ocular implant and one of said polymers has a molecular weight of about 100,000 and is present in an amount of about 35-54 weight percent of the ocular implant, and wherein the at least one therapeutic agent is present in an amount of about 40-60 weight percent of the ocular implant.
16. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 9-10 weight percent of the ocular implant and one of said polymers has a molecular weight of about 100,000 and is present in an amount of about 75-80 weight percent of the ocular implant, and wherein the at least one therapeutic agent is present in an amount of about 10-15 weight percent of the ocular implant.
17. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 10-11 weight percent of the ocular implant and one of said polymers has a molecular weight of about 100,000 and is present in an amount of about 82-85 weight percent of the ocular implant, and wherein the at least one therapeutic agent is present in an amount of about 5-8 weight percent of the ocular implant.
18. The extended treatment rapid delivery system of item 1, wherein the biocompatible polymer matrix comprises a mixture of two or more poly(ethylene glycol) polymers of different molecular weight, wherein one of said polymers has a molecular weight of about 3,350 and is present in an amount of about 6-8 weight percent of the ocular implant and one of said polymers has a molecular weight of about 100,000 and is present in an amount of about 49-63 weight percent of the ocular implant, and wherein the at least one therapeutic agent is present in an amount of about 29-45 weight percent of the ocular implant.
19. The extended treatment rapid delivery system of item 1, wherein the at least one therapeutic agent comprises about 15-35 weight percent of the ocular implant.
20. The extended treatment rapid delivery system of item 1, wherein the at least one therapeutic agent comprises about 16-24 weight percent of the ocular implant.
21. The extended treatment rapid delivery system of item 1, wherein the at least one therapeutic agent comprises about 8-12 weight percent of the ocular implant.
22. The extended treatment rapid delivery system of item 1, wherein the at least one therapeutic agent comprises about 320-480 weight percent of the ocular implant.
23. The extended treatment rapid delivery system of item 1, wherein the at least one therapeutic agent comprises about 16-24 weight percent of the ocular implant.
24. The extended treatment rapid delivery system of item 1, wherein the at least one therapeutic agent comprises a corticosteroid.
25. The extended treatment rapid delivery system of item 1, wherein the at least one therapeutic agent comprises difluprednate.
26. The extended treatment rapid delivery system of item 1, wherein the ocular implant rapid delivery vehicle is configured for intracameral or subconjunctival administration to the eye of a human.
27. The extended treatment rapid delivery system of item 1, wherein the ocular implant rapid delivery vehicle is rod-shaped with a shortest dimension of about 200-500 µm and a longest dimension of about 2,000-8,000 µm in length.
28. The extended treatment rapid delivery system of item 1, wherein the ocular implant rapid delivery vehicle is rod-shaped with dimensions of about 130 µm × 180 µm × 1,500 µm, about 225 µm × 225 µm × 2,925 µm, or about 300 µm × 300 µm × 6,000 µm.
29. The extended treatment rapid delivery system of item 1, wherein the ocular implant rapid delivery vehicle is rod-shaped with a volume of 35,100,000 cubic microns ± 10%, 148,078,125 cubic microns ± 10%, or 540,000,000 cubic microns ± 10%,.
30. The extended treatment rapid delivery system of item 1, wherein the ocular implant rapid delivery vehicle is rod-shaped with dimensions of about 225 µm × 225 µm × 2,925 µm, and wherein the at least one therapeutic agent is difluprednate present in an amount of from about 24 µg to about 56 µg.
31. The extended treatment rapid delivery system of item 1, wherein the ocular implant rapid delivery vehicle is rod-shaped with dimensions of about 225 µm × 225 µm × 2,925 µm, and wherein the at least one therapeutic agent is difluprednate present in an amount of from about 32 µg to about 48 µg.
32. The extended treatment rapid delivery system of item 1, wherein the ocular implant rapid delivery vehicle is rod-shaped with dimensions of about 225 µm × 225 µm × 2,925 µm, and wherein the at least one therapeutic agent is difluprednate present in an amount of from about 16 µg to about 24 µg.
33. The extended treatment rapid delivery system of item 1, wherein the ocular implant rapid delivery vehicle is rod-shaped with dimensions of about 225 µm × 225 µm × 2,925 µm, and wherein the at least one therapeutic agent is difluprednate present in an amount of from about 8 µg to about 12 µg.
34. The extended treatment rapid delivery system of item 1, wherein the ocular implant rapid delivery vehicle is rod-shaped with dimensions of about 300 µm × 300 µm × 6,000 µm, and wherein the at least one therapeutic agent is difluprednate present in an amount of from about 320 µg to about 480 µg.
35. The extended treatment rapid delivery system of item 1, wherein the ocular implant rapid delivery vehicle is rod-shaped with dimensions of about 130 µm × 180 µm × 1,500 µm, and wherein the at least one therapeutic agent is difluprednate present in an amount of from about 16 µg to about 24 µg.
36. The extended treatment rapid delivery system of any of items 1, wherein the post-operative corneal thickening is reduced to a baseline within 30 days after administration of said extended treatment rapid delivery system.
37. The extended treatment rapid delivery system of any of items 1, wherein the post-operative corneal thickening is reduced to at least a baseline within 15 days after administration of said extended treatment rapid delivery system.
38. The extended treatment rapid delivery system of any of items 1, wherein the post-operative corneal thickening is reduced to at least a baseline within 7 days after administration of said extended treatment rapid delivery system.
39. The extended treatment rapid delivery system of any of items 1, wherein the post-operative corneal thickening is reduced to at least a baseline within 1 day after administration of said extended treatment rapid delivery system.
40. The extended treatment rapid delivery system of item 1, wherein the post-operative corneal thickening is reduced below a baseline by about 1% to about 10%.
41. The extended treatment rapid delivery system of item 1, wherein the post-operative corneal thickening is reduced below the baseline by about 1% to about 10% within 1 day after after administration of said extended treatment rapid delivery system.
42. The extended treatment rapid delivery system of item 1, wherein when the extended treatment rapid delivery system is tested in a rabbit model, the average Cₘₐₓ for the therapeutic agent or active metabolite thereof in the plasma is about 80% to about 120% of 0.99-2.75 ng/mL.
43. The extended treatment rapid delivery system of item 1, wherein when the extended treatment rapid delivery system is tested in a rabbit model, the average AUCₗₐₛₜ for the therapeutic agent or active metabolite thereof in the plasma is about 80% to about 120% of 0.495-2.44 d*ng/mL.
44. The extended treatment rapid delivery system of item 1, wherein when the extended treatment rapid delivery system is tested in a rabbit model, the average Cₘₐₓ for the therapeutic agent or active metabolite thereof in the aqueous humor is about 80% to about 120% of 3,420-11,158 ng/mL.
45. The extended treatment rapid delivery system of item 1, wherein when the extended treatment rapid delivery system is tested in a rabbit model, the average AUCₗₐₛₜ for the therapeutic agent or active metabolite thereof in the aqueous humor is about 80% to about 120% of 3,323-10,427 d*ng/mL.
46. The extended treatment rapid delivery system of item 1, wherein when the extended treatment rapid delivery system is tested in a rabbit model, the average Cₘₐₓ for the therapeutic agent or active metabolite thereof in the bulbar conjunctiva is about 80% to about 120% of 106-233 ng/mL.
47. The extended treatment rapid delivery system of item 1, wherein when the extended treatment rapid delivery system is tested in a rabbit model, the average AUCₗₐₛₜ for the therapeutic agent or active metabolite thereof in the bulbar conjunctiva is about 80% to about 120% of 135-716 d*ng/mL.
48. The extended treatment rapid delivery system of item 1, wherein when the extended treatment rapid delivery system is tested in a rabbit model, the average Cₘₐₓ for the therapeutic agent or active metabolite thereof in the cornea is about 80% to about 120% of 11,283-39,875 ng/mL.
49. The extended treatment rapid delivery system of item 1, wherein when the extended treatment rapid delivery system is tested in a rabbit model, the average AUCₗₐₛₜ for the therapeutic agent or active metabolite thereof in the cornea is about 80% to about 120% of 9,329-33,262 d*ng/mL.
50. The extended treatment rapid delivery system of item 1, wherein when the extended treatment rapid delivery system is tested in a rabbit model, the average Cₘₐₓ for the therapeutic agent or active metabolite thereof in the trabecular meshwork is about 80% to about 120% of 9,435-24,825 ng/mL.
51. The extended treatment rapid delivery system of item 1, wherein when the extended treatment rapid delivery system is tested in a rabbit model, the average AUCₗₐₛₜ for the therapeutic agent or active metabolite thereof in the trabecular meshwork is about 80% to about 120% of 8,571-29,174 d*ng/mL.
52. The extended treatment rapid delivery system of item 1, wherein when the extended treatment rapid delivery system is tested in a rabbit model, the average Cₘₐₓ for the therapeutic agent or active metabolite thereof in the iris/ciliary body is about 80% to about 120% of 13,115-41,700 ng/mL.
53. The extended treatment rapid delivery system of item 1, wherein when the extended treatment rapid delivery system is tested in a rabbit model, the average AUCₗₐₛₜ for the therapeutic agent or active metabolite thereof in the iris/ciliary body is about 80% to about 120% of 13,174-49,403 d*ng/mL.
54. The extended treatment rapid delivery system of item 1, wherein when the extended treatment rapid delivery system is tested in a rabbit model, the average Cₘₐₓ for the therapeutic agent or active metabolite thereof in the retina is about 80% to about 120% of 89.9-92.1 ng/mL.
55. The extended treatment rapid delivery system of item 1, wherein when the extended treatment rapid delivery system is tested in a rabbit model, the average AUCₗₐₛₜ for the therapeutic agent or active metabolite thereof in the retina is about 80% to about 120% of 222-764 ng/mL.
56. A method for treating or preventing post-operative ocular inflammation in the eye of a human patient, comprising: administering the extended treatment rapid delivery system of item 1, via intracameral injection or subconjunctival injection, to the eye of a human patient that has undergone ocular surgery or who is about to undergo ocular surgery.
57. The method for treating or preventing post-operative ocular inflammation in the eye of a human patient according to item 56, wherein following said administration the patient has a plasma Cₘₐₓ of difluprednate, or its active metabolite, that is 1 ng/mL or less.
58. The method for treating or preventing post-operative ocular inflammation in the eye of a human patient according to item 56 , wherein following said administration intraocular pressure of the patient is not elevated by more than 10 mmHg.
59. A method for treating or preventing post-operative ocular inflammation in the eye of a human patient, comprising: administering the extended treatment rapid delivery system of item 1, via intracameral injection or subconjunctival injection, to the eye of a human patient that has undergone cataract surgery or who is about to undergo cataract surgery.
60. The method for treating or preventing post-operative ocular inflammation in the eye of a human patient according to item 59, wherein following said administration the patient has a plasma Cₘₐₓ of difluprednate, or its active metabolite, that is 1 ng/mL or less.
61. The method for treating or preventing post-operative ocular inflammation in the eye of a human patient according to item 59 , wherein following said administration intraocular pressure of the patient is not elevated by more than 10 mmHg.
62. A method for making the extended treatment rapid delivery system of item 1, comprising:
   i) providing a mold, wherein the mold comprises a plurality of recessed areas formed therein;
   ii) disposing a volume of liquid material in the plurality of recessed areas, wherein said liquid material is a suspension of at least one crystalline therapeutic agent in a poly(ethylene glycol) melt;
   iii) forming a plurality of substantially uniform implants; and
   iv) harvesting the implants, wherein each of said implants substantially mimics the recessed areas.
63. An extended treatment rapid delivery system for treating post-operative ocular inflammation in an eye of a patient in need thereof, comprising:
   A) a biocompatible polymer matrix based ocular implant rapid delivery vehicle; and
   B) at least one therapeutic agent homogeneously dispersed within the polymer matrix,
   wherein said ocular implant rapid delivery vehicle is formulated to reduce corneal thickening, wherein the corneal thickening is associated with administration of a hydrophilic implant.
64. The extended treatment rapid delivery system of item 63, wherein the biocompatible polymer matrix comprises poly(ethylene glycol).
65. The extended treatment rapid delivery system of item 63, wherein the biocompatible polymer matrix comprises a mixture of two or more poly(ethylene glycol) polymers of different molecular weight.
66. The extended treatment rapid delivery system of item 63, wherein the biocompatible polymer matrix comprises a mixture of two or more poly(ethylene glycol) polymers of different molecular weight, wherein one of said polymers has a molecular weight of about 3,350 and one of said polymers has a molecular weight of about 100,000.
67. The extended treatment rapid delivery system of item 63, wherein the biocompatible polymer matrix comprises a mixture of two or more poly(ethylene glycol) polymers of different molecular weight, wherein one of said polymers has a molecular weight of about 3,350 and is present in an amount of about 10-20 weight percent of the total polymer and one of said polymers has a molecular weight of about 100,000 and is present in an amount of about 80-90 weight percent of the total polymer.
68. The extended treatment rapid delivery system of item 63, wherein the biocompatible polymer matrix comprises a mixture of two or more poly(ethylene glycol) polymers of different molecular weight, wherein one of said polymers has a molecular weight of about 3,350 and is present in an amount of about 11 weight percent of the total polymer and one of said polymers has a molecular weight of about 100,000 and is present in an amount of about 89 weight percent of the total polymer.
69. The extended treatment rapid delivery system of item 63, wherein the biocompatible polymer matrix comprises as a percent weight/weight ratio (%w/w) of the extended treatment rapid delivery system from about 40% w/w to about 85% w/w.
70. The extended treatment rapid delivery system of item 63, wherein the biocompatible polymer matrix comprises as a percent weight/weight (%w/w) ratio of the extended treatment rapid delivery system from about 55% w/w to about 70% w/w.
71. The extended treatment rapid delivery system of item 63, wherein the biocompatible polymer matrix comprises as a percent weight/weight (%w/w) ratio of the extended treatment rapid delivery system from about 65% w/w to about 85% w/w.
72. The extended treatment rapid delivery system of item 63, wherein the biocompatible polymer matrix comprises as a mass content of the extended treatment rapid delivery system from about 20 µg to about 136 µg.
73. The extended treatment rapid delivery system of item 63, wherein the biocompatible polymer matrix comprises as a mass content of the extended treatment rapid delivery system from about 30 µg to about 38 µg.
74. The extended treatment rapid delivery system of item 63, wherein the biocompatible polymer matrix comprises as a mass content of the extended treatment rapid delivery system from about 104 µg to about 136 µg.
75. The extended treatment rapid delivery system of item 63, wherein the ocular implant rapid delivery vehicle is rod-shaped with dimensions of about 130 µm × 180 µm × 1,500 µm, and wherein the at least one therapeutic agent is difluprednate present in an amount of from about 16 µg to about 24 µg.
76. The extended treatment rapid delivery system of item 63, wherein the ocular implant rapid delivery vehicle is rod-shaped with dimensions of about 225 µm × 225 µm × 2,925 µm, and wherein the at least one therapeutic agent is difluprednate present in an amount of from about 24 µg to about 56 µg.

## Claims

1. An extended treatment rapid delivery system for treating post-operative ocular inflammation in an eye of a patient in need thereof, comprising:
A) a biocompatible polymer matrix based ocular implant rapid delivery vehicle; and
B) at least one therapeutic agent homogeneously dispersed within the polymer matrix,
wherein said ocular implant rapid delivery vehicle is formulated to substantially disintegrate within 24 hours of being placed into the eye, and
wherein said disintegration of the ocular implant rapid delivery vehicle results in substantially all of the at least one therapeutic agent being released into the eye within 24 hours, and
wherein the at least one therapeutic agent provides a therapeutic effect to the eye of the patient for at least 2 days, and
wherein said ocular implant rapid delivery vehicle is formulated to reduce post-operative corneal thickening in a patient in need thereof.

2. The extended treatment rapid delivery system of claim 1, wherein the at least one therapeutic agent provides a therapeutic effect to the eye of the patient for at least 3 days.

3. The extended treatment rapid delivery system of claim 1, wherein the at least one therapeutic agent provides a therapeutic effect to the eye of the patient for at least 5 days.

4. The extended treatment rapid delivery system of claim 1, wherein the at least one therapeutic agent provides a therapeutic effect to the eye of the patient for at least 7 days.

5. The extended treatment rapid delivery system of claim 1, wherein the at least one therapeutic agent provides a therapeutic effect to the eye of the patient for at least 14 days.

6. The extended treatment rapid delivery system of claim 1, wherein the at least one therapeutic agent provides a therapeutic effect to the eye of the patient for at least 28 days.

7. The extended treatment rapid delivery system of claim 1, wherein the at least one therapeutic agent has a low solubility under normal human eye physiological conditions.

8. The extended treatment rapid delivery system of claim 1, wherein the at least one therapeutic agent has a water solubility of about 0.0097 mg/mL.

9. The extended treatment rapid delivery system of claim 1, wherein the biocompatible polymer matrix comprises poly(ethylene glycol).

10. The extended treatment rapid delivery system of claim 1, wherein the biocompatible polymer matrix comprises a mixture of two or more poly(ethylene glycol) polymers of different molecular weight.

11. The extended treatment rapid delivery system of claim 1, wherein the biocompatible polymer matrix comprises a mixture of two or more poly(ethylene glycol) polymers of different molecular weight, wherein one of said polymers has a molecular weight of about 3,350 and one of said polymers has a molecular weight of about 100,000.

12. The extended treatment rapid delivery system of claim 1, wherein the biocompatible polymer matrix comprises a mixture of two or more poly(ethylene glycol) polymers of different molecular weight, wherein one of said polymers has a molecular weight of about 3,350 and is present in an amount of about 10-20 weight percent of the total polymer and one of said polymers has a molecular weight of about 100,000 and is present in an amount of about 80-90 weight percent of the total polymer.

13. The extended treatment rapid delivery system of claim 1, wherein the biocompatible polymer matrix comprises a mixture of two or more poly(ethylene glycol) polymers of different molecular weight, wherein one of said polymers has a molecular weight of about 3,350 and is present in an amount of about 11 weight percent of the total polymer and one of said polymers has a molecular weight of about 100,000 and is present in an amount of about 89 weight percent of the total polymer.

14. The extended treatment rapid delivery system of claim 1, wherein the biocompatible polymer matrix comprises a mixture of two or more poly(ethylene glycol) polymers of different molecular weight, wherein one of said polymers has a molecular weight of about 3,350 and is present in an amount of about 7-10 weight percent of the ocular implant and one of said polymers has a molecular weight of about 100,000 and is present in an amount of about 57-76 weight percent of the ocular implant, and wherein the at least one therapeutic agent is present in an amount of about 15-35 weight percent of the ocular implant.

15. The extended treatment rapid delivery system of claim 1, wherein the biocompatible polymer matrix comprises a mixture of two or more poly(ethylene glycol) polymers of different molecular weight, wherein one of said polymers has a molecular weight of about 3,350 and is present in an amount of about 4-7 weight percent of the ocular implant and one of said polymers has a molecular weight of about 100,000 and is present in an amount of about 35-54 weight percent of the ocular implant, and wherein the at least one therapeutic agent is present in an amount of about 40-60 weight percent of the ocular implant.
